# EUROPEAN PATENT APPLICATION

(11) **EP 1 731 607 A1**
(43) Date of publication of application: **13.12.2006**
(21) Application number: 05105076.3
(22) Date of filing: 09.06.2005
(51) Int. Cl.: C12N 15/11, C12N 15/63, C12N 5/16

(54) **shRNA and siRNA expression in a living organism under control of a codon-optimized tetracycline repressor gene**

(71) Applicant: ARTEMIS Pharmaceuticals GmbH, 51063 Köln (DE)
(72) Inventor: Seibler, Jost, Dr., 50733 Köln (DE); Schwenk, Frieder, 50733 Köln (DE); Küter-Luks, Birgit, 50670 Köln (DE)
(74) Representative: von Kreisler Selting Werner

(57) **Abstract**

The present invention relates to a biological entity carrying a regulator construct comprising a specific tetracycline repressor gene and a responder construct comprising at least one segment corresponding to a short hairpin RNA (shRNA) or corresponding to complementary short interfering RNA (siRNA) strands, said at least one segment being under control of a promoter which contains an operator sequence corresponding to the repressor. The invention further relates to a method for preparing said biological entity and its use.

SEQUENCE LISTING

<110> Artemis Pharmaceuticals GmbH

<120> shRNA and siRNA Expression in a Living Organism Under Control of a
      Codon-Optimized Repressor Gene

<130> 050910ep/JH/HR

<140>
<141>

<160> 220

<170> PatentIn Ver. 2.1

<210> 1
<211> 17743
<212> DNA
<213> Artificial Sequence

<220>
<223> Description of Artificial Sequence: Targeting vector for rosa26
      locus expressing the wt tet-repressor

<400> 1
tagggataac agggtaatat agccgcggca ggccctccga gcgtggtgga gccgttctgt     60

gagacagccg ggtacgagtc gtgacgctgg aaggggcaag cgggtggtgg gcaggaatgc    120

ggtccgccct gcagcaaccg gagggggagg gagaagggag cggaaaagtc tccaccggac    180

gcggccatgg ctcggggggg ggggggcagc ggaggagcgc ttccggccga cgtctcgtcg    240

ctgattggct tcttttcctc ccgccgtgtg tgaaaacaca aatggcgtgt tttggttggc    300

gtaaggcgcc tgtcagttaa cggcagccgg agtgcgcagc cgccggcagc ctcgctctgc    360

ccactgggtg gggcgggagg taggtggggt gaggcgagct ggacgtgcgg gcgcggtcgg    420

cctctggcgg ggcgggggag gggagggagg gtcagcgaaa gtagctcgcg cgcgagcggc    480

cgcccaccct ccccttcctc tgggggagtc gttttacccg ccgccggccg ggcctcgtcg    540

tctgattggc tctcggggcc cagaaaactg gcccttgcca ttggctcgtg ttcgtgcaag    600

ttgagtccat ccgccggcca gcgggggcgg cgaggaggcg ctcccaggtt ccggccctcc    660

cctcggcccc gcgccgcaga gtctggccgc gcgcccctgc gcaacgtggc aggaagcgcg    720

cgctgggggc ggggacgggc agtagggctg agcggctgcg gggcgggtgc aagcacgttt    780

ccgacttgag ttgcctcaag aggggcgtgc tgagccagac ctccatcgcg cactccgggg    840

agtggaggga aggagcgagg gctcagttgg gctgttttgg aggcaggaag cacttgctct    900

cccaaagtcg ctctgagttg ttatcagtaa gggagctgca gtggagtagg cggggagaag    960

gccgcaccct tctccggagg ggggagggga gtgttgcaat acctttctgg gagttctctg   1020

ctgcctcctg gcttctgagg accgccctgg gcctgggaga atcccttccc cctcttccct   1080

cgtgatctgc aactccagtc tttctaggta accgatatcc ctgcaggggt gacctgcacg 1140

tctagggcgc agtagtccag ggtttccttg atgatgtcat acttatcctg tccctttttt 1200

ttccacagct cgcggttgag gacaaactct tcgcggtctt tccagtactc ctgcaggtga 1260

ctgactgagt cgagatctgc gatctaagta agcttggcat tccggtactg ttggtaaagc 1320

caccatggaa gacgccaaaa acataaagaa aggcccggcg ccattctatc cgctggaaga 1380

tggaaccgct ggagagcaac tgcataaggc tatgaagaga tacgccctgg ttcctggaac 1440

aattgctttt acagatgcac atatcgaggt ggacatcact tacgctgagt acttcgaaat 1500

gtccgttcgg ttggcagaag ctatgaaacg atatgggctg aatacaaatc acagaatcgt 1560

cgtatgcagt gaaaactctc ttcaattctt tatgccggtg ttgggcgcgt tatttatcgg 1620

agttgcagtt gcgcccgcga acgacattta taatgaacgt gaattgctca acagtatggg 1680

catttcgcag cctaccgtgg tgttcgtttc caaaaagggg ttgcaaaaaa ttttgaacgt 1740

gcaaaaaaag ctcccaatca tccaaaaaat tattatcatg gattctaaaa cggattacca 1800

gggatttcag tcgatgtaca cgttcgtcac atctcatcta cctcccggtt ttaatgaata 1860

cgattttgtg ccagagtcct tcgataggga caagacaatt gcactgatca tgaactcctc 1920

tggatctact ggtctgccta aaggtgtcgc tctgcctcat agaactgcct gcgtgagatt 1980

ctcgcatgcc agagatccta tttttggcaa tcaaatcatt ccggatactg cgattttaag 2040

tgttgttcca ttccatcacg gttttggaat gtttactaca ctcggatatt tgatatgtgg 2100

atttcgagtc gtcttaatgt atagatttga agaagagctg tttctgagga gccttcagga 2160

ttacaagatt caaagtgcgc tgctggtgcc aaccctattc tccttcttcg ccaaaagcac 2220

tctgattgac aaatacgatt tatctaattt acacgaaatt gcttctggtg gcgctcccct 2280

ctctaaggaa gtcggggaag cggttgccaa gaggttccat ctgccaggta tcaggcaagg 2340

atatgggctc actgagacta catcagctat tctgattaca cccgaggggg atgataaacc 2400

gggcgcggtc ggtaaagttg ttccattttt tgaagcgaag gttgtggatc tggataccgg 2460

gaaaacgctg ggcgttaatc aaagaggcga actgtgtgtg agaggtccta tgattatgtc 2520

cggttatgta aacaatccgg aagcgaccaa cgccttgatt gacaaggatg gatggctaca 2580

ttctggagac atagcttact gggacgaaga cgaacacttc ttcatcgttg accgcctgaa 2640

gtctctgatt aagtacaaag gctatcaggt ggctcccgct gaattggaat ccatcttgct 2700

ccaacacccc aacatcttcg acgcaggtgt cgcaggtctt cccgacgatg acgccggtga 2760

acttcccgcc gccgttgttg ttttggagca cggaaagacg atgacggaaa aagagatcgt 2820

ggattacgtc gccagtcaag taacaaccgc gaaaaagttg cgcggaggag ttgtgtttgt 2880

ggacgaagta ccgaaaggtc ttaccggaaa actcgacgca agaaaaatca gagagatcct 2940

cataaaggcc aagaagggcg gaaagatcgc cgtgtaattc tagaccggtt cgagatccag 3000

gcgcggatca ataaaagatc attattttca atagatctgt gtgttggttt tttgtgtgcc 3060

ttgggggagg gggaggccag aatgaggcgc ggccaagggg gagggggagg ccagaatgac 3120

cttgggggag ggggaggcca gaatgacctt gggggagggg gaggccagaa tgaggcgcgc 3180

ccccgatccg tcgacgcccg ggctagcttg catgcctgca ggttttcgac attgattatt 3240

gactagttat taatagtaat caattacggg gtcattagtt catagcccat atatggagtt 3300

ccgcgttaca taacttacgg taaatggccc gcctggctga ccgcccaacg acccccgccc 3360

attgacgtca ataatgacgt atgttcccat agtaacgcca atagggactt tccattgacg 3420

tcaatgggtg gactatttac ggtaaactgc ccacttggca gtacatcaag tgtatcatat 3480

gccaagtacg ccccctattg acgtcaatga cggtaaatgg cccgcctggc attatgccca 3540

gtacatgacc ttatgggact ttcctacttg gcagtacatc tacgtattag tcatcgctat 3600

taccatgggt cgaggtgagc cccacgttct gcttcactct ccccatctcc cccccctccc 3660

cacccccaat tttgtattta tttatttttt aattattttg tgcagcgatg ggggcggggg 3720

gggggggggc gcgcgccagg cggggcgggg cggggcgagg ggcggggcgg ggcgaggcgg 3780

agaggtgcgg cggcagccaa tcagagcggc gcgctccgaa agtttccttt tatggcgagg 3840

cggcggcggc ggcggcccta taaaaagcga agcgcgcggc gggcgggagt cgctgcgttg 3900

ccttcgcccc gtgccccgct ccgcgccgcc tcgcgccgcc cgccccggct ctgactgacc 3960

gcgttactcc cacaggtgag cgggcgggac ggcccttctc ctccgggctg taattagcgc 4020

ttggtttaat gacggctcgt ttcttttctg tggctgcgtg aaagccttaa agggctccgg 4080

gagggccctt tgtgcggggg ggagcggctc ggggggtgcg tgcgtgtgtg tgtgcgtggg 4140

gagcgccgcg tgcggcccgc gctgcccggc ggctgtgagc gctgcgggcg cggcgcgggg 4200

ctttgtgcgc tccgcgtgtg cgcgagggga gcgcggccgg gggcggtgcc ccgcggtgcg 4260

ggggggctgc gaggggaaca aaggctgcgt gcggggtgtg tgcgtggggg ggtgagcagg 4320

gggtgtgggc gcggcggtcg ggctgtaacc cccccctgca cccccctccc cgagttgctg 4380

agcacggccc ggcttcgggt gcggggctcc gtgcggggcg tggcgcgggg ctcgccgtgc 4440

cgggcggggg gtggcggcag gtgggggtgc cgggcggggc ggggccgcct cgggccgggg 4500

agggctcggg ggaggggcgc ggcggccccg gagcgccggc ggctgtcgag gcgcggcgag 4560

ccgcagccat tgccttttat ggtaatcgtg cgagagggcg cagggacttc ctttgtccca 4620

aatctggcgg agccgaaatc tgggaggcgc cgccgcaccc cctctagcgg gcgcgggcga 4680

agcggtgcgg cgccggcagg aaggaaatgg gcggggaggg ccttcgtgcg tcgccgcgcc 4740

gccgtcccct tctccatctc cagcctcggg gctgccgcag ggggacggct gccttcgggg 4800

gggacggggc agggcggggt tcggcttctg gcgtgtgacc ggcggctcta gaagcgttgg 4860

ggtgagtact ccctctcaaa agcgggcatg acttctgcgc taagattgtc agtttccaaa 4920

aacgaggagg atttgatatt cacctggccc gcggtgatgc ctttgagggt ggccgcgtcc 4980

atctggtcag aaaagacaat ctttttgttg tcaagcttga ggtgtggcag gcttgagatc 5040

tggccataca cttgagtgac attgacatcc actttgcctt tctctccaca ggtgtccact 5100

cccagggcgg cctccggagc gatcgccgat ccgcctagca ttcaaccatg gctagattag 5160

ataaaagtaa agtgattaac agcgcattag agctgcttaa tgaggtcgga atcgaaggtt 5220

taacaacccg taaactcgcc cagaagctag gtgtagagca gcctacattg tattggcatg 5280

taaaaaataa gcgggctttg ctcgacgcct tagccattga gatgttagat aggcaccata 5340

ctcacttttg ccctttagaa ggggaaagct ggcaagattt tttacgtaat aacgctaaaa 5400

gttttagatg tgctttacta agtcatcgcg atggagcaaa agtacattta ggtacacggc 5460

ctacagaaaa acagtatgaa actctcgaaa atcaattagc ctttttatgc caacaaggtt 5520

tttcactaga gaatgcatta tatgcactca gcgctgtggg gcattttact ttaggttgcg 5580

tattggaaga tcaagagcat caagtcgcta aagaagaaag ggaaacacct actactgata 5640

gtatgccgcc attattacga caagctatcg aattatttga tcaccaaggt gcagagccag 5700

ccttcttatt cggccttgaa ttgatcatat gcggattaga aaaacaactt aaatgtgaaa 5760

gtgggtcgta accggttcga gatccaggcg cggatcaata aaagatcatt attttcaata 5820

gatctgtgtg ttggtttttt gtgtgccttg ggggaggggg aggccagaat gaggcgcggc 5880

caagggggag ggggaggcca gaatgacctt gggggagggg gaggccagaa tgaccttggg 5940

ggagggggag gccagaatga ggcgcgcccc cgggtaccga gctcgaattg ttaattaagg 6000

ccatagcggc cgccctgagg ccgcgggcga tcgcctaggg gtaaccgaag ttcctatact 6060

ttctagagaa taggaacttc ggaataggaa cttcttataa tctagaagat ctggatccac 6120

gattcgaggg cccctgcagg tcaattctac cgggtagggg aggcgctttt cccaaggcag 6180

tctggagcat gcgctttagc agccccgctg gcacttggcg ctacacaagt ggcctctggc 6240

ctcgcacaca ttccacatcc accggtagcg ccaaccggct ccgttctttg gtggcccctt 6300

cgcgccacct tctactcctc ccctagtcag gaagttcccc cccgccccgc agctcgcgtc 6360

gtgcaggacg tgacaaatgg aagtagcacg tctcactagt ctcgtgcaga tggacagcac 6420

cgctgagcaa tggaagcggg taggcctttg gggcagcggc caatagcagc tttgctcctt 6480

cgctttctgg gctcagaggc tgggaagggg tgggtccggg ggcgggctca ggggcgggct 6540

caggggcggg gcgggcgcga aggtcctccc gaggcccggc attctcgcac gcttcaaaag 6600

cgcacgtctg ccgcgctgtt ctcctcttcc tcatctccgg gcctttcgac gatccagccg 6660

ccaccatgaa aaagcctgaa ctcaccgcga cgtctgtcga gaagtttctg atcgaaaagt 6720

tcgacagcgt ctccgacctg atgcagctct cggagggcga agaatctcgt gctttcagct 6780

tcgatgtagg agggcgtgga tatgtcctgc gggtaaatag ctgcgccgat ggtttctaca 6840

aagatcgtta tgtttatcgg cactttgcat cggccgcgct cccgattccg gaagtgcttg 6900

acattgggga attcagcgag agcctgacct attgcatctc ccgccgtgca cagggtgtca 6960

cgttgcaaga cctgcctgaa accgaactgc ccgctgttct gcagccggtc gcggaggcca 7020

tggatgccat cgctgcggcc gatcttagcc agacgagcgg gttcggccca ttcggaccgc 7080

aaggaatcgg tcaatacact acatggcgtg atttcatatg cgcgattgct gatccccatg 7140

tgtatcactg gcaaactgtg atggacgaca ccgtcagtgc gtccgtcgcg caggctctcg 7200

atgagctgat gctttgggcc gaggactgcc ccgaagtccg gcacctcgtg cacgcggatt 7260

tcggctccaa caatgtcctg acggacaatg gccgcataac agcggtcatt gactggagcg 7320

aggcgatgtt cggggattcc caatacgagg tcgccaacat cttcttctgg aggccgtggt 7380

tggcttgtat ggagcagcag acgcgctact tcgagcggag gcatccggag cttgcaggat 7440

cgccgcggct ccgggcgtat atgctccgca ttggtcttga ccaactctat cagagcttgg 7500

ttgacggcaa tttcgatgat gcagcttggg cgcagggtcg atgcgacgca atcgtccgat 7560

ccggagccgg gactgtcggg cgtacacaaa tcgcccgcag aagcgcggcc gtctggaccg 7620

atggctgtgt agaagtactc gccgatagtg gaaaccgacg ccccagcact cgtccgaggg 7680

caaaggaata gtcgatgcag aaattgatga tctattaaac aataaagatg tccactaaaa 7740

tggaagtttt tcctgtcata ctttgttaag aagggtgaga acagagtacc tacattttga 7800

atggaaggat tggagctacg ggggtggggg tggggtggga ttagataaat gcctgctctt 7860

tactgaaggc tctttactat tgctttatga taatgtttca tagttggata tcataattta 7920

aacaagcaaa accaaattaa gggccagctc attcctccca ctcatgatct atagatctat 7980

agatctctcg tgggatcatt gtttttctct tgattcccac tttgtggttc taagtactgt 8040

ggtttccaaa tgtgtcagtt tcatagcctg aagaacgaga tcagcagcct ctgttccaca 8100

tacacttcat tctcagtatt gttttgccaa gttctaattc catcagaagc tgactctaga 8160

tcctgcagga attcatatga agttcctata ctttctagag aataggaact tcggaatagg 8220

aacttcaaaa tgtcgcggcg cgccggtaac cgaagttcct atactttcta gagaatagga 8280

acttcggaat aggaacttca agcttaagcg ctagaagatg ggcgggagtc ttctgggcag 8340

gcttaaaggc taacctggtg tgtgggcgtt gtcctgcagg ggaattgaac aggtgtaaaa 8400

ttggagggac aagacttccc acagattttc ggttttgtcg ggaagttttt taataggggc 8460

aaataaggaa aatgggagga taggtagtca tctggggttt tatgcagcaa aactacaggt 8520

tattattgct tgtgatccgc ctcggagtat tttccatcga ggtagattaa agacatgctc 8580

acccgagttt tatactctcc tgcttgagat ccttactaca gtatgaaatt acagtgtcgc 8640

gagttagact atgtaagcag aattttaatc atttttaaag agcccagtac ttcatatcca 8700

tttctcccgc tccttctgca gccttatcaa aaggtatttt agaacactca ttttagcccc 8760

attttcattt attatactgg cttatccaac ccctagacag agcattggca ttttcccttt 8820

cctgatctta gaagtctgat gactcatgaa accagacaga ttagttacat acaccacaaa 8880

tcgaggctgt agctggggcc tcaacactgc agttctttta taactcctta gtacactttt 8940

tgttgatcct ttgccttgat ccttaatttt cagtgtctat cacctctccc gtcagtggtg 9000

ttccacattt gggcctattc tcagtccagg gagttttaca acaatagatg tattgagaat 9060

ccaacctaaa gcttaacttt ccactcccat gaatgcctct ctcctttttc tccatttata 9120

aactgagcta ttaaccatta atggttccag gtggatgtct cctccccata ttacctgatg 9180

tatcttacat attgccaggc tgatatttta agacattaaa aggtatattt cattattgag 9240

ccacatggta ttgattactg cttactaaaa ttttgtcatt gtacacatct gtaaaaggtg 9300

gttccttttg gaatgcaaag ttcaggtgtt tgttgtcttt cctgacctaa ggtcttgtga 9360

gcttgtattt tttctattta agcagtgctt tctcttggac tggcttgact catggcattc 9420

tacacgttat tgctggtcta aatgtgattt tgccaagctt cttcaggacc tataattttg 9480

cttgacttgt agccaaacac aagtaaaatg attaagcaac aaatgtattt gtgaagcttg 9540

gtttttaggt tgttgtgttg tgtgtgcttg tgctctataa taatactatc caggggctgg 9600

agaggtggct cggagttcaa gagcacagac tgctcttcca gaagtcctga gttcaattcc 9660

cagcaaccac atggtggctc acaaccatct gtaatgggat ctgatgccct cttctggtgt 9720

gtctgaagac cacaagtgta ttcacattaa ataaataaat cctccttctt cttctttttt 9780

ttttttttaa agagaatact gtctccagta gaatttactg aagtaatgaa atactttgtg 9840

tttgttccaa tatggtagcc aataatcaaa ttactcttta agcactggaa atgttaccaa 9900

ggaactaatt tttatttgaa gtgtaactgt ggacagagga gccataactg cagacttgtg 9960

ggatacagaa gaccaatgca gactttaatg tcttttctct tacactaagc aataaagaaa 10020

taaaaattga acttctagta tcctatttgt ttaaactgct agctttactt aacttttgtg 10080

cttcatctat acaaagctga aagctaagtc tgcagccatt actaaacatg aaagcaagta 10140

atgataattt tggatttcaa aaatgtaggg ccagagttta gccagccagt ggtggtgctt 10200

gcctttatgc ctttaatccc agcactctgg aggcagagac aggcagatct ctgagtttga 10260

gcccagcctg gtctacacat caagttctat ctaggatagc caggaataca cacagaaacc 10320

ctgttgggga ggggggctct gagatttcat aaaattataa ttgaagcatt ccctaatgag 10380

ccactatgga tgtggctaaa tccgtctacc tttctgatga gatttgggta ttattttttc 10440

tgtctctgct gttggttggg tcttttgaca ctgtgggctt tctttaaagc ctccttcctg 10500

ccatgtggtc tcttgtttgc tactaacttc ccatggctta aatggcatgg ctttttgcct 10560

tctaagggca gctgctgaga tttgcagcct gatttccagg gtggggttgg gaaatctttc 10620

aaacactaaa attgtccttt aatttttttt ttaaaaaatg ggttatataa taaacctcat 10680

aaaatagtta tgaggagtga ggtggactaa tattaaatga gtccctcccc tataaaagag 10740

ctattaaggc tttttgtctt atacttaact ttttttttaa atgtggtatc tttagaacca 10800

agggtcttag agttttagta tacagaaact gttgcatcgc ttaatcagat tttctagttt 10860

caaatccaga gaatccaaat tcttcacagc caaagtcaaa ttaagaattt ctgactttta 10920

atgttaattt gcttactgtg aatataaaaa tgatagcttt tcctgaggca gggtctcact 10980

atgtatctct gcctgatctg caacaagata tgtagactaa agttctgcct gcttttgtct 11040

cctgaatact aaggttaaaa tgtagtaata cttttggaac ttgcaggtca gattctttta 11100

taggggacac actaagggag cttgggtgat agttggtaaa atgtgtttca agtgatgaaa 11160

acttgaatta ttatcaccgc aacctacttt ttaaaaaaaa aagccaggcc tgttagagca 11220

tgcttaaggg atccctagga cttgctgagc acacaagagt agttacttgg caggctcctg 11280

gtgagagcat atttcaaaaa acaaggcaga caaccaagaa actacagtta aggttacctg 11340

tctttaaacc atctgcatat acacagggat attaaaatat tccaaataat atttcattca 11400

agttttcccc catcaaattg ggacatggat ttctccggtg aataggcaga gttggaaact 11460

aaacaaatgt tggttttgtg atttgtgaaa ttgttttcaa gtgatagtta aagcccatga 11520

gatacagaac aaagctgcta tttcgaggtc tcttggttta tactcagaag cacttctttg 11580

ggtttccctg cactatcctg atcatgtgct aggcctacct taggctgatt gttgttcaaa 11640

taaacttaag tttcctgtca ggtgatgtca tatgatttca tatatcaagg caaaacatgt 11700

tatatatgtt aaacatttgt acttaatgtg aaagttaggt ctttgtgggt ttgattttta 11760

attttcaaaa cctgagctaa ataagtcatt tttacatgtc ttacatttgg tggaattgta 11820

taattgtggt ttgcaggcaa gactctctga cctagtaacc ctacctatag agcactttgc 11880

tgggtcacaa gtctaggagt caagcatttc accttgaagt tgagacgttt tgttagtgta 11940

tactagttta tatgttggag gacatgttta tccagaagat attcaggact atttttgact 12000

gggctaagga attgattctg attagcactg ttagtgagca ttgagtggcc tttaggcttg 12060

aattggagtc acttgtatat ctcaaataat gctggccttt tttaaaaagc ccttgttctt 12120

tatcaccctg ttttctacat aatttttgtt caaagaaata cttgtttgga tctccttttg 12180

acaacaatag catgttttca agccatattt tttttccttt tttttttttt ttttggtttt 12240

tcgagacagg gtttctctgt atagccctgg ctgtcctgga actcactttg tagaccaggc 12300

tggcctcgaa ctcagaaatc cgcctgcctc tgcctcctga gtgccgggat taaaggcgtg 12360

caccaccacg cctggctaag ttggatattt tgttatataa ctataaccaa tactaactcc 12420

actgggtgga tttttaattc agtcagtagt cttaagtggt ctttattggc ccttcattaa 12480

aatctactgt tcactctaac agaggctgtt ggtactagtg gcacttaagc aacttcctac 12540

ggatatacta gcagattaag ggtcagggat agaaactagt ctagcgtttt gtatacctac 12600

cagctttata ctaccttgtt ctgatagaaa tatttcagga catctagcac gtgttaactc 12660

gagctgcagg attcgagggc cccggcaggt caattctacc gggtagggga ggcgcttttc 12720

ccaaggcagt ctggagcatg cgctttagca gccccgctgg gcacttggcg ctacacaagt 12780

ggcctctggc ctcgcacaca ttccacatcc accggtaggc gccaaccggc tccgttcttt 12840

ggtggcccct tcgcgccacc ttctactcct cccctagtca ggaagttccc ccccgccccg 12900

cagctcgcgt cgtgcaggac gtgacaaatg gaagtagcac gtctcactag tctcgtgcag 12960

atggacagca ccgctgagca atggaagcgg gtaggccttt ggggcagcgg ccaatagcag 13020

ctttgctcct tcgctttctg ggctcagagg ctgggaaggg gtgggtccgg gggcgggctc 13080

aggggcgggc tcaggggcgg ggcgggcgcc cgaaggtcct ccggaggccc ggcattctgc 13140

acgcttcaaa agcgcacgtc tgccgcgctg ttctcctctt cctcatctcc gggcctttcg 13200

acctgcagcc aatgcaccgt ccttgccatc atggcctcgt accccggcca tcaacacgcg 13260

tctgcgttcg accaggctgc gcgttctcgc ggccatagca accgacgtac ggcgttgcgc 13320

cctcgccggc agcaagaagc cacggaagtc cgcccggagc agaaaatgcc cacgctactg 13380

cgggtttata tagacggtcc ccacgggatg gggaaaacca ccaccacgca actgctggtg 13440

gccctgggtt cgcgcgacga tatcgtctac gtacccgagc cgatgactta ctggcgggtg 13500

ctgggggctt ccgagacaat cgcgaacatc tacaccacac aacaccgcct cgaccagggt 13560

gagatatcgg ccggggacgc ggcggtggta atgacaagcg cccagataac aatgggcatg 13620

ccttatgccg tgaccgacgc cgttctggct cctcatatcg ggggggaggc tgggagctca 13680

catgccccgc ccccggccct caccctcatc ttcgaccgcc atcccatcgc cgccctcctg 13740

tgctacccgg ccgcgcggta ccttatgggc agcatgaccc cccaggccgt gctggcgttc 13800

gtggccctca tcccgccgac cttgcccggc accaacatcg tgcttggggc ccttccggag 13860

gacagacaca tcgaccgcct ggccaaacgc cagcgccccg gcgagcggct ggacctggct 13920

atgctggctg cgattcgccg cgtttacggg ctacttgcca atacggtgcg gtatctgcag 13980

tgcggcgggt cgtggcggga ggactgggga cagctttcgg ggacggccgt gccgccccag 14040

ggtgccgagc cccagagcaa cgcgggccca cgaccccata tcggggacac gttatttacc 14100

ctgtttcggg cccccgagtt gctggccccc aacggcgacc tgtataacgt gtttgcctgg 14160

gccttggacg tcttggccaa acgcctccgt tccatgcacg tctttatcct ggattacgac 14220

caatcgcccg ccggctgccg ggacgccctg ctgcaactta cctccgggat ggtccagacc 14280

cacgtcacca cccccggctc cataccgacg atatgcgacc tggcgcgcac gtttgcccgg 14340

gagatggggg aggctaactg aggggatcga tccgtcctgt aagtctgcag aaattgatga 14400

tctattaaac aataaagatg tccactaaaa tggaagtttt tcctgtcata ctttgttaag 14460

aagggtgaga acagagtacc tacattttga atggaaggat tggagctacg ggggtggggg 14520

tggggtggga ttagataaat gcctgctctt tactgaaggc tctttactat tgctttatga 14580

taatgtttca tagttggata tcataattta aacaagcaaa accaaattaa gggccagctc 14640

attcctccca ctcatgatct atagatctat agatctctcg tgggatcatt gtttttctct 14700

tgattcccac tttgtggttc taagtactgt ggtttccaaa tgtgtcagtt tcatagcctg 14760

aagaacgaga tcagcagcct ctgttccaca tacacttcat tctcagtatt gttttgccaa 14820

gttctaattc catcagaagc tgactctagg ccgagctcca attcgcccta tagtgagtcg 14880

tattacaatt cactggccgt cgttttacaa cgtcgtgact gggaaaaccc tggcgttacc 14940

caacttaatc gccttgcagc acatccccct ttcgccagct ggcgtaatag cgaagaggcc 15000

cgcaccgatc gcccttccca acagttgcgc agcctgaatg gcgaatggga cgcgccctgt 15060

agcggcgcat taagcgcggc gggtgtggtg gttacgcgca gcgtgaccgc tacacttgcc 15120

agcgccctag cgcccgctcc tttcgctttc ttcccttcct ttctcgccac gttcgccggc 15180

tttccccgtc aagctctaaa tcgggggctc cctttagggt tccgatttag tgctttacgg 15240

cacctcgacc ccaaaaaact tgattagggt gatggttcac gtagtgggcc atcgccctga 15300

tagacggttt ttcgcccttt gacgttggag tccacgttct ttaatagtgg actcttgttc 15360

caaactggaa caacactcaa ccctatctcg gtctattctt ttgatttata agggattttg 15420

ccgatttcgg cctattggtt aaaaaatgag ctgatttaac aaaaatttaa cgcgaatttt 15480

aacaaaatat taacgcttac aatttaggtg gcacttttcg gggaaatgtg cgcggaaccc 15540

ctatttgttt atttttctaa atacattcaa atatgtatcc gctcatgaga caataaccct 15600

gataaatgct tcaataatat tgaaaaagga agagtatgag tattcaacat ttccgtgtcg 15660

cccttattcc cttttttgcg gcattttgcc ttcctgtttt tgctcaccca gaaacgctgg 15720

tgaaagtaaa agatgctgaa gatcagttgg gtgcacgagt gggttacatc gaactggatc 15780

tcaacagcgg taagatcctt gagagttttc gccccgaaga acgttttcca atgatgagca 15840

cttttaaagt tctgctatgt ggcgcggtat tatcccgtat tgacgccggg caagagcaac 15900

tcggtcgccg catacactat tctcagaatg acttggttga gtactcacca gtcacagaaa 15960

agcatcttac ggatggcatg acagtaagag aattatgcag tgctgccata accatgagtg 16020

ataacactgc ggccaactta cttctgacaa cgatcggagg accgaaggag ctaaccgctt 16080

ttttgcacaa catgggggat catgtaactc gccttgatcg ttgggaaccg gagctgaatg 16140

aagccatacc aaacgacgag cgtgacacca cgatgcctgt agcaatggca acaacgttgc 16200

gcaaactatt aactggcgaa ctacttactc tagcttcccg gcaacaatta atagactgga 16260

tggaggcgga taaagttgca ggaccacttc tgcgctcggc ccttccggct ggctggttta 16320

ttgctgataa atctggagcc ggtgagcgtg ggtctcgcgg tatcattgca gcactggggc 16380

cagatggtaa gccctcccgt atcgtagtta tctacacgac ggggagtcag gcaactatgg 16440

atgaacgaaa tagacagatc gctgagatag gtgcctcact gattaagcat tggtaactgt 16500

cagaccaagt ttactcatat atactttaga ttgatttaaa acttcatttt taatttaaaa 16560

ggatctaggt gaagatcctt tttgataatc tcatgaccaa aatcccttaa cgtgagtttt 16620

cgttccactg agcgtcagac cccgtagaaa agatcaaagg atcttcttga gatccttttt 16680

ttctgcgcgt aatctgctgc ttgcaaacaa aaaaaccacc gctaccagcg gtggtttgtt 16740

tgccggatca agagctacca actctttttc cgaaggtaac tggcttcagc agagcgcaga 16800

taccaaatac tgtccttcta gtgtagccgt agttaggcca ccacttcaag aactctgtag 16860

caccgcctac atacctcgct ctgctaatcc tgttaccagt ggctgctgcc agtggcgata 16920

agtcgtgtct taccgggttg gactcaagac gatagttacc ggataaggcg cagcggtcgg 16980

gctgaacggg gggttcgtgc acacagccca gcttggagcg aacgacctac accgaactga 17040

gatacctaca gcgtgagcta tgagaaagcg ccacgcttcc cgaagggaga aaggcggaca 17100

ggtatccggt aagcggcagg gtcggaacag gagagcgcac gagggagctt ccagggggaa 17160

acgcctggta tctttatagt cctgtcgggt ttcgccacct ctgacttgag cgtcgatttt 17220

tgtgatgctc gtcagggggg cggagcctat ggaaaaacgc cagcaacgcg gcctttttac 17280

ggttcctggc cttttgctgg ccttttgctc acatgttctt tcctgcgtta tcccctgatt 17340

ctgtggataa ccgtattacc gcctttgagt gagctgatac cgctcgccgc agccgaacga 17400

ccgagcgcag cgagtcagtg agcgaggaag cggaagagcg cccaatacgc aaaccgcctc 17460

tccccgcgcg ttggccgatt cattaatgca gctggcacga caggtttccc gactggaaag 17520

cgggcagtga gcgcaacgca attaatgtga gttagctcac tcattaggca ccccaggctt 17580

tacactttat gcttccggct cgtatgttgt gtggaattgt gagcggataa caatttcaca 17640

caggaaacag ctatgaccat gattacgcca agcgcgcaat taaccctcac taaagggaac 17700

aaaagctgtc gagatctaga tatcgatggc catagagtta cgc 17743

<210> 2
<211> 17907
<212> DNA
<213> Artificial Sequence

<220>
<223> Description of Artificial Sequence: Targeting vector for rosa26
      locus expressing the codon optimized tet-repressor

<400> 2
tagggataac agggtaatat agccgcggca ggccctccga gcgtggtgga gccgttctgt 60

gagacagccg ggtacgagtc gtgacgctgg aaggggcaag cgggtggtgg gcaggaatgc 120

ggtccgccct gcagcaaccg gagggggagg gagaagggag cggaaaagtc tccaccggac 180

gcggccatgg ctcggggggg ggggggcagc ggaggagcgc ttccggccga cgtctcgtcg 240

ctgattggct tcttttcctc ccgccgtgtg tgaaaacaca aatggcgtgt tttggttggc 300

gtaaggcgcc tgtcagttaa cggcagccgg agtgcgcagc cgccggcagc ctcgctctgc 360

ccactgggtg gggcgggagg taggtggggt gaggcgagct ggacgtgcgg gcgcggtcgg 420

cctctggcgg ggcgggggag ggagggaggg gtcagcgaaa gtagctcgcg cgcgagcggc 480

cgcccaccct ccccttcctc tgggggagtc gttttacccg ccgccggccg ggcctcgtcg 540

tctgattggc tctcggggcc cagaaaactg gcccttgcca ttggctcgtg ttcgtgcaag 600

ttgagtccat ccgccggcca gcgggggcgg cgaggaggcg ctcccaggtt ccggccctcc 660

cctcggcccc gcgccgcaga gtctggccgc gcgcccctgc gcaacgtggc aggaagcgcg 720

cgctgggggc ggggacgggc agtagggctg agcggctgcg gggcgggtgc aagcacgttt 780

ccgacttgag ttgcctcaag aggggcgtgc tgagccagac ctccatcgcg cactccgggg 840

agtggaggga aggagcgagg gctcagttgg gctgttttgg aggcaggaag cacttgctct 900

cccaaagtcg ctctgagttg ttatcagtaa gggagctgca gtggagtagg cggggagaag 960

gccgcaccct tctccggagg ggggagggga gtgttgcaat acctttctgg gagttctctg 1020

ctgcctcctg gcttctgagg accgccctgg gcctgggaga atcccttccc cctcttccct 1080

cgtgatctgc aactccagtc tttctaggta accgatatcc ctgcaggggt gacctgcacg 1140

tctagggcgc agtagtccag ggtttccttg atgatgtcat acttatcctg tccctttttt 1200

ttccacagct cgcggttgag gacaaactct tcgcggtctt tccagtactc ctgcaggtga 1260

ctgactgagt cgagatctgc gatctaagta agcttggcat tccggtactg ttggtaaagc 1320

caccatggaa gacgccaaaa acataaagaa aggcccggcg ccattctatc cgctggaaga 1380

tggaaccgct ggagagcaac tgcataaggc tatgaagaga tacgccctgg ttcctggaac 1440

aattgctttt acagatgcac atatcgaggt ggacatcact tacgctgagt acttcgaaat 1500

gtccgttcgg ttggcagaag ctatgaaacg atatgggctg aatacaaatc acagaatcgt 1560

cgtatgcagt gaaaactctc ttcaattctt tatgccggtg ttgggcgcgt tatttatcgg 1620

agttgcagtt gcgcccgcga acgacattta taatgaacgt gaattgctca acagtatggg 1680

catttcgcag cctaccgtgg tgttcgtttc caaaaagggg ttgcaaaaaa ttttgaacgt 1740

gcaaaaaaag ctcccaatca tccaaaaaat tattatcatg gattctaaaa cggattacca 1800

gggatttcag tcgatgtaca cgttcgtcac atctcatcta cctcccggtt ttaatgaata 1860

cgattttgtg ccagagtcct tcgataggga caagacaatt gcactgatca tgaactcctc 1920

tggatctact ggtctgccta aaggtgtcgc tctgcctcat agaactgcct gcgtgagatt 1980

ctcgcatgcc agagatccta tttttggcaa tcaaatcatt ccggatactg cgattttaag 2040

tgttgttcca ttccatcacg gttttggaat gtttactaca ctcggatatt tgatatgtgg 2100

atttcgagtc gtcttaatgt atagatttga agaagagctg tttctgagga gccttcagga 2160

ttacaagatt caaagtgcgc tgctggtgcc aaccctattc tccttcttcg ccaaaagcac 2220

tctgattgac aaatacgatt tatctaattt acacgaaatt gcttctggtg gcgctcccct 2280

ctctaaggaa gtcggggaag cggttgccaa gaggttccat ctgccaggta tcaggcaagg 2340

atatgggctc actgagacta catcagctat tctgattaca cccgaggggg atgataaacc 2400

gggcgcggtc ggtaaagttg ttccattttt tgaagcgaag gttgtggatc tggataccgg 2460

gaaaacgctg ggcgttaatc aaagaggcga actgtgtgtg agaggtccta tgattatgtc 2520

cggttatgta aacaatccgg aagcgaccaa cgccttgatt gacaaggatg gatggctaca 2580

ttctggagac atagcttact gggacgaaga cgaacacttc ttcatcgttg accgcctgaa 2640

gtctctgatt aagtacaaag gctatcaggt ggctcccgct gaattggaat ccatcttgct 2700

ccaacacccc aacatcttcg acgcaggtgt cgcaggtctt cccgacgatg acgccggtga 2760

acttcccgcc gccgttgttg ttttggagca cggaaagacg atgacggaaa aagagatcgt 2820

ggattacgtc gccagtcaag taacaaccgc gaaaaagttg cgcggaggag ttgtgtttgt 2880

ggacgaagta ccgaaaggtc ttaccggaaa actcgacgca agaaaaatca gagagatcct 2940

cataaaggcc aagaagggcg gaaagatcgc cgtgtaattc tagaccggtt cgagatccag 3000

gcgcggatca ataaaagatc attattttca atagatctgt gtgttggttt tttgtgtgcc 3060

ttgggggagg gggaggccag aatgaggcgc ggccaagggg gagggggagg ccagaatgac 3120

cttgggggag ggggaggcca gaatgacctt gggggagggg gaggccagaa tgaggcgcgc 3180
ccccgatccg tcgacgcccg ggctagcttg catgcctgca ggttttcgac attgattatt 3240
gactagttat taatagtaat caattacggg gtcattagtt catagcccat atatggagtt 3300
ccgcgttaca taacttacgg taaatggccc gcctggctga ccgcccaacg acccccgccc 3360
attgacgtca ataatgacgt atgttcccat agtaacgcca atagggactt tccattgacg 3420
tcaatgggtg gactatttac ggtaaactgc ccacttggca gtacatcaag tgtatcatat 3480
gccaagtacg ccccctattg acgtcaatga cggtaaatgg cccgcctggc attatgccca 3540
gtacatgacc ttatgggact ttcctacttg gcagtacatc tacgtattag tcatcgctat 3600
taccatgggt cgaggtgagc cccacgttct gcttcactct ccccatctcc cccccctccc 3660
cacccccaat tttgtattta tttatttttt aattattttg tgcagcgatg ggggcggggg 3720
gggggggggc gcgcgccagg cggggcgggg cggggcgagg ggcggggcgg ggcgaggcgg 3780
agaggtgcgg cggcagccaa tcagagcggc gcgctccgaa agtttccttt tatggcgagg 3840
cggcggcggc ggcggcccta taaaaagcga agcgcgcggc gggcgggagt cgctgcgttg 3900
ccttcgcccc gtgccccgct ccgcgccgcc tcgcgccgcc cgccccggct ctgactgacc 3960
gcgttactcc cacaggtgag cgggcgggac ggcccttctc ctccgggctg taattagcgc 4020
ttggtttaat gacggctcgt ttcttttctg tggctgcgtg aaagccttaa agggctccgg 4080
gagggccctt tgtgcggggg ggagcggctc ggggggtgcg tgcgtgtgtg tgtgcgtggg 4140
gagcgccgcg tgcggcccgc gctgcccggc ggctgtgagc gctgcgggcg cggcgcgggg 4200
ctttgtgcgc tccgcgtgtg cgcgagggga gcgcggccgg gggcggtgcc ccgcggtgcg 4260
ggggggctgc gaggggaaca aaggctgcgt gcggggtgtg tgcgtggggg ggtgagcagg 4320
gggtgtgggc gcggcggtcg ggctgtaacc cccccctgca cccccctccc cgagttgctg 4380
agcacggccc ggcttcgggt gcggggctcc gtgcggggcg tggcgcgggg ctcgccgtgc 4440
cgggcggggg gtggcggcag gtgggggtgc cgggcggggc ggggccgcct cgggccgggg 4500
agggctcggg ggaggggcgc ggcggccccg gagcgccggc ggctgtcgag gcgcggcgag 4560
ccgcagccat tgccttttat ggtaatcgtg cgagagggcg cagggacttc ctttgtccca 4620
aatctggcgg agccgaaatc tgggaggcgc cgccgcaccc cctctagcgg gcgcgggcga 4680
agcggtgcgg cgccggcagg aaggaaatgg gcggggaggg ccttcgtgcg tcgccgcgcc 4740
gccgtcccct tctccatctc cagcctcggg gctgccgcag ggggacggct gccttcgggg 4800
gggacggggc agggcggggt tcggcttctg gcgtgtgacc ggcggctcta gaagcgttgg 4860
ggtgagtact ccctctcaaa agcgggcatg acttctgcgc taagattgtc agtttccaaa 4920

aacgaggagg atttgatatt cacctggccc gcggtgatgc ctttgagggt ggccgcgtcc 4980
atctggtcag aaaagacaat ctttttgttg tcaagcttga ggtgtggcag gcttgagatc 5040
tggccataca cttgagtgac attgacatcc actttgcctt tctctccaca ggtgtccact 5100
cccagggcgg cctccggagc gatcgccggt ccgcctaggc aattcaccat gtccagactg 5160
gacaagagca aagtcatcaa ctctgccttg gagctcctga atgaagttgg cattgagggc 5220
ttgaccacca ggaagctggc ccagaagctg ggtgtggagc agcctaccct gtactggcat 5280
gtgaagaaca agagggctct gcttgatgcc ctggccattg agatgttgga caggcaccac 5340
acccacttct gccctctgga aggggagtcc tggcaggact tcctgaggaa caatgccaag 5400
agcttcagat gtgccttgct ctcccaccgg gatggtgcca aagttcactt gggcaccagg 5460
cctacagaga agcagtatga gaccctggag aaccagctgg cattcctgtg ccaacaaggc 5520
ttctccctgg aaaatgcctt gtatgccctc tctgctgtgg gccacttcac cttgggctgt 5580
gtgctggagg accaggagca ccaagttgcc aaggaggaga gggagacccc caccactgac 5640
tccatgccac cactgctgcg gcaagctatt gagttgtttg accaccaagg ggctgagcct 5700
gcattccttt ttggcctgga actgatcatc tgtggcctgg aaaagcagct gaaatgtgag 5760
tctggctctc acgtgcccaa aaagagaaag cacgtgcctg ctgatgcctt ggatgatttt 5820
gacctggaca tgctgcctgc tgatgccctg gatgactttg atttggacat gctccctgct 5880
gatgcacttg atgattttga cctggatatg ctgtgatgga tccatcgatt ctagaccggt 5940
tcgagatcca ggcgcggatc aataaaagat cattattttc aatagatctg tgtgttggtt 6000
ttttgtgtgc cttgggggag ggggaggcca gaatgaggcg cggccaaggg ggagggggag 6060
gccagaatga ccttggggga gggggaggcc agaatgacct tgggggaggg ggaggccaga 6120
atgaggcgcg cccccgggta ccgagctcga attgttaatt aaggccatag cggccgccct 6180
gaggccgcgg gcgatcgcct aggggtaacc gaagttccta tactttctag agaataggaa 6240
cttcggaata ggaacttctt ataatctaga agatctggat ccacgattcg agggcccctg 6300
caggtcaatt ctaccgggta ggggaggcgc ttttcccaag gcagtctgga gcatgcgctt 6360
tagcagcccc gctggcactt ggcgctacac aagtggcctc tggcctcgca cacattccac 6420
atccaccggt agcgccaacc ggctccgttc tttggtggcc ccttcgcgcc accttctact 6480
cctcccctag tcaggaagtt cccccccgcc ccgcagctcg cgtcgtgcag gacgtgacaa 6540
atggaagtag cacgtctcac tagtctcgtg cagatggaca gcaccgctga gcaatggaag 6600
cgggtaggcc tttggggcag cggccaatag cagctttgct ccttcgcttt ctgggctcag 6660
aggctgggaa ggggtgggtc cgggggcggg ctcaggggcg ggctcagggg cggggcgggc 6720
gcgaaggtcc tcccgaggcc cggcattctc gcacgcttca aaagcgcacg tctgccgcgc 6780

tgttctcctc ttcctcatct ccgggccttt cgacgatcca gccgccacca tgaaaaagcc 6840

tgaactcacc gcgacgtctg tcgagaagtt tctgatcgaa aagttcgaca gcgtctccga 6900

cctgatgcag ctctcggagg gcgaagaatc tcgtgctttc agcttcgatg taggagggcg 6960

tggatatgtc ctgcgggtaa atagctgcgc cgatggtttc tacaaagatc gttatgttta 7020

tcggcacttt gcatcggccg cgctcccgat tccggaagtg cttgacattg gggaattcag 7080

cgagagcctg acctattgca tctcccgccg tgcacagggt gtcacgttgc aagacctgcc 7140

tgaaaccgaa ctgcccgctg ttctgcagcc ggtcgcggag gccatggatg ccatcgctgc 7200

ggccgatctt agccagacga gcgggttcgg cccattcgga ccgcaaggaa tcggtcaata 7260

cactacatgg cgtgatttca tatgcgcgat tgctgatccc catgtgtatc actggcaaac 7320

tgtgatggac gacaccgtca gtgcgtccgt cgcgcaggct ctcgatgagc tgatgctttg 7380

ggccgaggac tgccccgaag tccggcacct cgtgcacgcg gatttcggct ccaacaatgt 7440

cctgacggac aatggccgca taacagcggt cattgactgg agcgaggcga tgttcgggga 7500

ttcccaatac gaggtcgcca acatcttctt ctggaggccg tggttggctt gtatggagca 7560

gcagacgcgc tacttcgagc ggaggcatcc ggagcttgca ggatcgccgc ggctccgggc 7620

gtatatgctc cgcattggtc ttgaccaact ctatcagagc ttggttgacg gcaatttcga 7680

tgatgcagct tgggcgcagg gtcgatgcga cgcaatcgtc cgatccggag ccgggactgt 7740

cgggcgtaca caaatcgccc gcagaagcgc ggccgtctgg accgatggct gtgtagaagt 7800

actcgccgat agtggaaacc gacgccccag cactcgtccg agggcaaagg aatagtcgat 7860

gcagaaattg atgatctatt aaacaataaa gatgtccact aaaatggaag tttttcctgt 7920

catactttgt taagaagggt gagaacagag tacctacatt ttgaatggaa ggattggagc 7980

tacgggggtg ggggtggggt gggattagat aaatgcctgc tctttactga aggctcttta 8040

ctattgcttt atgataatgt ttcatagttg gatatcataa tttaaacaag caaaaccaaa 8100

ttaagggcca gctcattcct cccactcatg atctatagat ctatagatct ctcgtgggat 8160

cattgttttt ctcttgattc ccactttgtg gttctaagta ctgtggtttc caaatgtgtc 8220

agtttcatag cctgaagaac gagatcagca gcctctgttc cacatacact tcattctcag 8280

tattgttttg ccaagttcta attccatcag aagctgactc tagatcctgc aggaattcat 8340

atgaagttcc tatactttct agagaatagg aacttcggaa taggaacttc aaaatgtcgc 8400

ggcgcgccgg taaccgaagt tcctatactt tctagagaat aggaacttcg gaataggaac 8460

ttcaagctta agcgctagaa gatgggcggg agtcttctgg gcaggcttaa aggctaacct 8520

ggtgtgtggg cgttgtcctg caggggaatt gaacaggtgt aaaattggag ggacaagact 8580

tcccacagat tttcggtttt gtcgggaagt tttttaatag gggcaaataa ggaaaatggg 8640

aggataggta gtcatctggg gttttatgca gcaaaactac aggttattat tgcttgtgat 8700

ccgcctcgga gtattttcca tcgaggtaga ttaaagacat gctcacccga gttttatact 8760

ctcctgcttg agatccttac tacagtatga aattacagtg tcgcgagtta gactatgtaa 8820

gcagaatttt aatcattttt aaagagccca gtacttcata tccatttctc ccgctccttc 8880

tgcagcctta tcaaaaggta ttttagaaca ctcattttag ccccattttc atttattata 8940

ctggcttatc caacccctag acagagcatt ggcattttcc ctttcctgat cttagaagtc 9000

tgatgactca tgaaaccaga cagattagtt acatacacca caaatcgagg ctgtagctgg 9060

ggcctcaaca ctgcagttct tttataactc cttagtacac tttttgttga tcctttgcct 9120

tgatccttaa ttttcagtgt ctatcacctc tcccgtcagt ggtgttccac atttgggcct 9180

attctcagtc cagggagttt tacaacaata gatgtattga gaatccaacc taaagcttaa 9240

ctttccactc ccatgaatgc ctctctcctt tttctccatt tataaactga gctattaacc 9300

attaatggtt ccaggtggat gtctcctccc catattacct gatgtatctt acatattgcc 9360

aggctgatat tttaagacat taaaaggtat atttcattat tgagccacat ggtattgatt 9420

actgcttact aaaattttgt cattgtacac atctgtaaaa ggtggttcct tttggaatgc 9480

aaagttcagg tgtttgttgt ctttcctgac ctaaggtctt gtgagcttgt attttttcta 9540

tttaagcagt gctttctctt ggactggctt gactcatggc attctacacg ttattgctgg 9600

tctaaatgtg attttgccaa gcttcttcag gacctataat tttgcttgac ttgtagccaa 9660

acacaagtaa aatgattaag caacaaatgt atttgtgaag cttggttttt aggttgttgt 9720

gttgtgtgtg cttgtgctct ataataatac tatccagggg ctggagaggt ggctcggagt 9780

tcaagagcac agactgctct tccagaagtc ctgagttcaa ttcccagcaa ccacatggtg 9840

gctcacaacc atctgtaatg ggatctgatg ccctcttctg gtgtgtctga agaccacaag 9900

tgtattcaca ttaaataaat aaatcctcct tcttcttctt tttttttttt ttaaagagaa 9960

tactgtctcc agtagaattt actgaagtaa tgaaatactt tgtgtttgtt ccaatatggt 10020

agccaataat caaattactc tttaagcact ggaaatgtta ccaaggaact aatttttatt 10080

tgaagtgtaa ctgtggacag aggagccata actgcagact tgtgggatac agaagaccaa 10140

tgcagacttt aatgtctttt ctcttacact aagcaataaa gaaataaaaa ttgaacttct 10200

agtatcctat ttgtttaaac tgctagcttt acttaacttt tgtgcttcat ctatacaaag 10260

ctgaaagcta agtctgcagc cattactaaa catgaaagca agtaatgata attttggatt 10320

tcaaaaatgt agggccagag tttagccagc cagtggtggt gcttgccttt atgcctttaa 10380

tcccagcact ctggaggcag agacaggcag atctctgagt ttgagcccag cctggtctac 10440

acatcaagtt ctatctagga tagccaggaa tacacacaga aaccctgttg gggagggggg 10500
ctctgagatt tcataaaatt ataattgaag cattccctaa tgagccacta tggatgtggc 10560
taaatccgtc tacctttctg atgagatttg ggtattattt tttctgtctc tgctgttggt 10620
tgggtctttt gacactgtgg gctttcttta aagcctcctt cctgccatgt ggtctcttgt 10680
ttgctactaa cttcccatgg cttaaatggc atggcttttt gccttctaag ggcagctgct 10740
gagatttgca gcctgatttc cagggtgggg ttgggaaatc tttcaaacac taaaattgtc 10800
ctttaatttt ttttttaaaa aatgggttat ataataaacc tcataaaata gttatgagga 10860
gtgaggtgga ctaatattaa atgagtccct cccctataaa agagctatta aggctttttg 10920
tcttatactt aacttttttt ttaaatgtgg tatctttaga accaagggtc ttagagtttt 10980
agtatacaga aactgttgca tcgcttaatc agattttcta gtttcaaatc cagagaatcc 11040
aaattcttca cagccaaagt caaattaaga atttctgact tttaatgtta atttgcttac 11100
tgtgaatata aaaatgatag cttttcctga ggcagggtct cactatgtat ctctgcctga 11160
tctgcaacaa gatatgtaga ctaaagttct gcctgctttt gtctcctgaa tactaaggtt 11220
aaaatgtagt aatacttttg gaacttgcag gtcagattct tttatagggg acacactaag 11280
ggagcttggg tgatagttgg taaaatgtgt ttcaagtgat gaaaacttga attattatca 11340
ccgcaaccta ctttttaaaa aaaaaagcca ggcctgttag agcatgctta agggatccct 11400
aggacttgct gagcacacaa gagtagttac ttggcaggct cctggtgaga gcatatttca 11460
aaaaacaagg cagacaacca agaaactaca gttaaggtta cctgtcttta aaccatctgc 11520
atatacacag ggatattaaa atattccaaa taatatttca ttcaagtttt cccccatcaa 11580
attgggacat ggatttctcc ggtgaatagg cagagttgga aactaaacaa atgttggttt 11640
tgtgatttgt gaaattgttt tcaagtgata gttaaagccc atgagataca gaacaaagct 11700
gctatttcga ggtctcttgg tttatactca gaagcacttc tttgggtttc cctgcactat 11760
cctgatcatg tgctaggcct accttaggct gattgttgtt caaataaact taagtttcct 11820
gtcaggtgat gtcatatgat ttcatatatc aaggcaaaac atgttatata tgttaaacat 11880
ttgtacttaa tgtgaaagtt aggtctttgt gggtttgatt tttaattttc aaaacctgag 11940
ctaaataagt catttttaca tgtcttacat ttggtggaat tgtataattg tggtttgcag 12000
gcaagactct ctgacctagt aaccctacct atagagcact ttgctgggtc acaagtctag 12060
gagtcaagca tttcaccttg aagttgagac gttttgttag tgtatactag tttatatgtt 12120
ggaggacatg tttatccaga agatattcag gactattttt gactgggcta aggaattgat 12180
tctgattagc actgttagtg agcattgagt ggcctttagg cttgaattgg agtcacttgt 12240

atatctcaaa taatgctggc cttttttaaa aagcccttgt tctttatcac cctgttttct 12300

acataatttt tgttcaaaga aatacttgtt tggatctcct tttgacaaca atagcatgtt 12360

ttcaagccat attttttttc cttttttttt ttttttttgg tttttcgaga cagggtttct 12420

ctgtatagcc ctggctgtcc tggaactcac tttgtagacc aggctggcct cgaactcaga 12480

aatccgcctg cctctgcctc ctgagtgccg ggattaaagg cgtgcaccac cacgcctggc 12540

taagttggat attttgttat ataactataa ccaatactaa ctccactggg tggattttta 12600

attcagtcag tagtcttaag tggtctttat tggcccttca ttaaaatcta ctgttcactc 12660

taacagaggc tgttggtact agtggcactt aagcaacttc ctacggatat actagcagat 12720

taagggtcag ggatagaaac tagtctagcg ttttgtatac ctaccagctt tatactacct 12780

tgttctgata gaaatatttc aggacatcta gcacgtgtta actcgagctg caggattcga 12840

gggccccggc aggtcaattc taccgggtag gggaggcgct tttcccaagg cagtctggag 12900

catgcgcttt agcagccccg ctgggcactt ggcgctacac aagtggcctc tggcctcgca 12960

cacattccac atccaccggt aggcgccaac cggctccgtt ctttggtggc cccttcgcgc 13020

caccttctac tcctccccta gtcaggaagt tcccccccgc cccgcagctc gcgtcgtgca 13080

ggacgtgaca aatggaagta gcacgtctca ctagtctcgt gcagatggac agcaccgctg 13140

agcaatggaa gcgggtaggc ctttggggca gcggccaata gcagctttgc tccttcgctt 13200

tctgggctca gaggctggga aggggtgggt ccgggggcgg gctcaggggc gggctcaggg 13260

gcggggcggg cgcccgaagg tcctccggag gcccggcatt ctgcacgctt caaaagcgca 13320

cgtctgccgc gctgttctcc tcttcctcat ctccgggcct ttcgacctgc agccaatgca 13380

ccgtccttgc catcatggcc tcgtaccccg gccatcaaca cgcgtctgcg ttcgaccagg 13440

ctgcgcgttc tcgcggccat agcaaccgac gtacggcgtt gcgccctcgc cggcagcaag 13500

aagccacgga agtccgcccg gagcagaaaa tgcccacgct actgcgggtt tatatagacg 13560

gtccccacgg gatggggaaa accaccacca cgcaactgct ggtggccctg ggttcgcgcg 13620

acgatatcgt ctacgtaccc gagccgatga cttactggcg ggtgctgggg gcttccgaga 13680

caatcgcgaa catctacacc acacaacacc gcctcgacca gggtgagata tcggccgggg 13740

acgcggcggt ggtaatgaca agcgcccaga taacaatggg catgccttat gccgtgaccg 13800

acgccgttct ggctcctcat atcggggggg aggctgggag ctcacatgcc ccgcccccgg 13860

ccctcaccct catcttcgac cgccatccca tcgccgccct cctgtgctac ccggccgcgc 13920

ggtaccttat gggcagcatg accccccagg ccgtgctggc gttcgtggcc ctcatcccgc 13980

cgaccttgcc cggcaccaac atcgtgcttg gggcccttcc ggaggacaga cacatcgacc 14040

gcctggccaa acgccagcgc cccggcgagc ggctggacct ggctatgctg gctgcgattc 14100

gccgcgttta cgggctactt gccaatacgg tgcggtatct gcagtgcggc gggtcgtggc 14160

gggaggactg gggacagctt tcggggacgg ccgtgccgcc ccagggtgcc gagccccaga 14220

gcaacgcggg cccacgaccc catatcgggg acacgttatt taccctgttt cgggcccccg 14280

agttgctggc ccccaacggc gacctgtata acgtgtttgc ctgggccttg gacgtcttgg 14340

ccaaacgcct ccgttccatg cacgtcttta tcctggatta cgaccaatcg cccgccggct 14400

gccgggacgc cctgctgcaa cttacctccg ggatggtcca gacccacgtc accacccccg 14460

gctccatacc gacgatatgc gacctggcgc gcacgtttgc ccgggagatg ggggaggcta 14520

actgagggga tcgatccgtc ctgtaagtct gcagaaattg atgatctatt aaacaataaa 14580

gatgtccact aaaatggaag tttttcctgt catactttgt taagaagggt gagaacagag 14640

tacctacatt ttgaatggaa ggattggagc tacgggggtg ggggtggggt gggattagat 14700

aaatgcctgc tctttactga aggctcttta ctattgcttt atgataatgt ttcatagttg 14760

gatatcataa tttaaacaag caaaaccaaa ttaagggcca gctcattcct cccactcatg 14820

atctatagat ctatagatct ctcgtgggat cattgttttt ctcttgattc ccactttgtg 14880

gttctaagta ctgtggtttc caaatgtgtc agtttcatag cctgaagaac gagatcagca 14940

gcctctgttc cacatacact tcattctcag tattgttttg ccaagttcta attccatcag 15000

aagctgactc taggccgagc tccaattcgc cctatagtga gtcgtattac aattcactgg 15060

ccgtcgtttt acaacgtcgt gactgggaaa accctggcgt tacccaactt aatcgccttg 15120

cagcacatcc ccctttcgcc agctggcgta atagcgaaga ggcccgcacc gatcgccctt 15180

cccaacagtt gcgcagcctg aatggcgaat gggacgcgcc ctgtagcggc gcattaagcg 15240

cggcgggtgt ggtggttacg cgcagcgtga ccgctacact tgccagcgcc ctagcgcccg 15300

ctcctttcgc tttcttccct tcctttctcg ccacgttcgc cggctttccc cgtcaagctc 15360

taaatcgggg gctcccttta gggttccgat ttagtgcttt acggcacctc gaccccaaaa 15420

aacttgatta gggtgatggt tcacgtagtg ggccatcgcc ctgatagacg gtttttcgcc 15480

ctttgacgtt ggagtccacg ttctttaata gtggactctt gttccaaact ggaacaacac 15540

tcaaccctat ctcggtctat tcttttgatt tataagggat tttgccgatt tcggcctatt 15600

ggttaaaaaa tgagctgatt taacaaaaat ttaacgcgaa ttttaacaaa atattaacgc 15660

ttacaattta ggtggcactt ttcggggaaa tgtgcgcgga acccctattt gtttattttt 15720

ctaaatacat tcaaatatgt atccgctcat gagacaataa ccctgataaa tgcttcaata 15780

atattgaaaa aggaagagta tgagtattca acatttccgt gtcgccctta ttcccttttt 15840

tgcggcattt tgccttcctg tttttgctca cccagaaacg ctggtgaaag taaaagatgc 15900

tgaagatcag ttgggtgcac gagtgggtta catcgaactg gatctcaaca gcggtaagat 15960

ccttgagagt tttcgccccg aagaacgttt tccaatgatg agcactttta aagttctgct 16020

atgtggcgcg gtattatccc gtattgacgc cgggcaagag caactcggtc gccgcataca 16080

ctattctcag aatgacttgg ttgagtactc accagtcaca gaaaagcatc ttacggatgg 16140

catgacagta agagaattat gcagtgctgc cataaccatg agtgataaca ctgcggccaa 16200

cttacttctg acaacgatcg gaggaccgaa ggagctaacc gcttttttgc acaacatggg 16260

ggatcatgta actcgccttg atcgttggga accggagctg aatgaagcca taccaaacga 16320

cgagcgtgac accacgatgc ctgtagcaat ggcaacaacg ttgcgcaaac tattaactgg 16380

cgaactactt actctagctt cccggcaaca attaatagac tggatggagg cggataaagt 16440

tgcaggacca cttctgcgct cggcccttcc ggctggctgg tttattgctg ataaatctgg 16500

agccggtgag cgtgggtctc gcggtatcat tgcagcactg gggccagatg gtaagccctc 16560

ccgtatcgta gttatctaca cgacggggag tcaggcaact atggatgaac gaaatagaca 16620

gatcgctgag ataggtgcct cactgattaa gcattggtaa ctgtcagacc aagtttactc 16680

atatatactt tagattgatt taaaacttca tttttaattt aaaaggatct aggtgaagat 16740

cctttttgat aatctcatga ccaaaatccc ttaacgtgag ttttcgttcc actgagcgtc 16800

agaccccgta gaaaagatca aaggatcttc ttgagatcct ttttttctgc gcgtaatctg 16860

ctgcttgcaa acaaaaaaac caccgctacc agcggtggtt tgtttgccgg atcaagagct 16920

accaactctt tttccgaagg taactggctt cagcagagcg cagataccaa atactgtcct 16980

tctagtgtag ccgtagttag gccaccactt caagaactct gtagcaccgc ctacatacct 17040

cgctctgcta atcctgttac cagtggctgc tgccagtggc gataagtcgt gtcttaccgg 17100

gttggactca agacgatagt taccggataa ggcgcagcgg tcgggctgaa cggggggttc 17160

gtgcacacag cccagcttgg agcgaacgac ctacaccgaa ctgagatacc tacagcgtga 17220

gctatgagaa agcgccacgc ttcccgaagg gagaaaggcg gacaggtatc cggtaagcgg 17280

cagggtcgga acaggagagc gcacgaggga gcttccaggg ggaaacgcct ggtatcttta 17340

tagtcctgtc gggtttcgcc acctctgact tgagcgtcga tttttgtgat gctcgtcagg 17400

ggggcggagc ctatggaaaa acgccagcaa cgcggccttt ttacggttcc tggccttttg 17460

ctggcctttt gctcacatgt tctttcctgc gttatcccct gattctgtgg ataaccgtat 17520

taccgccttt gagtgagctg ataccgctcg ccgcagccga acgaccgagc gcagcgagtc 17580

agtgagcgag gaagcggaag agcgcccaat acgcaaaccg cctctccccg cgcgttggcc 17640

gattcattaa tgcagctggc acgacaggtt tcccgactgg aaagcgggca gtgagcgcaa 17700

cgcaattaat gtgagttagc tcactcatta ggcaccccag gctttacact ttatgcttcc 17760

ggctcgtatg ttgtgtggaa ttgtgagcgg ataacaattt cacacaggaa acagctatga 17820

ccatgattac gccaagcgcg caattaaccc tcactaaagg gaacaaaagc tgtcgagatc 17880

tagatatcga tggccataga gttacgc 17907

<210> 3
<211> 16467
<212> DNA
<213> Artificial Sequence

<220>
<223> Description of Artificial Sequence: Targeting vector for rosa26
      locus containing the H1-tet inducible shRNA

<400> 3
ttacgctagg gataacaggg taatatagcc gcggtggcgg ccgctctaga actagtggat 60

cccccgggct gcaggaattc gatatcaagc ttatcgatac cgtcatcgca aggatactgg 120

ggcatacgcc acagggagtc caagaatgtg aggtgggggt ggcgaaggta atgtctttgg 180

tgtgggaaaa gcagcagcca tctgagatag gaactggaaa accagaggag aggcgttcag 240

gaagattatg gaggggagga ctgggccccc acgagcgacc agagttgtca caaggccgca 300

agaacagggg aggtgggggg ctcagggaca gaaaaaaaag tatgtgtatt ttgagagcag 360

ggttgggagg cctctcctga aaagggtata aacgtggagt aggcaatacc caggcaaaaa 420

ggggagacca gagtaggggg aggggaagag tcctgaccca gggaagacat taaaaaggta 480

gtggggtcga ctagatgaag gagagccttt ctctctgggc aagagcggtg caatggtgtg 540

taaaggtagc tgagaagacg aaaagggcaa gcatcttcct gctaccaggc tggggaggcc 600

caggcccacg accccgagga gagggaacgc agggagactg aggtgaccct tctttccccc 660

ggggcccggt cgtgtggttc ggtgtctctt ttctgttgga cccttacctt gacccaggcg 720

ctgccggggc ctgggcccgg gctgcggcgc acggcactcc cgggaggcag cgagactcga 780

gttaggccca acgcggcgcc acggcgtttc ctggccggga atggcccgta cccgtgaggt 840

gggggtgggg ggcagaaaag gcggagcgag cccgagcggg gaggggaggg gccaggggcg 900

gagggggccg gcactactgt gttggcggac tggcgggact agggctgcgt gagtctctga 960

gcgcaggcgg gcggcggccg cccctccccc ggcggcggca gcggcggcag cggcggcagc 1020

tcactcagcc cgctgcccga gcggaaacgc cactgaccgc acggggattc ccagtgccgg 1080

cgccaggggc acgcgggaca cgccccctcc cgccgcgcca ttggcctctc cgcccaccgc 1140

cccacactta ttggccggtg cgccgccaat cagcggaggc tgccggggcc gcctaaagaa 1200

gaggctgtgc tttggggctc cggctcctca gagagcctcg gctaggtagg ggatcgggac 1260

tctggcggga gggcggcttg gtgcgtttgc ggggatgggc ggccgcggca ggccctccga 1320

gcgtggtgga gccgttctgt gagacagccg ggtacgagtc gtgacgctgg aaggggcaag 1380

cgggtggtgg gcaggaatgc ggtccgccct gcagcaaccg gagggggagg gagaagggag 1440

cggaaaagtc tccaccggac gcggccatgg ctcggggggg ggggggcagc ggaggagcgc 1500

ttccggccga cgtctcgtcg ctgattggct tcttttcctc ccgccgtgtg tgaaaacaca 1560

aatggcgtgt tttggttggc gtaaggcgcc tgtcagttaa cggcagccgg agtgcgcagc 1620

cgccggcagc ctcgctctgc ccactgggtg gggcgggagg taggtggggt gaggcgagct 1680

ggacgtgcgg gcgcggtcgg cctctggcgg ggcgggggag gggagggagg gtcagcgaaa 1740

gtagctcgcg cgcgagcggc cgcccaccct ccccttcctc tgggggagtc gttttacccg 1800

ccgccggccg ggcctcgtcg tctgattggc tctcggggcc cagaaaactg gcccttgcca 1860

ttggctcgtg ttcgtgcaag ttgagtccat ccgccggcca gcgggggcgg cgaggaggcg 1920

ctcccaggtt ccggccctcc cctcggcccc gcgccgcaga gtctggccgc gcgcccctgc 1980

gcaacgtggc aggaagcgcg cgctgggggc ggggacgggc agtagggctg agcggctgcg 2040

gggcgggtgc aagcacgttt ccgacttgag ttgcctcaag aggggcgtgc tgagccagac 2100

ctccatcgcg cactccgggg agtggaggga aggagcgagg gctcagttgg gctgttttgg 2160

aggcaggaag cacttgctct cccaaagtcg ctctgagttg ttatcagtaa gggagctgca 2220

gtggagtagg cggggagaag gccgcaccct tctccggagg ggggagggga gtgttgcaat 2280

acctttctgg gagttctctg ctgcctcctg gcttctgagg accgccctgg gcctgggaga 2340

atcccttccc cctcttccct cgtgatctgc aactccagtc tttctaggta accgatatcc 2400

ctgcaggggt gacctgcacg tctagggcgc agtagtccag ggtttccttg atgatgtcat 2460

acttatcctg tccctttttt ttccacagct cgcggttgag gacaaactct tcgcggtctt 2520

tccagtactc ctgcaggtga ctgactgagt cgagatctgc gatctaagta agcttggcat 2580

tccggtactg ttggtaaagc caccatggct tccaaggtgt acgaccccga gcaacgcaaa 2640

cgcatgatca ctgggcctca gtggtgggct cgctgcaagc aaatgaacgt gctggactcc 2700

ttcatcaact actatgattc cgagaagcac gccgagaacg ccgtgatttt tctgcatggt 2760

aacgctgcct ccagctacct gtggaggcac gtcgtgcctc acatcgagcc cgtggctaga 2820

tgcatcatcc ctgatctgat cggaatgggt aagtccggca agagcgggaa tggctcatat 2880

cgcctcctgg atcactacaa gtacctcacc gcttggttcg agctgctgaa ccttccaaag 2940

aaaatcatct ttgtgggcca cgactggggg gcttgtctgg cctttcacta ctcctacgag 3000

caccaagaca agatcaaggc catcgtccat gctgagagtg tcgtggacgt gatcgagtcc 3060

tgggacgagt ggcctgacat cgaggaggat atcgccctga tcaagagcga agagggcgag 3120

aaaatggtgc ttgagaataa cttcttcgtc gagaccatgc tcccaagcaa gatcatgcgg 3180

aaactggagc ctgaggagtt cgctgcctac ctggagccat tcaaggagaa gggcgaggtt 3240

agacggccta ccctctcctg gcctcgcgag atccctctcg ttaagggagg caagcccgac 3300

gtcgtccaga ttgtccgcaa ctacaacgcc taccttcggg ccagcgacga tctgcctaag 3360

atgttcatcg agtccgaccc tgggttcttt tccaacgcta ttgtcgaggg agctaagaag 3420

ttccctaaca ccgagttcgt gaaggtgaag ggcctccact tcagccagga ggacgctcca 3480

gatgaaatgg gtaagtacat caagagcttc gtggagcgcg tgctgaagaa cgagcagtaa 3540

ttctagaccg gttcgagatc caggcgcgga tcaataaaag atcattattt tcaatagatc 3600

tgtgtgttgg ttttttgtgt gccttggggg agggggaggc cagaatgagg cgcggccaag 3660

ggggaggggg aggccagaat gaccttgggg gagggggagg ccagaatgac cttgggggag 3720

ggggaggcca gaatgaggcg cggatccgtc gacttaatta aggccaggga tcttcaagca 3780

gacctacagc aagttcgaca caaactcaca caacgatgac gcactactca agaactacgg 3840

gctgctctac tgcttcagga aggacatgga caaggtcgag acattcctgc gcatcgtgca 3900

gtgccgctct gtggagggca gctgtggctt ctagctgccc gggtggcatc cctgtgaccc 3960

ctccccagtg cctctcctgg ccctggaagt tgccactcca gtgcccacca gccttgtcct 4020

aataaaatta agttgcatca ttttgtctga ctaggtgtcc ttctataata ttatggggtg 4080

gaggggggtg gtatggagca aggggcaagt tgggaagaca acctgtaggg cctgcggggt 4140

ctattgggaa ccaagctgga gtgcagtggc acaatcttgg ctcactgcaa tctccgcctc 4200

ctgggttcaa gcgattctcc tgcctcagcc tcccgagttg ttgggattcc aggcatgcat 4260

gaccaggctc agctaatttt tgtttttttg gtagagacgg ggtttcacca tattggccag 4320

gctggtctcc aactcctaat ctcaggtgat ctacccacct tggcctccca aattgctggg 4380

attacaggcg tgaaccactg ctcccttccc tgtccttctg attttaaaat aactatacca 4440

gcaggaggac gtccagacac agcataggct acctggccat gcccaaccgg tgggacattt 4500

gagttgcttg cttggcactg tcctctcatg cgttgggtcc actcagtaga tgcctgttga 4560

attaagctta tttaaatagg ccggccataa cttcgtataa tgtatgctat acgaagttat 4620

ggatcctcac agtaggtggc atcgttcctt tctgactgcc cgccccccgc atgccgtccc 4680

gcgatattga gctccgaacc tctcgccctg ccgccgccgg tgctccgtcg ccgccgcgcc 4740

gccatggaat tcgaacgctg acgtcatcaa cccgctccaa ggaatcgcgg gcccagtgtc 4800

actaggcggg aacacccagc gcgcgtgcgc cctggcagga agatggctgt gagggacagg 4860

ggagtggcgc cctgcaatat ttgcatgtcg ctatgtgttc tgggaaatca ccataaacgt 4920

gaaatgtctt tggatttggg aatcttataa gtccctatca gtgatagaga ttcccaggat 4980

tccaattcag cgggagccac ctgatgaagc ttgatcgggt ggctctcgct gagttggaat 5040

ccattttttt ctaggataac ttcgtataat gtatgctata cgaagttatg gcgcgccggt 5100

aaccgaagtt cctatacttt ctagagaata ggaacttcgg aataggaact tcttaggtca 5160

attctaccgg gtaggggagg cgcttttccc aaggcagtct ggagcatgcg ctttagcagc 5220

cccgctgggc acttggcgct acacaagtgg cctctggcct cgcacacatt ccacatccac 5280

cggtaggcgc caaccggctc cgttctttgg tggccccttc gcgccacctt ctactcctcc 5340

cctagtcagg aagttccccc ccgccccgca gctcgcgtcg tgcaggacgt gacaaatgga 5400

agtagcacgt ctcactagtc tcgtgcagat ggacagcacc gctgagcaat ggaagcgggt 5460

aggcctttgg ggcagcggcc aatagcagct ttgctccttc gctttctggg ctcagaggct 5520

gggaaggggt gggtccgggg gcgggctcag gggcgggctc aggggcgggg cgggcgcccg 5580

aaggtcctcc ggaggcccgg cattctgcac gcttcaaaag cgcacgtctg ccgcgctgtt 5640

ctcctcttcc tcatctccgg gcctttcgac ctgcagccaa tatgggatcg gccattgaac 5700

aagatggatt gcacgcaggt tctccggccg cttgggtgga gaggctattc ggctatgact 5760

gggcacaaca gacaatcggc tgctctgatg ccgccgtgtt ccggctgtca gcgcaggggc 5820

gcccggttct ttttgtcaag accgacctgt ccggtgccct gaatgaactg caggacgagg 5880

cagcgcggct atcgtggctg gccacgacgg gcgttccttg cgcagctgtg ctcgacgttg 5940

tcactgaagc gggaagggac tggctgctat tgggcgaagt gccggggcag gatctcctgt 6000

catctcacct tgctcctgcc gagaaagtat ccatcatggc tgatgcaatg cggcggctgc 6060

atacgcttga tccggctacc tgcccattcg accaccaagc gaaacatcgc atcgagcgag 6120

cacgtactcg gatggaagcc ggtcttgtcg atcaggatga tctggacgaa gagcatcagg 6180

ggctcgcgcc agccgaactg ttcgccaggc tcaaggcgcg catgcccgac ggcgaggatc 6240

tcgtcgtgac ccatggcgat gcctgcttgc cgaatatcat ggtggaaaat ggccgctttt 6300

ctggattcat cgactgtggc cggctgggtg tggcggaccg ctatcaggac atagcgttgg 6360

ctacccgtga tattgctgaa gagcttggcg gcgaatgggc tgaccgcttc ctcgtgcttt 6420

acggtatcgc cgctcccgat tcgcagcgca tcgccttcta tcgccttctt gacgagttct 6480

tctgagggga tcgatccgct gtaagtctgc agaaattgat gatctattaa acaataaaga 6540

tgtccactaa aatggaagtt tttcctgtca tactttgtta agaagggtga gaacagagta 6600

cctacatttt gaatggaagg attggagcta cgggggtggg ggtggggtgg gattagataa 6660

atgcctgctc tttactgaag gctctttact attgctttat gataatgttt catagttgga 6720

tatcataatt taaacaagca aaaccaaatt aagggccagc tcattcctcc cactcatgat 6780

ctatagatct atagatctct cgtgggatca ttgtttttct cttgattccc actttgtggt 6840

tctaagtact gtggtttcca aatgtgtcag tttcatagcc tgaagaacga gatcagcagc 6900

ctctgttcca catacacttc attctcagta ttgttttgcc aagttctaat tccatcagaa 6960

gctgactcta gatcccgcgc cgaagttcct atactttcta gagaatagga acttcggaat 7020

aggaacttca agcttaagcg ctagaagatg ggcgggagtc ttctgggcag gcttaaaggc 7080

taacctggtg tgtgggcgtt gtcctgcagg ggaattgaac aggtgtaaaa ttggagggac 7140

aagacttccc acagattttc ggttttgtcg ggaagttttt taataggggc aaataaggaa 7200

aatgggagga taggtagtca tctggggttt tatgcagcaa aactacaggt tattattgct 7260

tgtgatccgc ctcggagtat tttccatcga ggtagattaa agacatgctc acccgagttt 7320

tatactctcc tgcttgagat ccttactaca gtatgaaatt acagtgtcgc gagttagact 7380

atgtaagcag aattttaatc atttttaaag agcccagtac ttcatatcca tttctcccgc 7440

tccttctgca gccttatcaa aaggtatttt agaacactca ttttagcccc attttcattt 7500

attatactgg cttatccaac ccctagacag agcattggca ttttcccttt cctgatctta 7560

gaagtctgat gactcatgaa accagacaga ttagttacat acaccacaaa tcgaggctgt 7620

agctggggcc tcaacactgc agttctttta taactcctta gtacactttt tgttgatcct 7680

ttgccttgat ccttaatttt cagtgtctat cacctctccc gtcagtggtg ttccacattt 7740

gggcctattc tcagtccagg gagttttaca acaatagatg tattgagaat ccaacctaaa 7800

gcttaacttt ccactcccat gaatgcctct ctcctttttc tccatttata aactgagcta 7860

ttaaccatta atggttccag gtggatgtct cctccccata ttacctgatg tatcttacat 7920

attgccaggc tgatatttta agacattaaa aggtatattt cattattgag ccacatggta 7980

ttgattactg cttactaaaa ttttgtcatt gtacacatct gtaaaaggtg gttccttttg 8040

gaatgcaaag ttcaggtgtt tgttgtcttt cctgacctaa ggtcttgtga gcttgtattt 8100

tttctattta agcagtgctt tctcttggac tggcttgact catggcattc tacacgttat 8160

tgctggtcta aatgtgattt tgccaagctt cttcaggacc tataattttg cttgacttgt 8220

agccaaacac aagtaaaatg attaagcaac aaatgtattt gtgaagcttg gtttttaggt 8280

tgttgtgttg tgtgtgcttg tgctctataa taatactatc caggggctgg agaggtggct 8340

cggagttcaa gagcacagac tgctcttcca gaagtcctga gttcaattcc cagcaaccac 8400

atggtggctc acaaccatct gtaatgggat ctgatgccct cttctggtgt gtctgaagac 8460

cacaagtgta ttcacattaa ataaataaat cctccttctt cttctttttt ttttttttaa 8520

agagaatact gtctccagta gaatttactg aagtaatgaa atactttgtg tttgttccaa 8580

tatggtagcc aataatcaaa ttactcttta agcactggaa atgttaccaa ggaactaatt 8640

tttatttgaa gtgtaactgt ggacagagga gccataactg cagacttgtg ggatacagaa 8700

gaccaatgca gactttaatg tcttttctct tacactaagc aataaagaaa taaaaattga 8760

acttctagta tcctatttgt ttaaactgct agctttactt aacttttgtg cttcatctat 8820

acaaagctga aagctaagtc tgcagccatt actaaacatg aaagcaagta atgataattt 8880

tggatttcaa aaatgtaggg ccagagttta gccagccagt ggtggtgctt gcctttatgc 8940

ctttaatccc agcactctgg aggcagagac aggcagatct ctgagtttga gcccagcctg 9000

gtctacacat caagttctat ctaggatagc caggaataca cacagaaacc ctgttgggga 9060

ggggggctct gagatttcat aaaattataa ttgaagcatt ccctaatgag ccactatgga 9120

tgtggctaaa tccgtctacc tttctgatga gatttgggta ttattttttc tgtctctgct 9180

gttggttggg tcttttgaca ctgtgggctt tctttaaagc ctccttcctg ccatgtggtc 9240

tcttgtttgc tactaacttc ccatggctta aatggcatgg ctttttgcct tctaagggca 9300

gctgctgaga tttgcagcct gatttccagg gtggggttgg gaaatctttc aaacactaaa 9360

attgtccttt aatttttttt ttaaaaaatg ggttatataa taaacctcat aaaatagtta 9420

tgaggagtga ggtggactaa tattaaatga gtccctcccc tataaaagag ctattaaggc 9480

tttttgtctt atacttaact ttttttttaa atgtggtatc tttagaacca agggtcttag 9540

agttttagta tacagaaact gttgcatcgc ttaatcagat tttctagttt caaatccaga 9600

gaatccaaat tcttcacagc caaagtcaaa ttaagaattt ctgactttta atgttaattt 9660

gcttactgtg aatataaaaa tgatagcttt tcctgaggca gggtctcact atgtatctct 9720

gcctgatctg caacaagata tgtagactaa agttctgcct gcttttgtct cctgaatact 9780

aaggttaaaa tgtagtaata cttttggaac ttgcaggtca gattctttta taggggacac 9840

actaagggag cttgggtgat agttggtaaa atgtgtttca agtgatgaaa acttgaatta 9900

ttatcaccgc aacctacttt ttaaaaaaaa aagccaggcc tgttagagca tgcttaaggg 9960

atccctagga cttgctgagc acacaagagt agttacttgg caggctcctg gtgagagcat 10020

atttcaaaaa acaaggcaga caaccaagaa actacagtta aggttacctg tctttaaacc 10080

atctgcatat acacagggat attaaaatat tccaaataat atttcattca agttttcccc 10140

catcaaattg ggacatggat ttctccggtg aataggcaga gttggaaact aaacaaatgt 10200

tggttttgtg atttgtgaaa ttgttttcaa gtgatagtta aagcccatga gatacagaac 10260

aaagctgcta tttcgaggtc tcttggttta tactcagaag cacttctttg ggtttccctg 10320

cactatcctg atcatgtgct aggcctacct taggctgatt gttgttcaaa taaacttaag 10380

tttcctgtca ggtgatgtca tatgatttca tatatcaagg caaaacatgt tatatatgtt 10440

aaacatttgt acttaatgtg aaagttaggt ctttgtgggt ttgattttta attttcaaaa 10500

cctgagctaa ataagtcatt tttacatgtc ttacatttgg tggaattgta taattgtggt 10560

ttgcaggcaa gactctctga cctagtaacc ctacctatag agcactttgc tgggtcacaa 10620

gtctaggagt caagcatttc accttgaagt tgagacgttt tgttagtgta tactagttta 10680

tatgttggag gacatgttta tccagaagat attcaggact atttttgact gggctaagga 10740

attgattctg attagcactg ttagtgagca ttgagtggcc tttaggcttg aattggagtc 10800

acttgtatat ctcaaataat gctggccttt tttaaaaagc ccttgttctt tatcaccctg 10860

ttttctacat aatttttgtt caaagaaata cttgtttgga tctccttttg acaacaatag 10920

catgttttca agccatattt tttttccttt tttttttttt ttttggtttt tcgagacagg 10980

gtttctctgt atagccctgg ctgtcctgga actcactttg tagaccaggc tggcctcgaa 11040

ctcagaaatc cgcctgcctc tgcctcctga gtgccgggat taaaggcgtg caccaccacg 11100

cctggctaag ttggatattt tgttatataa ctataaccaa tactaactcc actgggtgga 11160

tttttaattc agtcagtagt cttaagtggt ctttattggc ccttcattaa aatctactgt 11220

tcactctaac agaggctgtt ggtactagtg gcacttaagc aacttcctac ggatatacta 11280

gcagattaag ggtcagggat agaaactagt ctagcgtttt gtatacctac cagctttata 11340

ctaccttgtt ctgatagaaa tatttcagga catctagcac gtgttaactc gagctgcagg 11400

attcgagggc cccggcaggt caattctacc gggtagggga ggcgcttttc ccaaggcagt 11460

ctggagcatg cgctttagca gccccgctgg gcacttggcg ctacacaagt ggcctctggc 11520

ctcgcacaca ttccacatcc accggtaggc gccaaccggc tccgttcttt ggtggcccct 11580

tcgcgccacc ttctactcct cccctagtca ggaagttccc ccccgccccg cagctcgcgt 11640

cgtgcaggac gtgacaaatg gaagtagcac gtctcactag tctcgtgcag atggacagca 11700

ccgctgagca atggaagcgg gtaggccttt ggggcagcgg ccaatagcag ctttgctcct 11760

tcgctttctg ggctcagagg ctgggaaggg gtgggtccgg gggcgggctc aggggcgggc 11820

tcaggggcgg ggcgggcgcc cgaaggtcct ccggaggccc ggcattctgc acgcttcaaa 11880

agcgcacgtc tgccgcgctg ttctcctctt cctcatctcc gggcctttcg acctgcagcc 11940

aatgcaccgt ccttgccatc atggcctcgt accccggcca tcaacacgcg tctgcgttcg 12000

accaggctgc gcgttctcgc ggccatagca accgacgtac ggcgttgcgc cctcgccggc 12060

agcaagaagc cacggaagtc cgcccggagc agaaaatgcc cacgctactg cgggtttata 12120

tagacggtcc ccacgggatg gggaaaacca ccaccacgca actgctggtg gccctgggtt 12180

cgcgcgacga tatcgtctac gtacccgagc cgatgactta ctggcgggtg ctgggggctt 12240

ccgagacaat cgcgaacatc tacaccacac aacaccgcct cgaccagggt gagatatcgg 12300

ccggggacgc ggcggtggta atgacaagcg cccagataac aatgggcatg ccttatgccg 12360

tgaccgacgc cgttctggct cctcatatcg ggggggaggc tgggagctca catgccccgc 12420

ccccggccct caccctcatc ttcgaccgcc atcccatcgc cgccctcctg tgctacccgg 12480

ccgcgcggta ccttatgggc agcatgaccc cccaggccgt gctggcgttc gtggccctca 12540

tcccgccgac cttgcccggc accaacatcg tgcttggggc ccttccggag gacagacaca 12600

tcgaccgcct ggccaaacgc cagcgccccg gcgagcggct ggacctggct atgctggctg 12660

cgattcgccg cgtttacggg ctacttgcca atacggtgcg gtatctgcag tgcggcgggt 12720

cgtggcggga ggactgggga cagctttcgg ggacggccgt gccgccccag ggtgccgagc 12780

cccagagcaa cgcgggccca cgaccccata tcggggacac gttatttacc ctgtttcggg 12840

cccccgagtt gctggccccc aacggcgacc tgtataacgt gtttgcctgg gccttggacg 12900

tcttggccaa acgcctccgt tccatgcacg tctttatcct ggattacgac caatcgcccg 12960

ccggctgccg ggacgccctg ctgcaactta cctccgggat ggtccagacc cacgtcacca 13020

cccccggctc cataccgacg atatgcgacc tggcgcgcac gtttgcccgg gagatggggg 13080

aggctaactg aggggatcga tccgtcctgt aagtctgcag aaattgatga tctattaaac 13140

aataaagatg tccactaaaa tggaagtttt tcctgtcata ctttgttaag aagggtgaga 13200

acagagtacc tacattttga atggaaggat tggagctacg ggggtggggg tggggtggga 13260

ttagataaat gcctgctctt tactgaaggc tctttactat tgctttatga taatgtttca 13320

tagttggata tcataattta aacaagcaaa accaaattaa gggccagctc attcctccca 13380

ctcatgatct atagatctat agatctctcg tgggatcatt gtttttctct tgattcccac 13440

tttgtggttc taagtactgt ggtttccaaa tgtgtcagtt tcatagcctg aagaacgaga 13500

tcagcagcct ctgttccaca tacacttcat tctcagtatt gttttgccaa gttctaattc 13560

catcagaagc tgactctagg ccgagctcca attcgcccta tagtgagtcg tattacaatt 13620

cactggccgt cgttttacaa cgtcgtgact gggaaaaccc tggcgttacc caacttaatc 13680

gccttgcagc acatccccct ttcgccagct ggcgtaatag cgaagaggcc cgcaccgatc 13740

gcccttccca acagttgcgc agcctgaatg gcgaatggga cgcgccctgt agcggcgcat 13800

taagcgcggc gggtgtggtg gttacgcgca gcgtgaccgc tacacttgcc agcgccctag 13860

cgcccgctcc tttcgctttc ttcccttcct ttctcgccac gttcgccggc tttccccgtc 13920

aagctctaaa tcgggggctc cctttagggt tccgatttag tgctttacgg cacctcgacc 13980

ccaaaaaact tgattagggt gatggttcac gtagtgggcc atcgccctga tagacggttt 14040

ttcgcccttt gacgttggag tccacgttct ttaatagtgg actcttgttc caaactggaa 14100

caacactcaa ccctatctcg gtctattctt ttgatttata agggattttg ccgatttcgg 14160

cctattggtt aaaaaatgag ctgatttaac aaaaatttaa cgcgaatttt aacaaaatat 14220

taacgcttac aatttaggtg gcacttttcg gggaaatgtg cgcggaaccc ctatttgttt 14280

atttttctaa atacattcaa atatgtatcc gctcatgaga caataaccct gataaatgct 14340

tcaataatat tgaaaaagga agagtatgag tattcaacat ttccgtgtcg cccttattcc 14400

cttttttgcg gcattttgcc ttcctgtttt tgctcaccca gaaacgctgg tgaaagtaaa 14460

agatgctgaa gatcagttgg gtgcacgagt gggttacatc gaactggatc tcaacagcgg 14520

taagatcctt gagagttttc gccccgaaga acgttttcca atgatgagca cttttaaagt 14580

tctgctatgt ggcgcggtat tatcccgtat tgacgccggg caagagcaac tcggtcgccg 14640

catacactat tctcagaatg acttggttga gtactcacca gtcacagaaa agcatcttac 14700

ggatggcatg acagtaagag aattatgcag tgctgccata accatgagtg ataacactgc 14760

ggccaactta cttctgacaa cgatcggagg accgaaggag ctaaccgctt ttttgcacaa 14820

catgggggat catgtaactc gccttgatcg ttgggaaccg gagctgaatg aagccatacc 14880

aaacgacgag cgtgacacca cgatgcctgt agcaatggca acaacgttgc gcaaactatt 14940

aactggcgaa ctacttactc tagcttcccg gcaacaatta atagactgga tggaggcgga 15000

taaagttgca ggaccacttc tgcgctcggc ccttccggct ggctggttta ttgctgataa 15060

atctggagcc ggtgagcgtg ggtctcgcgg tatcattgca gcactggggc cagatggtaa 15120

gccctcccgt atcgtagtta tctacacgac ggggagtcag gcaactatgg atgaacgaaa 15180

tagacagatc gctgagatag gtgcctcact gattaagcat tggtaactgt cagaccaagt 15240

ttactcatat atactttaga ttgatttaaa acttcatttt taatttaaaa ggatctaggt 15300

gaagatcctt tttgataatc tcatgaccaa aatcccttaa cgtgagtttt cgttccactg 15360

agcgtcagac cccgtagaaa agatcaaagg atcttcttga gatccttttt ttctgcgcgt 15420

aatctgctgc ttgcaaacaa aaaaaccacc gctaccagcg gtggtttgtt tgccggatca 15480

agagctacca actctttttc cgaaggtaac tggcttcagc agagcgcaga taccaaatac 15540

tgtccttcta gtgtagccgt agttaggcca ccacttcaag aactctgtag caccgcctac 15600

atacctcgct ctgctaatcc tgttaccagt ggctgctgcc agtggcgata agtcgtgtct 15660

taccgggttg gactcaagac gatagttacc ggataaggcg cagcggtcgg gctgaacggg 15720

gggttcgtgc acacagccca gcttggagcg aacgacctac accgaactga gatacctaca 15780

gcgtgagcta tgagaaagcg ccacgcttcc cgaagggaga aaggcggaca ggtatccggt 15840

aagcggcagg gtcggaacag gagagcgcac gagggagctt ccagggggaa acgcctggta 15900

tctttatagt cctgtcgggt ttcgccacct ctgacttgag cgtcgatttt tgtgatgctc 15960

gtcagggggg cggagcctat ggaaaaacgc cagcaacgcg gcctttttac ggttcctggc 16020

cttttgctgg ccttttgctc acatgttctt tcctgcgtta tcccctgatt ctgtggataa 16080

ccgtattacc gcctttgagt gagctgatac cgctcgccgc agccgaacga ccgagcgcag 16140

cgagtcagtg agcgaggaag cggaagagcg cccaatacgc aaaccgcctc tccccgcgcg 16200

ttggccgatt cattaatgca gctggcacga caggtttccc gactggaaag cgggcagtga 16260

gcgcaacgca attaatgtga gttagctcac tcattaggca ccccaggctt tacactttat 16320

gcttccggct cgtatgttgt gtggaattgt gagcggataa caatttcaca caggaaacag 16380

ctatgaccat gattacgcca agcgcgcaat taaccctcac taaagggaac aaaagctgtc 16440

gagatctaga tatcgatggc catagag 16467

<210> 4
<211> 23
<212> DNA
<213> Artificial Sequence

<220>
<223> Description of Artificial Sequence: Primer Rscreen1s

<400> 4
gacaggacag tgcttgttta agg 23

<210> 5
<211> 23
<212> DNA
<213> Artificial Sequence

<220>
<223> Description of Artificial Sequence: Primer Rscreen1as

<400> 5
tgactacaca atattgctcg cac 23

<210> 6
<211> 1073
<212> DNA
<213> Artificial Sequence

<220>
<223> Description of Artificial Sequence: 5' arm for Rosa26

<400> 6
caggccctcc gagcgtggtg gagccgttct gtgagacagc cgggtacgag tcgtgacgct 60

ggaaggggca agcgggtggt gggcaggaat gcggtccgcc ctgcagcaac cggaggggga 120

gggagaaggg agcggaaaag tctccaccgg acgcggccat ggctcggggg ggggggggca 180

gcggaggagc gcttccggcc gacgtctcgt cgctgattgg cttcttttcc tcccgccgtg 240

tgtgaaaaca caaatggcgt gttttggttg gcgtaaggcg cctgtcagtt aacggcagcc 300

ggagtgcgca gccgccggca gcctcgctct gcccactggg tggggcggga ggtaggtggg 360

gtgaggcgag ctggacgtgc gggcgcggtc ggcctctggc ggggcggggg aggggaggga 420

gggtcagcga aagtagctcg cgcgcgagcg gccgcccacc ctccccttcc tctgggggag 480

tcgttttacc cgccgccggc cgggcctcgt cgtctgattg gctctcgggg cccagaaaac 540

tggcccttgc cattggctcg tgttcgtgca agttgagtcc atccgccggc cagcgggggc 600

ggcgaggagg cgctcccagg ttccggccct cccctcggcc ccgcgccgca gagtctggcc 660

gcgcgcccct gcgcaacgtg gcaggaagcg cgcgctgggg gcggggacgg gcagtagggc 720

tgagcggctg cggggcgggt gcaagcacgt ttccgacttg agttgcctca agaggggcgt 780

gctgagccag acctccatcg cgcactccgg ggagtggagg gaaggagcga gggctcagtt 840

gggctgtttt ggaggcagga agcacttgct ctcccaaagt cgctctgagt tgttatcagt 900

aagggagctg cagtggagta ggcggggaga aggccgcacc cttctccgga gggggagggg 960

gagtgttgca atacctttct gggagttctc tgctgcctcc tggcttctga ggaccgccct 1020

gggcctggga gaatcccttc cccctcttcc ctcgtgatct gcaactccag tct 1073

<210> 7
<211> 4333
<212> DNA
<213> Artificial Sequence

<220>
<223> Description of Artificial Sequence: 3' arm for Rosa26

<400> 7
tagaagatgg gcgggagtct tctgggcagg cttaaaggct aacctggtgt gtgggcgttg 60

tcctgcaggg gaattgaaca ggtgtaaaat tggagggaca agacttccca cagattttcg 120

gttttgtcgg gaagtttttt aataggggca aataaggaaa atgggaggat aggtagtcat 180

ctggggtttt atgcagcaaa actacaggtt attattgctt gtgatccgcc tcggagtatt 240

ttccatcgag gtagattaaa gacatgctca cccgagtttt atactctcct gcttgagatc 300

cttactacag tatgaaatta cagtgtcgcg agttagacta tgtaagcaga attttaatca 360

tttttaaaga gcccagtact tcatatccat ttctcccgct ccttctgcag ccttatcaaa 420

aggtatttta gaacactcat tttagcccca ttttcattta ttatactggc ttatccaacc 480

cctagacaga gcattggcat tttccctttc ctgatcttag aagtctgatg actcatgaaa 540

ccagacagat tagttacata caccacaaat cgaggctgta gctggggcct caacactgca 600

gttcttttat aactccttag tacacttttt gttgatcctt tgccttgatc cttaattttc 660

agtgtctatc acctctcccg tcagtggtgt tccacatttg ggcctattct cagtccaggg 720

agttttacaa caatagatgt attgagaatc caacctaaag cttaactttc cactcccatg 780
aatgcctctc tcctttttct ccatttataa actgagctat taaccattaa tggttccagg 840
tggatgtctc ctccccatat tacctgatgt atcttacata ttgccaggct gatattttaa 900
gacattaaaa ggtatatttc attattgagc cacatggtat tgattactgc ttactaaaat 960
tttgtcattg tacacatctg taaaaggtgg ttccttttgg aatgcaaagt tcaggtgttt 1020
gttgtctttc ctgacctaag gtcttgtgag cttgtatttt ttctatttaa gcagtgcttt 1080
ctcttggact ggcttgactc atggcattct acacgttatt gctggtctaa atgtgatttt 1140
gccaagcttc ttcaggacct ataattttgc ttgacttgta gccaaacaca agtaaaatga 1200
ttaagcaaca aatgtatttg tgaagcttgg tttttaggtt gttgtgttgt gtgtgcttgt 1260
gctctataat aatactatcc aggggctgga gaggtggctc ggagttcaag agcacagact 1320
gctcttccag aagtcctgag ttcaattccc agcaaccaca tggtggctca caaccatctg 1380
taatgggatc tgatgccctc ttctggtgtg tctgaagacc acaagtgtat tcacattaaa 1440
taaataaatc ctccttcttc ttcttttttt tttttttaaa gagaatactg tctccagtag 1500
aatttactga agtaatgaaa tactttgtgt ttgttccaat atggtagcca ataatcaaat 1560
tactctttaa gcactggaaa tgttaccaag gaactaattt ttatttgaag tgtaactgtg 1620
gacagaggag ccataactgc agacttgtgg gatacagaag accaatgcag actttaatgt 1680
cttttctctt acactaagca ataaagaaat aaaaattgaa cttctagtat cctatttgtt 1740
taaactgcta gctttactta acttttgtgc ttcatctata caaagctgaa agctaagtct 1800
gcagccatta ctaaacatga aagcaagtaa tgataatttt ggatttcaaa aatgtagggc 1860
cagagtttag ccagccagtg gtggtgcttg cctttatgcc tttaatccca gcactctgga 1920
ggcagagaca ggcagatctc tgagtttgag cccagcctgg tctacacatc aagttctatc 1980
taggatagcc aggaatacac acagaaaccc tgttggggag gggggctctg agatttcata 2040
aaattataat tgaagcattc cctaatgagc cactatggat gtggctaaat ccgtctacct 2100
ttctgatgag atttgggtat tattttttct gtctctgctg ttggttgggt cttttgacac 2160
tgtgggcttt ctttaaagcc tccttcctgc catgtggtct cttgtttgct actaacttcc 2220
catggcttaa atggcatggc tttttgcctt ctaagggcag ctgctgagat ttgcagcctg 2280
atttccaggg tggggttggg aaatctttca aacactaaaa ttgtccttta attttttttt 2340
taaaaaatgg gttatataat aaacctcata aaatagttat gaggagtgag gtggactaat 2400
attaaatgag tccctcccct ataaaagagc tattaaggct tttgtcttta tacttaactt 2460
tttttttaaa tgtggtatct ttagaaccaa gggtcttaga gttttagtat acagaaactg 2520

ttgcatcgct taatcagatt ttctagtttc aaatccagag aatccaaatt cttcacagcc 2580

aaagtcaaat taagaatttc tgacttttaa tgttaatttg cttactgtga atataaaaat 2640

gatagctttt cctgaggcag ggtctcacta tgtatctctg cctgatctgc aacaagatat 2700

gtagactaaa gttctgcctg cttttgtctc ctgaatacta aggttaaaat gtagtaatac 2760

ttttggaact tgcaggtcag attcttttat aggggacaca ctaagggagc ttgggtgata 2820

gttggtaaaa tgtgtttcaa gtgatgaaaa cttgaattat tatcaccgca acctactttt 2880

taaaaaaaaa agccaggcct gttagagcat gcttaaggga tccctaggac ttgctgagca 2940

cacaagagta gttacttggc aggctcctgg tgagagcata tttcaaaaaa caaggcagac 3000

aaccaagaaa ctacagttaa ggttacctgt ctttaaacca tctgcatata cacagggata 3060

ttaaaatatt ccaaataata tttcattcaa gttttccccc atcaaattgg gacatggatt 3120

tctccggtga ataggcagag ttggaaacta aacaaatgtt ggttttgtga tttgtgaaat 3180

tgttttcaag tgatagttaa agcccatgag atacagaaca aagctgctat ttcgaggtct 3240

cttggtttat actcagaagc acttctttgg gtttccctgc actatcctga tcatgtgcta 3300

ggcctacctt aggctgattg ttgttcaaat aaacttaagt ttcctgtcag gtgatgtcat 3360

atgatttcat atatcaaggc aaaacatgtt atatatgtta aacatttgta cttaatgtga 3420

aagttaggtc tttgtgggtt tgatttttaa ttttcaaaac ctgagctaaa taagtcattt 3480

ttacatgtct tacatttggt ggaattgtat aattgtggtt tgcaggcaag actctctgac 3540

ctagtaaccc tacctataga gcactttgct gggtcacaag tctaggagtc aagcatttca 3600

ccttgaagtt gagacgtttt gttagtgtat actagtttat atgttggagg acatgtttat 3660

ccagaagata ttcaggacta tttttgactg ggctaaggaa ttgattctga ttagcactgt 3720

tagtgagcat tgagtggcct ttaggcttga attggagtca cttgtatatc tcaaataatg 3780

ctggcctttt ttaaaaagcc cttgttcttt atcaccctgt tttctacata atttttgttc 3840

aaagaaatac ttgtttggat ctccttttga caacaatagc atgttttcaa gccatatttt 3900

ttttcctttt tttttttttt tttggttttt cgagacaggg tttctctgta tagccctggc 3960

tgtcctggaa ctcactttgt agaccaggct ggcctcgaac tcagaaatcc gcctgcctct 4020

gcctcctgag tgccgggatt aaaggcgtgc accaccacgc ctggctaagt tggatatttt 4080

gttatataac tataaccaat actaactcca ctgggtggat ttttaattca gtcagtagtc 4140

ttaagtggtc tttattggcc cttcattaaa atctactgtt cactctaaca gaggctgttg 4200

gtactagtgg cacttaagca acttcctacg gatatactag cagattaagg gtcagggata 4260

gaaactagtc tagcgttttg tatacctacc agctttatac taccttgttc tgatagaaat 4320

atttcaggac atc 4333

<210> 8
<211> 75
<212> DNA
<213> Artificial Sequence

<220>
<223> Description of Artificial Sequence: Firefly luciferase-specific
      shRNA

<400> 8
aggattccaa ttcagcggga gccacctgat gaagcttgat cgggtggctc tcgctgagtt     60

ggaatccatt ttttt                                                      75


<210> 9
<211> 25
<212> DNA
<213> Artificial Sequence

<220>
<223> Description of Artificial Sequence: Primer

<400> 9
atcgaattca ccatgtccag actgg                                           25


<210> 10
<211> 34
<212> DNA
<213> Artificial Sequence

<220>
<223> Description of Artificial Sequence: Primer

<400> 10
ataggatcct taagagccag actcacattt cagc                                 34


<210> 11
<211> 13139
<212> DNA
<213> Artificial Sequence

<220>
<223> Description of Artificial Sequence: Murine Rosa26 locus

<400> 11
   aagcttctca cgtagcaacc agagctccag agccagcagc tgctgccgcc ttgtatactc     60
   actcctgtga tccaacacag gagcaacctt ttctttaccc cacccccact tcttaacaca    120
   cttttttttg gggggggggg gggaacaagt gctccatgct ggaaggattg gaactatgct    180
   tttagaaagg aacaatccta aggtcacttt taaattgagg tctttgattt gaaaatcaac    240
   aaataccaaa ttccaaatat tcgttttaat taaaccagca atgtggatat aagcattaag    300
   ttttagtttt aaaaaggtca attttccaaa cattcagcaa tcatatttaa atttacagct    360
   aggaacaaga gccttgggtc atgtcctacc aaagaacata actcaatatt ctacacatga 420
   caatctgaat aaccttaaag cctctaatcc cataacaggc cacaaatttt ggacagagaa 480
   ctaatgatcc tcctgagaaa actggaagaa atccagggaa aagaaattcc tgtgtcctcc 540
   aaactcagaa atctctaatt atgtcagtat tctctgcttt agtcctaggt cagattgcac 600
   acatctaaaa taacctctta aagttttcct cctagcgacc taaaccatta ttaatatcaa 660
   attaaccatc aaaacacttt cctctcaata tgctgcacac aaacctcctc ctggaacctc 720
   ctccatctgg atcctcccca atcaaaagta taggtattta acatataagc aaggaagtaa 780
   tgtaaacatg accttggtca caaatatgtc atctaaaaac aatttagtca aggtatggag 840
   gaaattcgag aacctgaatc tttttaagta ttttgagcac aggaacaatt ggcaaaagga 900
   atccaggtat agacaaaacc cagagcccag agctctgggc gaaaaatgag ttgctggtga 960
   agacgttaca caagtaacat gagaaagcag aaaatgcagg tcatccacgc acccctgacc 1020
   caggccagca gggcgggctg cagcatcagt acacaggaga aagatcctta ttcctaagaa 1080
   tgagaaaggc aaaggcgccc gatagaataa attagcatag aaggggcttt cccaggagtt 1140
   aaaactttcc ttctgagcga ttacctacta aaaccagggc ttttgcccac taccatttac 1200
   ctaggatctt ggcttgcacg gattcatagg ggcatatccc tccccctctt ctttagagtc 1260
   gttcttaaaa gatcgctctc cacgccctag gcagggaaaa cgacaaaatc tggctcaatt 1320
   ccaggctaga accctacaaa ttcaacaggg atatcgcaag gatactgggg catacgccac 1380
   agggagtcca agaatgtgag gtgggggtgg cgaaggtaat gtctttggtg tgggaaaagc 1440
   agcagccatc tgagatagga actggaaaac cagaggagag gcgttcagga agattatgga 1500
   ggggaggact gggcccccac gagcgaccag agttgtcaca aggccgcaag aacaggggag 1560
   gtggggggct cagggacaga aaaaaaagta tgtgtatttt gagagcaggg ttgggaggcc 1620
   tctcctgaaa agggtataaa cgtggagtag gcaataccca ggcaaaaagg ggagaccaga 1680
   gtagggggag gggaagagtc ctgacccagg gaagacatta aaaaggtagt ggggtcgact 1740
   agatgaagga gagcctttct ctctgggcaa gagcggtgca atggtgtgta aaggtagctg 1800
   agaagacgaa aagggcaagc atcttcctgc taccaggctg ggaggcccca ggcccacgac 1860
   cccgaggaga gggaacgcag ggagactgag gtgacccttc tttcccccgg ggcccggtcg 1920
   tgtggttcgg tgtctctttt ctgttggacc cttaccttga cccaggcgct gccggggcct 1980
   gggcccgggc tgcggcgcac ggcactcccg ggaggcagcg agactcgagt taggcccaac 2040
   gcggcgccac ggcgtttcct ggccgggaat ggcccgtacc cgtgaggtgg gggtgggggg 2100
   cagaaaaggc ggagcgagcc cgagcgggga gggggagggc caggggcgga gggggccggc 2160
   actactgtgt tggcggactg gcgggactag ggctgcgtga gtctctgagc gcaggcgggc 2220
   ggcggccgcc cctcccccgg cggcggcagc ggcggcagcg gcggcagctc actcagcccg 2280
   ctgcccgagc ggaaacgcca ctgaccgcac ggggattccc agtgccggcg ccaggggcac 2340
   gcgggacacg ccccctcccg ccgcgccatt ggcctctccg cccaccgccc cacacttatt 2400
   ggccggtgcg ccgccaatca gcggaggctg ccggggccgc ctaaagaaga ggctgtgctt 2460
   tggggctccg gctcctcaga gagcctcggc taggtagggg atcgggactc tggcgggagg 2520
   gcggcttggt gcgtttgcgg ggatgggcgg ccgcggcagg ccctccgagc gtggtggagc 2580
   cgttctgtga gacagccggg tacgagtcgt gacgctggaa ggggcaagcg ggtggtgggc 2640
   aggaatgcgg tccgccctgc agcaaccgga gggggaggga gaagggagcg gaaaagtctc 2700
   caccggacgc ggccatggct cggggggggg ggggcagcgg aggagcgctt ccggccgacg 2760
   tctcgtcgct gattggcttc ttttcctccc gccgtgtgtg aaaacacaaa tggcgtgttt 2820
   tggttggcgt aaggcgcctg tcagttaacg gcagccggag tgcgcagccg ccggcagcct 2880
   cgctctgccc actgggtggg gcgggaggta ggtggggtga ggcgagctgg acgtgcgggc 2940
   gcggtcggcc tctggcgggg cgggggaggg gagggagggt cagcgaaagt agctcgcgcg 3000
   cgagcggccg cccaccctcc ccttcctctg ggggagtcgt tttacccgcc gccggccggg 3060
   cctcgtcgtc tgattggctc tcggggccca gaaaactggc ccttgccatt ggctcgtgtt 3120
   cgtgcaagtt gagtccatcc gccggccagc gggggcggcg aggaggcgct cccaggttcc 3180
   ggccctcccc tcggccccgc gccgcagagt ctggccgcgc gcccctgcgc aacgtggcag 3240
   gaagcgcgcg ctgggggcgg ggacgggcag tagggctgag cggctgcggg gcgggtgcaa 3300
   gcacgtttcc gacttgagtt gcctcaagag gggcgtgctg agccagacct ccatcgcgca 3360
   ctccggggag tggagggaag gagcgagggc tcagttgggc tgttttggag gcaggaagca 3420
   cttgctctcc caaagtcgct ctgagttgtt atcagtaagg gagctgcagt ggagtaggcg 3480
   gggagaaggc cgcacccttc tccggagggg ggaggggagt gttgcaatac ctttctggga 3540
   gttctctgct gcctcctggc ttctgaggac cgccctgggc ctgggagaat cccttccccc 3600
   tcttccctcg tgatctgcaa ctccagtctt tctagaagat gggcgggagt cttctgggca 3660
   ggcttaaagg ctaacctggt gtgtgggcgt tgtcctgcag gggaattgaa caggtgtaaa 3720
   attggaggga caagacttcc cacagatttt cggttttgtc gggaagtttt ttaatagggg 3780
   caaataagga aaatgggagg ataggtagtc atctggggtt ttatgcagca aaactacagg 3840
   ttattattgc ttgtgatccg cctcggagta ttttccatcg aggtagatta aagacatgct 3900
   cacccgagtt ttatactctc ctgcttgaga tccttactac agtatgaaat tacagtgtcg 3960
   cgagttagac tatgtaagca gaattttaat catttttaaa gagcccagta cttcatatcc 4020
   atttctcccg ctccttctgc agccttatca aaaggtattt tagaacactc attttagccc 4080
   cattttcatt tattatactg gcttatccaa cccctagaca gagcattggc attttccctt 4140
   tcctgatctt agaagtctga tgactcatga aaccagacag attagttaca tacaccacaa 4200
   atcgaggctg tagctggggc ctcaacactg cagttctttt ataactcctt agtacacttt 4260
   ttgttgatcc tttgccttga tccttaattt tcagtgtcta tcacctctcc cgtcagtggt 4320
   gttccacatt tgggcctatt ctcagtccag ggagttttac aacaatagat gtattgagaa 4380
   tccaacctaa agcttaactt tccactccca tgaatgcctc tctccttttt ctccatttat 4440
   aaactgagct attaaccatt aatggttcca ggtggatgtc tcctccccat attacctgat 4500
   gtatcttaca tattgccagg ctgatatttt aagacattaa aaggtatatt tcattattga 4560
   gccacatggt attgattact gcttactaaa attttgtcat tgtacacatc tgtaaaaggt 4620
   ggttcctttt ggaatgcaaa gttcaggtgt ttgttgtctt tcctgaccta aggtcttgtg 4680
   agcttgtatt ttttctattt aagcagtgct ttctcttgga ctggcttgac tcatggcatt 4740
   ctacacgtta ttgctggtct aaatgtgatt ttgccaagct tcttcaggac ctataatttt 4800
   gcttgacttg tagccaaaca caagtaaaat gattaagcaa caaatgtatt tgtgaagctt 4860
   ggtttttagg ttgttgtgtt gtgtgtgctt gtgctctata ataatactat ccaggggctg 4920
   gagaggtggc tcggagttca agagcacaga ctgctcttcc agaagtcctg agttcaattc 4980
   ccagcaacca catggtggct cacaaccatc tgtaatggga tctgatgccc tcttctggtg 5040
   tgtctgaaga ccacaagtgt attcacatta aataaataaa tcctccttct tcttcttttt 5100
   ttttttttta aagagaatac tgtctccagt agaatttact gaagtaatga aatactttgt 5160
   gtttgttcca atatggtagc caataatcaa attactcttt aagcactgga aatgttacca 5220
   aggaactaat ttttatttga agtgtaactg tggacagagg agccataact gcagacttgt 5280
   gggatacaga agaccaatgc agactttaat gtcttttctc ttacactaag caataaagaa 5340
   ataaaaattg aacttctagt atcctatttg tttaaactgc tagctttact taacttttgt 5400
   gcttcatcta tacaaagctg aaagctaagt ctgcagccat tactaaacat gaaagcaagt 5460
   aatgataatt ttggatttca aaaatgtagg gccagagttt agccagccag tggtggtgct 5520
   tgcctttatg cctttaatcc cagcactctg gaggcagaga caggcagatc tctgagtttg 5580
   agcccagcct ggtctacaca tcaagttcta tctaggatag ccaggaatac acacagaaac 5640
   cctgttgggg aggggggctc tgagatttca taaaattata attgaagcat tccctaatga 5700
   gccactatgg atgtggctaa atccgtctac ctttctgatg agatttgggt attatttttt 5760
   ctgtctctgc tgttggttgg gtcttttgac actgtgggct ttctttaaag cctccttcct 5820
   gccatgtggt ctcttgtttg ctactaactt cccatggctt aaatggcatg gctttttgcc 5880
   ttctaagggc agctgctgag atttgcagcc tgatttccag ggtggggttg ggaaatcttt 5940
   caaacactaa aattgtcctt taattttttt tttaaaaaat gggttatata ataaacctca 6000
   taaaatagtt atgaggagtg aggtggacta atattaaatg agtccctccc ctataaaaga 6060
   gctattaagg ctttttgtct tatacttaac ttttttttta aatgtggtat ctttagaacc 6120
   aagggtctta gagttttagt atacagaaac tgttgcatcg cttaatcaga ttttctagtt 6180
   tcaaatccag agaatccaaa ttcttcacag ccaaagtcaa attaagaatt tctgactttt 6240
   aatgttaatt tgcttactgt gaatataaaa atgatagctt ttcctgaggc agggtctcac 6300
   tatgtatctc tgcctgatct gcaacaagat atgtagacta aagttctgcc tgcttttgtc 6360
   tcctgaatac taaggttaaa atgtagtaat acttttggaa cttgcaggtc agattctttt 6420
   ataggggaca cactaaggga gcttgggtga tagttggtaa aatgtgtttc aagtgatgaa 6480
   aacttgaatt attatcaccg caacctactt tttaaaaaaa aaagccaggc ctgttagagc 6540
   atgcttaagg gatccctagg acttgctgag cacacaagag tagttacttg gcaggctcct 6600
   ggtgagagca tatttcaaaa aacaaggcag acaaccaaga aactacagtt aaggttacct 6660
   gtctttaaac catctgcata tacacaggga tattaaaata ttccaaataa tatttcattc 6720
   aagttttccc ccatcaaatt gggacatgga tttctccggt gaataggcag agttggaaac 6780
   taaacaaatg ttggttttgt gatttgtgaa attgttttca agtgatagtt aaagcccatg 6840
   agatacagaa caaagctgct atttcgaggt ctcttggttt atactcagaa gcacttcttt 6900
   gggtttccct gcactatcct gatcatgtgc taggcctacc ttaggctgat tgttgttcaa 6960
   ataaacttaa gtttcctgtc aggtgatgtc atatgatttc atatatcaag gcaaaacatg 7020
   ttatatatgt taaacatttg tacttaatgt gaaagttagg tctttgtggg tttgattttt 7080
   aattttcaaa acctgagcta aataagtcat ttttacatgt cttacatttg gtggaattgt 7140
   ataattgtgg tttgcaggca agactctctg acctagtaac cctacctata gagcactttg 7200
   ctgggtcaca agtctaggag tcaagcattt caccttgaag ttgagacgtt ttgttagtgt 7260
   atactagttt atatgttgga ggacatgttt atccagaaga tattcaggac tatttttgac 7320
   tgggctaagg aattgattct gattagcact gttagtgagc attgagtggc ctttaggctt 7380
   gaattggagt cacttgtata tctcaaataa tgctggcctt ttttaaaaag cccttgttct 7440
   ttatcaccct gttttctaca taatttttgt tcaaagaaat acttgtttgg atctcctttt 7500
   gacaacaata gcatgttttc aagccatatt tttttccttt tttttttttt tttttggttt 7560
   ttcgagacag ggtttctctg tatagccctg gctgtcctgg aactcacttt gtagaccagg 7620
   ctggcctcga actcagaaat ccgcctgcct ctgcctcctg agtgccggga ttaaaggcgt 7680
   gcaccaccac gcctggctaa gttggatatt ttgttatata actataacca atactaactc 7740
   cactgggtgg atttttaatt cagtcagtag tcttaagtgg tctttattgg cccttcatta 7800
   aaatctactg ttcactctaa cagaggctgt tggtactagt ggcacttaag caacttccta 7860
   cggatatact agcagattaa gggtcaggga tagaaactag tctagcgttt tgtataccta 7920
   ccagctttat actaccttgt tctgatagaa atatttcagg acatctagag tgtactataa 7980
   ggttgatggt aagcttataa ggaacttgaa agtggagtaa ctactccatt tctctgaggg 8040
   gagaattaaa atttttgacc aagtgttgtt gagccactga gaatggtctc agaacataac 8100
   ttcttaagga accttcccag attgccctca acactgcacc acatttggtc ctgcttgaac 8160
   attgccatgg ctcttaaagt cttaattaag aatattaatt gtgtaattat tgtttttcct 8220
   cctttagatc attccttgag gacaggacag tgcttgttta aggctatatt tctgctgtct 8280
   gagcagcaac aggtcttcga gatcaacatg atgttcataa tcccaagatg ttgccattta 8340
   tgttctcaga agcaagcaga ggcatgatgg tcagtgacag taatgtcact gtgttaaatg 8400
   ttgctatgca gtttggattt ttctaatgta gtgtaggtag aacatatgtg ttctgtatga 8460
   attaaactct taagttacac cttgtataat ccatgcaatg tgttatgcaa ttaccatttt 8520
   aagtattgta gctttctttg tatgtgagga taaaggtgtt tgtcataaaa tgttttgaac 8580
   atttccccaa agttccaaat tataaaacca caacgttaga acttatttat gaacaatggt 8640
   tgtagtttca tgcttttaaa atgcttaatt attcaattaa caccgtttgt gttataatat 8700
   atataaaact gacatgtaga agtgtttgtc cagaacattt cttaaatgta tactgtcttt 8760
   agagagttta atatagcatg tcttttgcaa catactaact tttgtgttgg tgcgagcaat 8820
   attgtgtagt cattttgaaa ggagtcattt caatgagtgt cagattgttt tgaatgttat 8880
   tgaacatttt aaatgcagac ttgttcgtgt tttagaaagc aaaactgtca gaagctttga 8940
   actagaaatt aaaaagctga agtatttcag aagggaaata agctacttgc tgtattagtt 9000
   gaaggaaagt gtaatagctt agaaaattta aaaccatata gttgtcattg ctgaatatct 9060
   ggcagatgaa aagaaatact cagtggttct tttgagcaat ataacagctt gttatattaa 9120
   aaattttccc cacagatata aactctaatc tataactcat aaatgttaca aatggatgaa 9180
   gcttacaaat gtggcttgac ttgtcactgt gcttgtttta gttatgtgaa agtttggcaa 9240
   taaacctatg tcctaaatag tcaaactgtg gaatgacttt ttaatctatt ggtttgtcta 9300
   gaacagttat gttgccattt gccctaatgg tgaaagaaaa agtggggagt gccttggcac 9360
   tgttcatttg tggtgtgaac caaagagggg ggcatgcact tacacttcaa acatcctttt 9420
   gaaagactga caagtttggg tcttcacagt tggaattggg catccctttt gtcagggagg 9480
   gagggaggga gggaggctgg cttgttatgc tgacaagtgt gattaaattc aaactttgag 9540
   gtaagttgga ggaacttgta cattgttagg agtgtgacaa tttggactct taatgatttg 9600
   gtcatacaaa atgaacctag accaacttct ggaagatgta tataataact ccatgttaca 9660
   ttgatttcac ctgactaata cttatccctt atcaattaaa tacagaagat gccagccatc 9720
   tgggcctttt aacccagaaa tttagtttca aactcctagg ttagtgttct cactgagcta 9780
   catcctgatc tagtcctgaa aataggacca ccatcacccc caaaaaaatc tcaaataaga 9840
   tttatgctag tgtttcaaaa ttttaggaat aggtaagatt agaaagtttt aaattttgag 9900
   aaatggcttc tctagaaaga tgtacatagt gaacactgaa tggctcctaa agagcctaga 9960
   aaactggtac tgagcacaca ggactgagag gtctttcttg aaaagcatgt attgctttac 10020
   gtgggtcaca gaaggcaggc aggaagaact tgggctgaaa ctggtgtctt aagtggctaa 10080
   catcttcaca actgatgagc aagaacttta tcctgatgca aaaaccatcc aaacaaacta 10140
   agtgaaaggt ggcaatggat cccaggctgc tctagaggag gacttgactt ctcatcccat 10200
   cacccacacc agatagctca tagactgcca attaacacca gcttctagcc tccacaggca 10260
   cctgcactgg tacacataat ttcacacaaa cacagtaaga agccttccac ctggcatggt 10320
   attgcttatc tttagttccc aacacttggg aggcagaggc cagccagggc tatgtgacaa 10380
   aaaccttgtc tagaggagaa acttcatagc ttatttccta ttcacgtaac caggttagca 10440
   aaatttacca gccagagatg aagctaacag tgtccactat atttgtagtg ttttaagtca 10500
   attttttaaa tatacttaat agaattaaag ctatggtgaa ccaagtacaa acctggtgta 10560
   ttaacttgag aacttagcat aaaaagtagt tcatttgttc agtaaatatt aaatgcttac 10620
   tggcaaagat tatgtcagga acttggtaaa tggtgatgaa acaatcatag ttgtacatct 10680
   tggttctgtg atcaccttgg tttgaggtaa aagtggttcc tttgatcaag gatggaattt 10740
   taagtttata ttcaatcaat aatgtattat tttgtgattg caaaattgcc tatctagggt 10800
   ataaaacctt taaaaatttc ataataccag ttcattctcc agttactaat tccaaaaagc 10860
   cactgactat ggtgccaatg tggattctgt tctcaaagga aggattgtct gtgcccttta 10920
   ttctaataga aacatcacac tgaaaatcta agctgaaaga agccagactt tcctaaataa 10980
   ataactttcc ataaagctca aacaaggatt acttttagga ggcactgtta aggaactgat 11040
   aagtaatgag gttacttata taatgatagt cccacaagac tatctgagga aaaatcagta 11100
   caactcgaaa acagaacaac cagctaggca ggaataacag ggctcccaag tcaggaggtc 11160
   tatccaacac ccttttctgt tgagggcccc agacctacat attgtataca aacagggagg 11220
   tgggtgattt taactctcct gaggtacctt ggtaaatctt tgtcctgagt aagcagtaca 11280
   gtgtacagtt tacattttca tttaaagata cattagctcc ctctaccccc taagactgac 11340
   aggcactttg ggggtgggga gggctttgga aaataacgct tccatacact aaaagagaaa 11400
   tttctttaat taggcttgtt ggttccatac atctactggt gtttctacta cttagtaata 11460
   ttataatagt cacacaagca tctttgctct gtttaggttg tatatttatt ttaaggcaga 11520
   tgataaaact gtagatctta agggatgctt ctgcttctga gatgatacaa agaatttaga 11580
   ccataaaaca gtaggttgca caagcaatag aatatggcct aaagtgttct gacacttaga 11640
   agccaagcag tgtaggcttc ttaagaaata ccattacaat caccttgcta gaaatcaagc 11700
   attctggagt ggtcaagcag tgtaacctgt actgtaagtt acttttctgc tatttttctc 11760
   ccaaagcaag ttctttatgc tgatatttcc agtgttagga actacaaata ttaataagtt 11820
   gtcttcactc ttttctttac caaggagggt ctcttccttc atcttgatct gaaggatgaa 11880
   caaaggcttg agcagtgcgc tttagaagat aaactgcagc atgaaggccc ccgatgttca 11940
   cccagactac atggaccttt cgccacacat gtcccattcc agataaggcc tggcacacac 12000
   aaaaaacata agtcattagg ctaccagtct gattctaaaa caacctaaaa tcttcccact 12060
   taaatgctat gggtggtggg ttggaaagtt gactcagaaa atcacttgct gtttttagag 12120
   aggatctggg ttcagtttct gatacattgt ggcttacaac tataactcca gttctagggg 12180
   gtccatccaa catcctcttc tgttgagggc accaaataaa tgtattgtgt acaaacaggg 12240
   aggtgagtga tttaactctc gtgtatagta ccttggtaaa acatttcttg tcctgagtaa 12300
   gcagtacagc tctgcctgtc cctggtctac agacacggct catttcccga aggcaagctg 12360
   gatagagatt ccaatttctc ttcttggatc ccatcctata aaagaaggtc aagtttaatc 12420
   tattgcaaaa ggtaaatagg tagtttctta catgagacaa gaacaaatct taggtgtgaa 12480
   gcagtcatct tttacaggcc agagcctcta ttctatgcca atgaaggaaa ctgttagtcc 12540
   agtgttatag agttagtcca gtgtatagtt ttctatcaga acactttttt tttaaacaac 12600
   tgcaacttag cttattgaag acaaaccacg agtagaaatc tgtccaagaa gcaagtgctt 12660
   ctcagcctac aatgtggaat aggaccatgt aatggtacag tgagtgaaat gaattatggc 12720
   atgtttttct gactgagaag acagtacaat aaaaggtaaa ctcatggtat ttatttaaaa 12780
   agaatccaat ttctaccttt ttccaaatgg catatctgtt acaataatat ccacagaagc 12840
   agttctcagt gggaggttgc agatatccca ctgaacagca tcaatgggca aaccccaggt 12900
   tgtttttctg tggagacaaa ggtaagatat ttcaatatat tttcccaagc taatgagatg 12960
   gctcagcaaa taatggtact ggccattaag tctcatgacc tgagcttgat cctcagggac 13020
   catgtggtac aaggagagac ctaaatcctt cagttggact tcaatcttct accctcatgt 13080
   ccacacacaa ataaatacaa taaaaaacat tctgcagtcg aatttctaaa agggcgaat 13139

<210> 12
<211> 21
<212> DNA
<213> Artificial Sequence

<220>
<223> Description of Artificial Sequence: DNA encoding siRNA 1

<400> 12
gcctgtgcct cttcagctac c                                                21


<210> 13
<211> 21
<212> DNA
<213> Artificial Sequence

<220>
<223> Description of Artificial Sequence: DNA encoding siRNA 2

<400> 13
gcggagacag cgacgaagag c                                                21


<210> 14
<211> 19
<212> DNA
<213> Artificial Sequence

<220>
<223> Description of Artificial Sequence: DNA encoding siRNA 3

<400> 14
cttattggag agagcacga                                                   19


<210> 15
<211> 30
<212> DNA
<213> Artificial Sequence

<220>
<223> Description of Artificial Sequence: Primer 1 for isolation of SA
      from adenovirus

<400> 15
atacctgcag gggtgacctg cacgtctagg                                       30


<210> 16
<211> 32
<212> DNA
<213> Artificial Sequence

<220>
<223> Description of Artificial Sequence: Primer 2 for isolation of SA
      from adenovirus

<400> 16
atacctgcag gagtactgga aagaccgcga ag                                    32

<210> 17
<211> 35
<212> DNA
<213> Artificial Sequence

<220>
<223> Description of Artificial Sequence: Primer 1 for isolation of H1
      promoter

<400> 17
aactatggcc ggccgaagaa ctcgtcaaga aggcg                                35


<210> 18
<211> 39
<212> DNA
<213> Artificial Sequence

<220>
<223> Description of Artificial Sequence: Primer 2 for isolation of H1
      promoter

<400> 18
tatggtaccg tttaaacgcg gccgcaaatt tattagagc                            39


<210> 19
<211> 47
<212> DNA
<213> Artificial Sequence

<220>
<223> Description of Artificial Sequence: DNA encoding
      an shRNA directed against the CDH-1 gene

<400> 19
tgagaagtct cccagtcagt tcaagagact gactgggaga cttctca                   47


<210> 20
<211> 47
<212> DNA
<213> Artificial Sequence

<220>
<223> Description of Artificial Sequence: DNA encoding
      an shRNA directed against the p53 gene

<400> 20
gactccagtg gtaatctact tcaagagagt agattaccac tggagtc                   47


<210> 21
<211> 47
<212> DNA
<213> Artificial Sequence

<220>
<223> Description of Artificial Sequence: DNA encoding

an shRNA directed against the CDC20 gene

<400> 21
cggcaggact ccgggccgat tcaagagatc ggcccggagt cctgccg          47


<210> 22
<211> 47
<212> DNA
<213> Artificial Sequence

<220>
<223> Description of Artificial Sequence: DNA encoding
      an shRNA directed against the CYLD gene

<400> 22
cctcatgcag ttctctttgt tcaagagaca aagagaactg catgagg          47


<210> 23
<211> 50
<212> DNA
<213> Artificial Sequence

<220>
<223> Description of Artificial Sequence: DNA encoding
      an shRNA directed against the RAS-GAP gene

<400> 23
aagatgaagc cactccctat ttcaagagaa aatagggagt ggcttcatct       50


<210> 24
<211> 41
<212> DNA
<213> Artificial Sequence

<220>
<223> Description of Artificial Sequence: DNA encoding
      an shRNA directed against the tubulin gene

<400> 24
gacagagcca agtggactca cagagtccac ttggctctgt c                41


<210> 25
<211> 42
<212> DNA
<213> Artificial Sequence

<220>
<223> Description of Artificial Sequence: DNA encoding
      an shRNA directed against the lamin gene

<400> 25
ctggacttcc agaagaacat tcgtgttctt ctggaagtcc ag               42


<210> 26
<211> 47
<212> DNA

<213> Artificial Sequence

<220>
<223> Description of Artificial Sequence: DNA encoding
      an shRNA directed against the gene for the
      ubiquitin carboxyl-terminal hydrolase 12

<400> 26
gagattggtc cagaacagtt tcaagagaac tgttctggac caatctc                47


<210> 27
<211> 47
<212> DNA
<213> Artificial Sequence

<220>
<223> Description of Artificial Sequence: DNA encoding
      an shRNA directed against the gene for  the
      ubiquitin carboxyl-terminal hydrolase 12

<400> 27
gcccttccga tcatggtagt tcaagagact accatgatcg gaagggc                47


<210> 28
<211> 47
<212> DNA
<213> Artificial Sequence

<220>
<223> Description of Artificial Sequence: DNA encoding
      an shRNA directed against the gene for the
      ubiquitin carboxyl-terminal hydrolase 12

<400> 28
tctttagaat tcttaagtat tcaagagata cttaagaatt ctaaaga                47


<210> 29
<211> 47
<212> DNA
<213> Artificial Sequence

<220>
<223> Description of Artificial Sequence: DNA encoding
      an shRNA directed against the gene of the
      ubiquitin carboxyl-terminal hydrolase

<400> 29
cattagctat atcaacatgt tcaagagaca tgttgatata gctaatg                47


<210> 30
<211> 47
<212> DNA
<213> Artificial Sequence

<220>
<223> Description of Artificial Sequence: DNA encoding
      an shRNA directed against the gene for the

ubiquitin carboxyl-terminal hydrolase11

<400> 30
accacaaacg gcggaacgat tcaagagatc gttccgccgt ttgtggt          47


<210> 31
<211> 47
<212> DNA
<213> Artificial Sequence

<220>
<223> Description of Artificial Sequence: DNA encoding
      an shRNA directed against the gene for the
      ubiquitin carboxyl-terminal hydrolase 11

<400> 31
gagggtcttg gaggtcttct tcaagagaga agacctccaa gaccctc          47


<210> 32
<211> 47
<212> DNA
<213> Artificial Sequence

<220>
<223> Description of Artificial Sequence: DNA encoding
      an shRNA directed against the gene for the
      ubiquitin carboxyl-terminal hydrolase 11

<400> 32
gtccatgccc agccgtacat tcaagagatg tacggctggg catggac          47


<210> 33
<211> 47
<212> DNA
<213> Artificial Sequence

<220>
<223> Description of Artificial Sequence: DNA encoding
      an shRNA directed against the gene for the
      ubiquitin carboxyl-terminal hydrolase 11

<400> 33
gctggacacc ctcgtggagt tcaagagact ccacgagggt gtccagc          47


<210> 34
<211> 47
<212> DNA
<213> Artificial Sequence

<220>
<223> Description of Artificial Sequence: DNA encoding
      an shRNA directed against the gene for the
      ubiquitin carboxyl-terminal hydrolase 10

<400> 34
gaatatcaga gaattgagtt tcaagagaac tcaattctct gatattc          47

<210> 35
<211> 47
<212> DNA
<213> Artificial Sequence

<220>
<223> Description of Artificial Sequence: DNA encoding
      an shRNA directed against the gene for the
      ubiquitin carboxyl-terminal hydrolase 10

<400> 35
tggacttcat gaggaaatgt tcaagagaca tttcctcatg aagtcca                47


<210> 36
<211> 47
<212> DNA
<213> Artificial Sequence

<220>
<223> Description of Artificial Sequence: DNA encoding
      an shRNA directed against the  gene for the
      ubiquitin carboxyl-terminal hydrolase 10

<400> 36
tattgaatat cctgtggact tcaagagagt ccacaggata ttcaata                47


<210> 37
<211> 47
<212> DNA
<213> Artificial Sequence

<220>
<223> Description of Artificial Sequence: DNA encoding
      an shRNA directed against the gene for the
      ubiquitin carboxyl-terminal hydrolase 10

<400> 37
ttgtactgag agaaactgct tcaagagagc agtttctctc agtacaa                47


<210> 38
<211> 47
<212> DNA
<213> Artificial Sequence

<220>
<223> Description of Artificial Sequence: DNA encoding
      an shRNA directed against the HAUSP gene

<400> 38
gatcaatgat aggtttgaat tcaagagatt caaacctatc attgatc                47


<210> 39
<211> 47
<212> DNA
<213> Artificial Sequence

<220>
<223> Description of Artificial Sequence: DNA encoding
      an shRNA directed against the HAUSP gene

<400> 39
ggagtttgag aagtttaaat tcaagagatt taaacttctc aaactcc                47


<210> 40
<211> 47
<212> DNA
<213> Artificial Sequence

<220>
<223> Description of Artificial Sequence: DNA encoding
      an shRNA directed against the HAUSP gene

<400> 40
gaactcctcg cttgctgagt tcaagagact cagcaagcga ggagttc                47


<210> 41
<211> 47
<212> DNA
<213> Artificial Sequence

<220>
<223> Description of Artificial Sequence: DNA encoding
      an shRNA directed against the HAUSP gene

<400> 41
ccgaatttaa cagagagaat tcaagagatt ctctctgtta aattcgg                47


<210> 42
<211> 47
<212> DNA
<213> Artificial Sequence

<220>
<223> Description of Artificial Sequence: DNA encoding
      an shRNA directed against the gene for the
      ubiquitin carboxyl-terminal hydrolase 8

<400> 42
gacagcagaa gaatgcagat tcaagagatc tgcattcttc tgctgtc                47


<210> 43
<211> 47
<212> DNA
<213> Artificial Sequence

<220>
<223> Description of Artificial Sequence: DNA encoding
      an shRNA directed against the gene for the
      ubiquitin carboxyl-terminal hydrolase 8

<400> 43
ataaagctca acgagaacct tcaagagagg ttctcgttga gctttat                47

<210> 44
<211> 47
<212> DNA
<213> Artificial Sequence

<220>
<223> Description of Artificial Sequence: DNA encoding
      an shRNA directed against the gene for the
      ubiquitin carboxyl-terminal hydrolase 8

<400> 44
ggtgaagtgg cagaagaatt tcaagagaat tcttctgcca cttcacc                47


<210> 45
<211> 47
<212> DNA
<213> Artificial Sequence

<220>
<223> Description of Artificial Sequence: DNA encoding
      an shRNA directed against the gene for the
      ubiquitin carboxyl-terminal hydrolase 8

<400> 45
gtattgcagt aatcatcact tcaagagagt gatgattact gcaatac                47


<210> 46
<211> 47
<212> DNA
<213> Artificial Sequence

<220>
<223> Description of Artificial Sequence: DNA encoding
      an shRNA directed against the FLJ10785 gene

<400> 46
gatatggggt tccatgtcat tcaagagatg acatggaacc ccatatc                47


<210> 47
<211> 47
<212> DNA
<213> Artificial Sequence

<220>
<223> Description of Artificial Sequence: DNA encoding
      an shRNA directed against the FLJ10785 gene

<400> 47
ggagacatgg ttcttagtgt tcaagagaca ctaagaacca tgtctcc                47


<210> 48
<211> 47
<212> DNA
<213> Artificial Sequence

<220>

<223> Description of Artificial Sequence: DNA encoding
      an shRNA directed against the FLJ10785 gene

<400> 48
agcaccaagt tcgtctcagt tcaagagact gagacgaact tggtgct                47


<210> 49
<211> 47
<212> DNA
<213> Artificial Sequence

<220>
<223> Description of Artificial Sequence: DNA encoding
      an shRNA directed against the FLJ10785 gene

<400> 49
gatgcaacac tgaaagaact tcaagagagt tctttcagtg ttgcatc                47


<210> 50
<211> 47
<212> DNA
<213> Artificial Sequence

<220>
<223> Description of Artificial Sequence: DNA encoding
      an shRNA directed against the KIAA0710 gene

<400> 50
gtcaatggca gtgatgatat tcaagagata tcatcactgc cattgac                47


<210> 51
<211> 47
<212> DNA
<213> Artificial Sequence

<220>
<223> Description of Artificial Sequence: DNA encoding
      an shRNA directed against the KIAA0710 gene

<400> 51
cctgctagct gcctgtggct tcaagagagc cacaggcagc tagcagg                47


<210> 52
<211> 47
<212> DNA
<213> Artificial Sequence

<220>
<223> Description of Artificial Sequence: DNA encoding
      an shRNA directed against the KIAA0710 gene

<400> 52
ccacctttgc cagaaggagt tcaagagact ccttctggca aaggtgg                47


<210> 53
<211> 47

<212> DNA
<213> Artificial Sequence

<220>
<223> Description of Artificial Sequence: DNA encoding
      an shRNA directed against the KIAA0710 gene

<400> 53
ccctattgag gcaagtgtct tcaagagaga cacttgcctc aataggg                47


<210> 54
<211> 47
<212> DNA
<213> Artificial Sequence

<220>
<223> Description of Artificial Sequence: DNA encoding
      an shRNA directed against the genes
      FLJ12552/FLJ14256

<400> 54
gaaggaaaac ttgctgacgt tcaagagacg tcagcaagtt ttccttc                47


<210> 55
<211> 47
<212> DNA
<213> Artificial Sequence

<220>
<223> Description of Artificial Sequence: DNA encoding
      an shRNA directed against the FLJ12552/FLJ14256
      genes

<400> 55
ctcacctggg tccatgagat tcaagagatc tcatggaccc aggtgag                47


<210> 56
<211> 47
<212> DNA
<213> Artificial Sequence

<220>
<223> Description of Artificial Sequence: DNA encoding
      an shRNA directed against the FLJ12552/FLJ14256
      genes

<400> 56
gctgtcttac cgtgtggtct tcaagagaga ccacacggta agacagc                47


<210> 57
<211> 47
<212> DNA
<213> Artificial Sequence

<220>
<223> Description of Artificial Sequence: DNA encoding
      an shRNA directed against the FLJ12552/FLJ14256

genes

<400> 57
cctggaccgc atgtatgact tcaagagagt catacatgcg gtccagg                47

<210> 58
<211> 47
<212> DNA
<213> Artificial Sequence

<220>
<223> Description of Artificial Sequence: DNA encoding
      an shRNA directed against the KIAA1203 gene

<400> 58
gtcaatggca gtgatgatat tcaagagata tcatcactgc cattgac                47

<210> 59
<211> 47
<212> DNA
<213> Artificial Sequence

<220>
<223> Description of Artificial Sequence: DNA encoding
      an shRNA directed against the KIAA1203 gene

<400> 59
cctgctagct gcctgtggct tcaagagagc cacaggcagc tagcagg                47

<210> 60
<211> 47
<212> DNA
<213> Artificial Sequence

<220>
<223> Description of Artificial Sequence: DNA encoding
      an shRNA directed against the KIAA1203 gene

<400> 60
ccacctttgc cagaaggagt tcaagagact ccttctggca aaggtgg                47

<210> 61
<211> 47
<212> DNA
<213> Artificial Sequence

<220>
<223> Description of Artificial Sequence: DNA encoding
      an shRNA directed against the KIAA1203 gene

<400> 61
ccctattgag gcaagtgtct tcaagagaga cacttgcctc aataggg                47

<210> 62
<211> 47
<212> DNA

<213> Artificial Sequence

<220>
<223> Description of Artificial Sequence: DNA encoding
      an shRNA directed against the FLJ23277 gene

<400> 62
ggaaatccga attgcttggt tcaagagacc aagcaattcg gatttcc                47


<210> 63
<211> 47
<212> DNA
<213> Artificial Sequence

<220>
<223> Description of Artificial Sequence: DNA encoding
      an shRNA directed against the FLJ23277 gene

<400> 63
cacatttctt caagtgtggt tcaagagacc acacttgaag aaatgtg                47


<210> 64
<211> 47
<212> DNA
<213> Artificial Sequence

<220>
<223> Description of Artificial Sequence: DNA encoding
      an shRNA directed against the FLJ23277 gene

<400> 64
cagcaggatg ctcaagaatt tcaagagaat tcttgagcat cctgctg                47


<210> 65
<211> 47
<212> DNA
<213> Artificial Sequence

<220>
<223> Description of Artificial Sequence: DNA encoding
      an shRNA directed against the FLJ23277 gene

<400> 65
gctgaatacc tacattggct tcaagagagc caatgtaggt attcagc                47


<210> 66
<211> 47
<212> DNA
<213> Artificial Sequence

<220>
<223> Description of Artificial Sequence: DNA encoding
      an shRNA directed against the FLJ14914 (similar to
      UBP4) gene

<400> 66
gggcttgtgc ctggccttgt tcaagagaca aggccaggca caagccc                47

<210> 67
<211> 47
<212> DNA
<213> Artificial Sequence

<220>
<223> Description of Artificial Sequence: DNA encoding
      an shRNA directed against the FLJ14914 (similar to
      UBP4) gene

<400> 67
gccttgtcct gccaagaagt tcaagagact tcttggcagg acaaggc                47


<210> 68
<211> 47
<212> DNA
<213> Artificial Sequence

<220>
<223> Description of Artificial Sequence: DNA encoding
      an shRNA directed against the FLJ14914 (similar to
      UBP4) gene

<400> 68
gattgaagcc aagggaacgt tcaagagacg ttcccttggc ttcaatc                47


<210> 69
<211> 47
<212> DNA
<213> Artificial Sequence

<220>
<223> Description of Artificial Sequence: DNA encoding
      an shRNA directed against the FLJ14914 (similar to
      UBP4) gene

<400> 69
tggcgcctgc tccccatctt tcaagagaag atggggagca ggcgcca                47


<210> 70
<211> 47
<212> DNA
<213> Artificial Sequence

<220>
<223> Description of Artificial Sequence: DNA encoding
      an shRNA directed against the gene for the
      ubiquitin carboxyl-terminal hydrolase isozyme L5

<400> 70
gaaccagcag gctctgtggt tcaagagacc acagagcctg ctggttc                47


<210> 71
<211> 47
<212> DNA

<213> Artificial Sequence

<220>
<223> Description of Artificial Sequence: DNA encoding
      an shRNA directed against the gene for the
      ubiquitin carboxyl-terminal hydrolase isozyme L5

<400> 71
ggaagcataa ttatctgcct tcaagagagg cagataatta tgcttcc                47


<210> 72
<211> 47
<212> DNA
<213> Artificial Sequence

<220>
<223> Description of Artificial Sequence: DNA encoding
      an shRNA directed against the gene for the
      ubiquitin carboxyl-terminal hydrolase isozyme L5

<400> 72
agaagaagat gcttttcact tcaagagagt gaaaagcatc ttcttct                47


<210> 73
<211> 47
<212> DNA
<213> Artificial Sequence

<220>
<223> Description of Artificial Sequence: DNA encoding
      an shRNA directed against the gene for the
      ubiquitin carboxyl-terminal hydrolase isozyme L5

<400> 73
cttgcagagg aggaacccat tcaagagatg ggttcctcct ctgcaag                47


<210> 74
<211> 47
<212> DNA
<213> Artificial Sequence

<220>
<223> Description of Artificial Sequence: DNA encoding
      an shRNA directed against the gene for the
      ubiquitin carboxyl-terminal hydrolase isozyme L3

<400> 74
gcaaacaatc agcaatgcct tcaagagagg cattgctgat tgtttgc                47


<210> 75
<211> 47
<212> DNA
<213> Artificial Sequence

<220>
<223> Description of Artificial Sequence: DNA encoding
      an shRNA directed against the gene for the

ubiquitin carboxyl-terminal hydrolase isozyme L3

<400> 75
ttggactgat tcatgctatt tcaagagaat agcatgaatc agtccaa 47

<210> 76
<211> 47
<212> DNA
<213> Artificial Sequence

<220>
<223> Description of Artificial Sequence: DNA encoding
an shRNA directed against the gene for the
ubiquitin carboxyl-terminal hydrolase isozyme L3

<400> 76
ctggcaattc gttgatgtat tcaagagata catcaacgaa ttgccag 47

<210> 77
<211> 47
<212> DNA
<213> Artificial Sequence

<220>
<223> Description of Artificial Sequence: DNA encoding
an shRNA directed against the gene for the
ubiquitin carboxyl-terminal hydrolase isozyme L3

<400> 77
ttagatgggc ggaagccatt tcaagagaat ggcttccgcc catctaa 47

<210> 78
<211> 47
<212> DNA
<213> Artificial Sequence

<220>
<223> Description of Artificial Sequence: DNA encoding
an shRNA directed against the gene for the
ubiquitin carboxyl-terminal hydrolase isozyme L1

<400> 78
gaggagtctc tgggctcggt tcaagagacc gagcccagag actcctc 47

<210> 79
<211> 47
<212> DNA
<213> Artificial Sequence

<220>
<223> Description of Artificial Sequence: DNA encoding
an shRNA directed against the gene for the
ubiquitin carboxyl-terminal hydrolase isozyme L1

<400> 79
gagctgaagg gacaagaagt tcaagagact tcttgtccct tcagctc 47

<210> 80
<211> 47
<212> DNA
<213> Artificial Sequence

<220>
<223> Description of Artificial Sequence: DNA encoding
      an shRNA directed against the gene for the
      ubiquitin carboxyl-terminal hydrolase isozyme L1

<400> 80
tgtcgggtag atgacaaggt tcaagagacc ttgtcatcta cccgaca                47


<210> 81
<211> 47
<212> DNA
<213> Artificial Sequence

<220>
<223> Description of Artificial Sequence: DNA encoding
      an shRNA directed against the gene for the
      ubiquitin carboxyl-terminal hydrolase isozyme L1

<400> 81
cacagctgtt cttctgttct tcaagagaga acagaagaac agctgtg                47


<210> 82
<211> 47
<212> DNA
<213> Artificial Sequence

<220>
<223> Description of Artificial Sequence: DNA encoding
      an shRNA directed against the KIAA1891/FLJ25263
      genes

<400> 82
gtggaagcct ttacagatct tcaagagaga tctgtaaagg cttccac                47


<210> 83
<211> 47
<212> DNA
<213> Artificial Sequence

<220>
<223> Description of Artificial Sequence: DNA encoding
      an shRNA directed against the KIAA1891/FLJ25236
      genes

<400> 83
caacagctgc cttcatctgt tcaagagaca gatgaaggca gctgttg                47


<210> 84
<211> 47
<212> DNA
<213> Artificial Sequence

<220>
<223> Description of Artificial Sequence: DNA encoding
      an shRNA directed against the KIAA1891/FLJ25263
      genes

<400> 84
ccataggcag tcctcctaat tcaagagatt aggaggactg cctatgg                47


<210> 85
<211> 47
<212> DNA
<213> Artificial Sequence

<220>
<223> Description of Artificial Sequence: DNA encoding
      an shRNA directed against the KIAA1891/FLJ25263
      genes

<400> 85
tgtatcactg ccactggttt tcaagagaaa ccagtggcag tgataca                47


<210> 86
<211> 47
<212> DNA
<213> Artificial Sequence

<220>
<223> Description of Artificial Sequence: DNA encoding
      an shRNA directed against the FLJ14528 (similar to
      UBP8) gene

<400> 86
catgttgggc agctgcagct tcaagagagc tgcagctgcc caacatg                47


<210> 87
<211> 47
<212> DNA
<213> Artificial Sequence

<220>
<223> Description of Artificial Sequence: DNA encoding
      an shRNA directed against the FLJ14528 (similar to
      UBP8) gene

<400> 87
cacaactgga gacctgaagt tcaagagact tcaggtctcc agttgtg                47


<210> 88
<211> 47
<212> DNA
<213> Artificial Sequence

<220>
<223> Description of Artificial Sequence: DNA encoding
      an shRNA directed against the FLJ14528 (similar to
      UBP8) gene

<400> 88
gtatgcctcc aagaaagagt tcaagagact ctttcttgga ggcatac                47

<210> 89
<211> 47
<212> DNA
<213> Artificial Sequence

<220>
<223> Description of Artificial Sequence: DNA encoding
      an shRNA directed against the FLJ14528 (similar to
      UBP8) gene

<400> 89
cttcacagta catttctctt tcaagagaag agaaatgtac tgtgaag                47

<210> 90
<211> 47
<212> DNA
<213> Artificial Sequence

<220>
<223> Description of Artificial Sequence: DNA encoding
      an shRNA directed against the gene for the U4/U6
      TRI snRNP 65 kDa protein

<400> 90
gtactttcaa ggccggggtt tcaagagaac cccggccttg aaagtac                47

<210> 91
<211> 47
<212> DNA
<213> Artificial Sequence

<220>
<223> Description of Artificial Sequence: DNA encoding
      an shRNA directed against the gene for the U4/U6
      TRI snRNP 65 kDa protein

<400> 91
cttggacaag caagccaaat tcaagagatt tggcttgctt gtccaag                47

<210> 92
<211> 47
<212> DNA
<213> Artificial Sequence

<220>
<223> Description of Artificial Sequence: DNA encoding
      an shRNA directed against the gene for the U4/U6
      TRI snRNP 65 kDa protein

<400> 92
gactattgtg actgatgttt tcaagagaaa catcagtcac aatagtc                47

<210> 93
<211> 47
<212> DNA
<213> Artificial Sequence

<220>
<223> Description of Artificial Sequence: DNA encoding
      an shRNA directed against the gene for the U4/U6
      TRI snRNP 65kDa protein

<400> 93
ggagaacttt ctgaagcgct tcaagagagc gcttcagaaa gttctcc                47


<210> 94
<211> 47
<212> DNA
<213> Artificial Sequence

<220>
<223> Description of Artificial Sequence: DNA encoding
      an shRNA for the XM_089437 gene

<400> 94
gacgagagaa accttcacct tcaagagagg tgaaggtttc tctcgtc                47


<210> 95
<211> 47
<212> DNA
<213> Artificial Sequence

<220>
<223> Description of Artificial Sequence: DNA encoding
      an shRNA directed against the XM_089437 gene

<400> 95
acattattct acattctttt tcaagagaaa agaatgtaga ataatgt                47


<210> 96
<211> 47
<212> DNA
<213> Artificial Sequence

<220>
<223> Description of Artificial Sequence: DNA encoding
      an shRNA directed against the XM_089437 gene

<400> 96
agattcgcaa atggatgtat tcaagagata catccatttg cgaatct                47


<210> 97
<211> 47
<212> DNA
<213> Artificial Sequence

<220>
<223> Description of Artificial Sequence: DNA encoding
      an shRNA directed against the XM_089437 gene

<400> 97
cattcccacc atgagtctgt tcaagagaca gactcatggt gggaatg                47

<210> 98
<211> 47
<212> DNA
<213> Artificial Sequence

<220>
<223> Description of Artificial Sequence: DNA encoding
      an shRNA directed against the KIAA1453 gene

<400> 98
gatcgcccga cacttccgct tcaagagagc ggaagtgtcg ggcgatc                47

<210> 99
<211> 47
<212> DNA
<213> Artificial Sequence

<220>
<223> Description of Artificial Sequence: DNA encoding
      an shRNA directed against the KIAA1453 gene

<400> 99
ccagcaggcc tacgtgctgt tcaagagaca gcacgtaggc ctgctgg                47

<210> 100
<211> 47
<212> DNA
<213> Artificial Sequence

<220>
<223> Description of Artificial Sequence: DNA encoding
      an shRNA directed against the KIAA1453 gene

<400> 100
gccagctcct ccacagcact tcaagagagt gctgtggagg agctggc                47

<210> 101
<211> 47
<212> DNA
<213> Artificial Sequence

<220>
<223> Description of Artificial Sequence: DNA encoding
      an shRNA directed against the KIAA1453 gene

<400> 101
cgccgccaag tggagcagat tcaagagatc tgctccactt ggcggcg                47

<210> 102
<211> 47
<212> DNA
<213> Artificial Sequence

<220>
<223> Description of Artificial Sequence: DNA encoding
      an shRNA directed against the FLJ12697 gene

<400> 102
gaagatgccc atgaattcct tcaagagagg aattcatggg catcttc                47


<210> 103
<211> 47
<212> DNA
<213> Artificial Sequence

<220>
<223> Description of Artificial Sequence: DNA encoding
      an shRNA directed against the FLJ12697 gene

<400> 103
caaacaggct gcgccaggct tcaagagagc ctggcgcagc ctgtttg                47


<210> 104
<211> 47
<212> DNA
<213> Artificial Sequence

<220>
<223> Description of Artificial Sequence: DNA encoding
      an shRNA directed against the FLJ12697 gene

<400> 104
acggcctagc gcctgatggt tcaagagacc atcaggcgct aggccgt                47


<210> 105
<211> 47
<212> DNA
<213> Artificial Sequence

<220>
<223> Description of Artificial Sequence: DNA encoding
      an shRNA directed against the FLJ12697 gene

<400> 105
ctgtaacctc tctgatcggt tcaagagacc gatcagagag gttacag                47


<210> 106
<211> 47
<212> DNA
<213> Artificial Sequence

<220>
<223> Description of Artificial Sequence: DNA encoding
      an shRNA directed against the gene for the
      ubiquitin specific protease (USP18)

<400> 106
tctgtcagtc catcctggct tcaagagagc caggatggac tgacaga                47

<210> 107
<211> 47
<212> DNA
<213> Artificial Sequence

<220>
<223> Description of Artificial Sequence: DNA encoding
      an shRNA directed against the gene for the
      ubiquitin specific protease (USP18)

<400> 107
tgaagcgaga gtcttgtgat tcaagagatc acaagactct cgcttca                47


<210> 108
<211> 47
<212> DNA
<213> Artificial Sequence

<220>
<223> Description of Artificial Sequence: DNA encoding
      an shRNA directed against the gene for the
      ubiquitin specific protease (USP18)

<400> 108
gatggagtgc taatggaaat tcaagagatt tccattagca ctccatc                47


<210> 109
<211> 47
<212> DNA
<213> Artificial Sequence

<220>
<223> Description of Artificial Sequence: DNA encoding
      an shRNA directed against the gene for the
      ubiquitin specific protease (USP18)

<400> 109
ccttcagaga ttgacacgct tcaagagagc gtgtcaatct ctgaagg                47


<210> 110
<211> 47
<212> DNA
<213> Artificial Sequence

<220>
<223> Description of Artificial Sequence: DNA encoding
      an shRNA directed against the gene for the
      ubiquitin carboxyl-terminal hydrolase 20

<400> 110
cctgaccacg ttccgactgt tcaagagaca gtcggaacgt ggtcagg                47


<210> 111
<211> 47
<212> DNA
<213> Artificial Sequence

<220>
<223> Description of Artificial Sequence: DNA encoding
      an shRNA directed against the gene for the
      ubiquitin carboxyl-terminal hydrolase 20

<400> 111
gagttccttc gctgcctgat tcaagagatc aggcagcgaa ggaactc                47


<210> 112
<211> 47
<212> DNA
<213> Artificial Sequence

<220>
<223> Description of Artificial Sequence: DNA encoding
      an shRNA directed against the gene for the
      ubiquitin carboxyl-terminal hydrolase 20

<400> 112
gactgccttg ctgccttctt tcaagagaag aaggcagcaa ggcagtc                47


<210> 113
<211> 47
<212> DNA
<213> Artificial Sequence

<220>
<223> Description of Artificial Sequence: DNA encoding
      an shRNA directed against the gene for the
      ubiquitin carboxyl-terminal hydrolase 20

<400> 113
cgccgagggc tacgtactct tcaagagaga gtacgtagcc ctcggcg                47


<210> 114
<211> 47
<212> DNA
<213> Artificial Sequence

<220>
<223> Description of Artificial Sequence: DNA encoding
      an shRNA directed against the gene for the
      ubiquitin carboxyl-terminal hydrolase 24

<400> 114
ggcgagaaga aaggactgtt tcaagagaac agtcctttct tctcgcc                47


<210> 115
<211> 47
<212> DNA
<213> Artificial Sequence

<220>
<223> Description of Artificial Sequence: DNA encoding
      an shRNA directed against the gene for the
      ubiquitin carboxyl terminal hydrolase 24

<400> 115
ggacgagaat tgataaagat tcaagagatc tttatcaatt ctcgtcc                47

<210> 116
<211> 47
<212> DNA
<213> Artificial Sequence

<220>
<223> Description of Artificial Sequence: DNA encoding
      an shRNA directed against the gene for the
      ubiquitin carboxyl-terminal hydrolase 24

<400> 116
gcacgagaat ttgggaatct tcaagagaga ttcccaaatt ctcgtgc                47

<210> 117
<211> 47
<212> DNA
<213> Artificial Sequence

<220>
<223> Description of Artificial Sequence: DNA encoding
      an shRNA directed against the gene for the
      ubiquitin carboxyl-terminal hydrolase 24

<400> 117
ctacttcatg aaatattggt tcaagagacc aatatttcat gaagtag                47

<210> 118
<211> 47
<212> DNA
<213> Artificial Sequence

<220>
<223> Description of Artificial Sequence: DNA encoding
      an shRNA directed against the KIAA1594 gene

<400> 118
gataacagct tcttgtctat tcaagagata gacaagaagc tgttatc                47

<210> 119
<211> 47
<212> DNA
<213> Artificial Sequence

<220>
<223> Description of Artificial Sequence: DNA encoding
      an shRNA directed against the KIAA1594 gene

<400> 119
gagaatagga catcagggct tcaagagagc cctgatgtcc tattctc                47

<210> 120
<211> 47

<212> DNA
<213> Artificial Sequence

<220>
<223> Description of Artificial Sequence: DNA encoding
      an shRNA directed against the KIAA1594 gene

<400> 120
cttggaagac tgaacctgtt tcaagagaac aggttcagtc ttccaag                47


<210> 121
<211> 47
<212> DNA
<213> Artificial Sequence

<220>
<223> Description of Artificial Sequence: DNA encoding
      an shRNA directed against the KIAA1594 gene

<400> 121
caactccttt gtggatgcat tcaagagatg catccacaaa ggagttg                47


<210> 122
<211> 47
<212> DNA
<213> Artificial Sequence

<220>
<223> Description of Artificial Sequence: DNA encoding
      an shRNA directed against the KIAA1350 gene

<400> 122
gatgttgtct ccaaatgcat tcaagagatg catttggaga caacatc                47


<210> 123
<211> 47
<212> DNA
<213> Artificial Sequence

<220>
<223> Description of Artificial Sequence: DNA encoding
      an shRNA directed against the KIAA1350 gene

<400> 123
cgtggggact gtacctccct tcaagagagg gaggtacagt ccccacg                47


<210> 124
<211> 47
<212> DNA
<213> Artificial Sequence

<220>
<223> Description of Artificial Sequence: DNA encoding
      an shRNA direcetd against the KIAA1350 gene

<400> 124
gtacagcttc agaaccaagt tcaagagact tggttctgaa gctgtac                47

<210> 125
<211> 47
<212> DNA
<213> Artificial Sequence

<220>
<223> Description of Artificial Sequence: DNA encoding
      an shRNA directed against the gene for the
      ubiquitin carboxyl-terminal hydrolase 25

<400> 125
gatgatcttc agagagcaat tcaagagatt gctctctgaa gatcatc                47


<210> 126
<211> 47
<212> DNA
<213> Artificial Sequence

<220>
<223> Description of Artificial Sequence: DNA encoding
      an shRNA directed against the gene for the
      ubiquitin carboxyl-terminal hydrolase 25

<400> 126
ggaacatcgg aatttgcctt tcaagagaag gcaaattccg atgttcc                47


<210> 127
<211> 47
<212> DNA
<213> Artificial Sequence

<220>
<223> Description of Artificial Sequence: DNA encoding
      an shRNA directed against the gene for the
      ubiquitin carboxyl-terminal hydrolase 25

<400> 127
gagctagtga gggactcttt tcaagagaaa gagtccctca ctagctc                47


<210> 128
<211> 47
<212> DNA
<213> Artificial Sequence

<220>
<223> Description of Artificial Sequence: DNA encoding
      an shRNA directed against the gene for the
      ubiquitin carboxyl-terminal hydrolase 25

<400> 128
gcagggttct ttaaggcaat tcaagagatt gccttaaaga accctgc                47


<210> 129
<211> 47
<212> DNA

<213> Artificial Sequence

<220>
<223> Description of Artificial Sequence: DNA encoding
      an shRNA directed against the gene for the
      ubiquitin carboxyl-terminal hydrolase 16

<400> 129
tcgatgattc ctctgaaact tcaagagagt ttcagaggaa tcatcga                47


<210> 130
<211> 47
<212> DNA
<213> Artificial Sequence

<220>
<223> Description of Artificial Sequence: DNA encoding
      an shRNA directed against the gene for the
      ubiquitin carboxyl-terminal hydrolase 16

<400> 130
gataatggaa atattgaact tcaagagagt tcaatatttc cattatc                47


<210> 131
<211> 47
<212> DNA
<213> Artificial Sequence

<220>
<223> Description of Artificial Sequence: DNA encoding
      an shRNA directed against the gene for the
      ubiquitin carboxyl-terminal hydrolase 16

<400> 131
gttcttcatt taaatgatat tcaagagata tcatttaaat gaagaac                47


<210> 132
<211> 47
<212> DNA
<213> Artificial Sequence

<220>
<223> Description of Artificial Sequence: DNA encoding
      an shRNA directed against the gene for the
      ubiquitin carboxyl-terminal hydrolase 16

<400> 132
gttaacaaac acataaagtt tcaagagaac tttatgtgtt tgttaac                47


<210> 133
<211> 47
<212> DNA
<213> Artificial Sequence

<220>
<223> Description of Artificial Sequence: DNA encoding
      an shRNA directed against the USP9X gene

<400> 133
gttagagaag attcttcgtt tcaagagaac gaagaatctt ctctaac                47

<210> 134
<211> 47
<212> DNA
<213> Artificial Sequence

<220>
<223> Description of Artificial Sequence: DNA encoding
      an shRNA directed against the USP9X gene

<400> 134
gttgattgga caattaaact tcaagagagt ttaattgtcc aatcaac                47

<210> 135
<211> 47
<212> DNA
<213> Artificial Sequence

<220>
<223> Description of Artificial Sequence: DNA encoding
      an shRNA directed against the USP9X gene

<400> 135
ggttgatacc gtaaagcgct tcaagagagc gctttacggt atcaacc                47

<210> 136
<211> 47
<212> DNA
<213> Artificial Sequence

<220>
<223> Description of Artificial Sequence: DNA encoding
      an shRNA directed against the USP9X gene

<400> 136
gcaatgaaac gtccaatggt tcaagagacc attggacgtt tcattgc                47

<210> 137
<211> 47
<212> DNA
<213> Artificial Sequence

<220>
<223> Description of Artificial Sequence: DNA encoding
      an shRNA directed against the USP9Y gene

<400> 137
agctagagaa aattcttcgt tcaagagacg aagaattttc tctagct                47

<210> 138
<211> 47
<212> DNA
<213> Artificial Sequence

<220>
<223> Description of Artificial Sequence: DNA encoding
      an shRNA directed against the USP9Y gene

<400> 138
gatcctatat gatggatgat tcaagagatc atccatcata taggatc                47


<210> 139
<211> 47
<212> DNA
<213> Artificial Sequence

<220>
<223> Description of Artificial Sequence: DNA encoding
      an shRNA directed against the USP9Y gene

<400> 139
gttcttcttg tcagtgaaat tcaagagatt tcactgacaa gaagaac                47


<210> 140
<211> 47
<212> DNA
<213> Artificial Sequence

<220>
<223> Description of Artificial Sequence: DNA encoding
      an shRNA directed against the USP9Y gene

<400> 140
cttgagcttg agtgaccact tcaagagagt ggtcactcaa gctcaag                47


<210> 141
<211> 47
<212> DNA
<213> Artificial Sequence

<220>
<223> Description of Artificial Sequence: DNA encoding
      an shRNA direcetd against the gene for the
      ubiquitin carboxyl-terminal hydrolase 5

<400> 141
gaccggccag cgagtctact tcaagagagt agactcgctg gccggtc                47


<210> 142
<211> 47
<212> DNA
<213> Artificial Sequence

<220>
<223> Description of Artificial Sequence: DNA encoding
      an shRNA directed against the gene for the
      ubiquitin carboxyl-terminal hydrolase 5

<400> 142
ggacctgggc tacatctact tcaagagagt agatgtagcc caggtcc                47

<210> 143
<211> 47
<212> DNA
<213> Artificial Sequence

<220>
<223> Description of Artificial Sequence: DNA encoding
      an shRNA directed against the gene for the
      ubiquitin carboxyl-terminal hydrolase 5

<400> 143
ctctgtggtc caggtgctct tcaagagaga gcacctggac cacagag                47


<210> 144
<211> 47
<212> DNA
<213> Artificial Sequence

<220>
<223> Description of Artificial Sequence: DNA encoding
      an shRNA directed against the gene for the
      ubiquitin carboxyl-terminal hydrolase 5

<400> 144
gaccacacga tttgcctcat tcaagagatg aggcaaatcg tgtggtc                47


<210> 145
<211> 47
<212> DNA
<213> Artificial Sequence

<220>
<223> Description of Artificial Sequence: DNA encoding
      an shRNA directed against the gene for the
      ubiquitin carboxyl-terminal hydrolase 26

<400> 145
tggcttgttt attgaaggat tcaagagatc cttcaataaa caagcca                47


<210> 146
<211> 47
<212> DNA
<213> Artificial Sequence

<220>
<223> Description of Artificial Sequence: DNA encoding
      an shRNA directed against the gene for the
      ubiquitin carboxyl-terminal hydrolase 26

<400> 146
gtgaatttgg ggaagataat tcaagagatt atcttcccca aattcac                47


<210> 147
<211> 47
<212> DNA

<213> Artificial Sequence

<220>
<223> Description of Artificial Sequence: DNA encoding
      an shRNA directed against the gene for the
      ubiquitin carboxyl- terminal hydrolase 26

<400> 147
cgctatagct tgaatgagtt tcaagagaac tcattcaagc tatagcg                47


<210> 148
<211> 47
<212> DNA
<213> Artificial Sequence

<220>
<223> Description of Artificial Sequence: DNA encoding
      an shRNA directed against the gene for the
      ubiquitin carboxyl- terminal hydrolase 26

<400> 148
gatatcctgg ctccacacat tcaagagatg tgtggagcca ggatatc                47


<210> 149
<211> 47
<212> DNA
<213> Artificial Sequence

<220>
<223> Description of Artificial Sequence: DNA encoding
      an shRNA directed against the KIAA1097 gene

<400> 149
gagccagtcg gatgtagatt tcaagagaat ctacatccga ctggctc                47


<210> 150
<211> 47
<212> DNA
<213> Artificial Sequence

<220>
<223> Description of Artificial Sequence: DNA encoding
      an shRNA directed against the KIAA1097 gene

<400> 150
gtaaattctg aaggcgaatt tcaagagaat tcgccttcag aatttac                47


<210> 151
<211> 47
<212> DNA
<213> Artificial Sequence

<220>
<223> Description of Artificial Sequence: DNA encoding
      an shRNA directed against the KIAA1097 gene

<400> 151

gccctcctaa atcaggcaat tcaagagatt gcctgattta ggagggc                47

<210> 152
<211> 47
<212> DNA
<213> Artificial Sequence

<220>
<223> Description of Artificial Sequence: DNA encoding
      an shRNA directed against the KIAA1097 gene

<400> 152
gttgagaaat ggagtgaagt tcaagagact tcactccatt tctcaac                47

<210> 153
<211> 47
<212> DNA
<213> Artificial Sequence

<220>
<223> Description of Artificial Sequence: DNA encoding
      an shRNA directed against the gene for the
      ubiquitin specific protease (USP22) gene

<400> 153
gcttggaaaa tgcaaggcgt tcaagagacg ccttgcattt tccaagc                47

<210> 154
<211> 47
<212> DNA
<213> Artificial Sequence

<220>
<223> Description of Artificial Sequence: DNA encoding
      an shRNA directed against the gene for the
      ubiquitin specific protease (USP22) gene

<400> 154
ctgcatcata gaccagatct tcaagagaga tctggtctat gatgcag                47

<210> 155
<211> 47
<212> DNA
<213> Artificial Sequence

<220>
<223> Description of Artificial Sequence: DNA encoding
      an shRNA directed against the gene for the
      ubiquitin specific protease (USP22) gene

<400> 155
gatcaccacg tatgtgtcct tcaagagagg acacatacgt ggtgatc                47

<210> 156
<211> 47
<212> DNA

<213> Artificial Sequence

<220>
<223> Description of Artificial Sequence: DNA encoding
      an shRNA directed against the gene for the
      ubiquitin specific protease 22 (USP22) gene

<400> 156
tgacaacaag tattccctgt tcaagagaca gggaatactt gttgtca                47


<210> 157
<211> 47
<212> DNA
<213> Artificial Sequence

<220>
<223> Description of Artificial Sequence: DNA encoding
      an shRNA directed against the gene for the
      ubiquitin specific processing protease 29

<400> 157
gaaatataag acagattcct tcaagagagg aatctgtctt atatttc                47


<210> 158
<211> 47
<212> DNA
<213> Artificial Sequence

<220>
<223> Description of Artificial Sequence: DNA encoding
      an shRNA directed against the gene for the
      ubiquitin specific processing protease 29

<400> 158
cccatcaagt ttagaggatt tcaagagaat cctctaaact tgatggg                47


<210> 159
<211> 47
<212> DNA
<213> Artificial Sequence

<220>
<223> Description of Artificial Sequence: DNA encoding
      an shRNA directed against the gene for the
      ubiquitin specific processing protease 29

<400> 159
ggtgtcccat gggaatatat tcaagagata tattcccatg ggacacc                47


<210> 160
<211> 47
<212> DNA
<213> Artificial Sequence

<220>
<223> Description of Artificial Sequence: DNA encoding
      an shRNA directed against the gene for the

ubiquitin specific processing protease 29

<400> 160
gaatgccgac ctacaaagat tcaagagatc tttgtaggtc ggcattc                47


<210> 161
<211> 47
<212> DNA
<213> Artificial Sequence

<220>
<223> Description of Artificial Sequence: DNA encoding
      an shRNA directed against the CYLD gene

<400> 161
cagttatatt ctgtgatgtt tcaagagaac atcacagaat ataactg                47


<210> 162
<211> 47
<212> DNA
<213> Artificial Sequence

<220>
<223> Description of Artificial Sequence: DNA encoding
      an shRNA directed against the CYLD gene

<400> 162
gaggtgttgg ggacaaaggt tcaagagacc tttgtcccca acacctc                47


<210> 163
<211> 47
<212> DNA
<213> Artificial Sequence

<220>
<223> Description of Artificial Sequence: DNA encoding
      an shRNA directed against the CYLD gene

<400> 163
gtgggctcat tggctgaagt tcaagagact tcagccaatg agcccac                47


<210> 164
<211> 47
<212> DNA
<213> Artificial Sequence

<220>
<223> Description of Artificial Sequence: DNA encoding
      an shRNA directed against the CYLD gene

<400> 164
gagctactga ggacagaaat tcaagagatt tctgtcctca gtagctc                47


<210> 165
<211> 47
<212> DNA

<213> Artificial Sequence

<220>
<223> Description of Artificial Sequence: DNA encoding
      an shRNA directed against the gene for the
      ubiquitin carboxyl-terminal hydrolase 2

<400> 165
tcagcaggat gctcaggagt tcaagagact cctgagcatc ctgctga                47


<210> 166
<211> 47
<212> DNA
<213> Artificial Sequence

<220>
<223> Description of Artificial Sequence: DNA encoding
      an shRNA directed against the gene for  the
      ubiquitin carboxyl-terminal hydrolase 2

<400> 166
gaagttctcc atccagaggt tcaagagacc tctggatgga gaacttc                47


<210> 167
<211> 47
<212> DNA
<213> Artificial Sequence

<220>
<223> Description of Artificial Sequence: DNA encoding
      an shRNA directed against the gene for the
      ubiquitin carboxyl-terminal hydrolase 2

<400> 167
gccggtcccc accagcagct tcaagagagc tgctggtggg gaccggc                47


<210> 168
<211> 47
<212> DNA
<213> Artificial Sequence

<220>
<223> Description of Artificial Sequence: DNA encoding
      an shRNA directed against the gene for the
      ubiquitin carboxyl-terminal hydrolase 2

<400> 168
cactcgggag ttgagagatt tcaagagaat ctctcaactc ccgagtg                47


<210> 169
<211> 47
<212> DNA
<213> Artificial Sequence

<220>
<223> Description of Artificial Sequence: DNA encoding
      an shRNA directed against the gene for the

ubiquitin specific protease 3 (USP3)

<400> 169
gcccttgggt ctgtttgact tcaagagagt caaacagacc caagggc                47


<210> 170
<211> 47
<212> DNA
<213> Artificial Sequence

<220>
<223> Description of Artificial Sequence: DNA encoding
      an shRNA directed against the gene for the
      ubiquitin specific protease 3 (USP3)

<400> 170
ctcaacacta aacagcaagt tcaagagact tgctgtttag tgttgag                47


<210> 171
<211> 47
<212> DNA
<213> Artificial Sequence

<220>
<223> Description of Artificial Sequence: DNA encoding
      an shRNA directed against the gene for the
      ubiquitin specific protease 3 (USP3)

<400> 171
gatttcattg gacagcatat tcaagagata tgctgtccaa tgaaatc                47


<210> 172
<211> 47
<212> DNA
<213> Artificial Sequence

<220>
<223> Description of Artificial Sequence: DNA encoding
      an shRNA directed against the gene for the
      ubiquitin specific protease 3 (USP3)

<400> 172
catggggcac caactaattt tcaagagaaa ttagttggtg ccccatg                47


<210> 173
<211> 47
<212> DNA
<213> Artificial Sequence

<220>
<223> Description of Artificial Sequence: DNA encoding
      an shRNA directed against the gene for the
      ubiquitin carboxyl-terminal hydrolase 23

<400> 173
ggtgtctctg cgggattgtt tcaagagaac aatcccgcag agacacc                47

<210> 174
<211> 47
<212> DNA
<213> Artificial Sequence

<220>
<223> Description of Artificial Sequence: DNA encoding
      an shRNA directed against the gene for the
      ubiquitin carboxyl-terminal hydrolase 23

<400> 174
agttcagtag gtgtagactt tcaagagaag tctacaccta ctgaact                47


<210> 175
<211> 47
<212> DNA
<213> Artificial Sequence

<220>
<223> Description of Artificial Sequence: DNA encoding
      an shRNA directed against the gene for the
      ubiquitin carboxyl-terminal hydrolase 23

<400> 175
gagttcctga agctcctcat tcaagagatg aggagcttca ggaactc                47


<210> 176
<211> 47
<212> DNA
<213> Artificial Sequence

<220>
<223> Description of Artificial Sequence: DNA encoding
      an shRNA directed against the gene for the
      ubiquitin carboxyl-terminal hydrolase 23

<400> 176
ggatttgctg ggggcaaggt tcaagagacc ttgcccccag caaatcc                47


<210> 177
<211> 47
<212> DNA
<213> Artificial Sequence

<220>
<223> Description of Artificial Sequence: DNA encoding
      an shRNA directed against the UBP-32.7 gene

<400> 177
ctcagaaagc caacattcat tcaagagatg aatgttggct ttctgag                47


<210> 178
<211> 47
<212> DNA
<213> Artificial Sequence

<220>
<223> Description of Artificial Sequence: DNA encoding
      an shRNA directed against the UBP-32.7 gene

<400> 178
cgcattgtaa taagaaggtt tcaagagaac cttcttatta caatgcg                47


<210> 179
<211> 47
<212> DNA
<213> Artificial Sequence

<220>
<223> Description of Artificial Sequence: DNA encoding
      an shRNA directed against the UBP-32.7 gene

<400> 179
gggaggaaaa tgcagaaatt tcaagagaat ttctgcattt tcctccc                47


<210> 180
<211> 47
<212> DNA
<213> Artificial Sequence

<220>
<223> Description of Artificial Sequence: DNA encoding
      an shRNA directed against the UBP-32.7 gene

<400> 180
ttacaaattt aggaaatact tcaagagagt atttcctaaa tttgtaa                47


<210> 181
<211> 47
<212> DNA
<213> Artificial Sequence

<220>
<223> Description of Artificial Sequence: DNA encoding
      an shRNA directed against the gene for the Homo
      sapiens ubiquitin specific protease 13
      (isopeptidase T-3)

<400> 181
gttatgaatt gatatgcagt tcaagagact gcatatcaat tcataac                47


<210> 182
<211> 47
<212> DNA
<213> Artificial Sequence

<220>
<223> Description of Artificial Sequence: DNA encoding
      an shRNA directed against the gene for the Homo
      sapiens ubiquitin specific protease 13
      (isopeptidase T-3)

<400> 182

gtgataacac aactaatggt tcaagagacc attagttgtg ttatcac                47

<210> 183
<211> 47
<212> DNA
<213> Artificial Sequence

<220>
<223> Description of Artificial Sequence: DNA encoding
      an shRNA directed against the gene for the Homo
      sapiens ubiquitin specific protease 13
      (isopeptidase T-3)

<400> 183
gtagaggaga gttctgaaat tcaagagatt tcagaactct cctctac                47

<210> 184
<211> 47
<212> DNA
<213> Artificial Sequence

<220>
<223> Description of Artificial Sequence: DNA encoding
      an shRNA directed against the gene for the Homo
      sapiens ubiquitin specific protease 13
      (isopeptidase T-3)

<400> 184
gcctctaatc ctgataaggt tcaagagacc ttatcaggat tagaggc                47

<210> 185
<211> 47
<212> DNA
<213> Artificial Sequence

<220>
<223> Description of Artificial Sequence: DNA encoding
      an shRNA directed against the gene for the
      ubiquitin carboxyl-terminal hydrolase 28

<400> 185
gatgatcttc aggctgccat tcaagagatg gcagcctgaa gatcatc                47

<210> 186
<211> 47
<212> DNA
<213> Artificial Sequence

<220>
<223> Description of Artificial Sequence: DNA encoding
      an shRNA directed against the gene for the
      ubiquitin carboxyl-terminal hydrolase 28

<400> 186
gtatggacaa gagcgttggt tcaagagacc aacgctcttg tccatac                47

<210> 187
<211> 47
<212> DNA
<213> Artificial Sequence

<220>
<223> Description of Artificial Sequence: DNA encoding
      an shRNA directed against the gene for the
      ubiquitin carboxyl-terminal hydrolase 28

<400> 187
cgaacccttc tggaacagtt tcaagagaac tgttccagaa gggttcg                47


<210> 188
<211> 47
<212> DNA
<213> Artificial Sequence

<220>
<223> Description of Artificial Sequence: DNA encoding
      an shRNA directed against the gene for the
      ubiquitin carboxyl-terminal hydrolase 28

<400> 188
gtggcatgaa gattatagtt tcaagagaac tataatcttc atgccac                47


<210> 189
<211> 47
<212> DNA
<213> Artificial Sequence

<220>
<223> Description of Artificial Sequence: DNA encoding
      an shRNA directed against the ubiquitin
      carboxyl-terminal hydrolase 14

<400> 189
ggtgaacaag gacagtatct tcaagagaga tactgtcctt gttcacc                47


<210> 190
<211> 47
<212> DNA
<213> Artificial Sequence

<220>
<223> Description of Artificial Sequence: DNA encoding
      an shRNA directed against the gene for the
      ubiquitin carboxyl-terminal hydrolase 14

<400> 190
gcaatagagg atgattctgt tcaagagaca gaatcatcct ctattgc                47


<210> 191
<211> 47
<212> DNA
<213> Artificial Sequence

<220>
<223> Description of Artificial Sequence: DNA encoding
      an shRNA directed against the gene for the
      ubiquitin carboxyl-terminal hydrolase 14

<400> 191
tctgtgaatg ccaaagttct tcaagagaga actttggcat tcacaga                47


<210> 192
<211> 47
<212> DNA
<213> Artificial Sequence

<220>
<223> Description of Artificial Sequence: DNA encoding
      an shRNA directed against the gene for the
      ubiquitin carboxyl-terminal hydrolase 14

<400> 192
cacaccaggg aaggtctagt tcaagagact agaccttccc tggtgtg                47


<210> 193
<211> 47
<212> DNA
<213> Artificial Sequence

<220>
<223> Description of Artificial Sequence: DNA encoding
      an shRNA directed against the DUB1 gene

<400> 193
gcaggaagat gcccatgaat tcaagagatt catgggcatc ttcctgc                47


<210> 194
<211> 47
<212> DNA
<213> Artificial Sequence

<220>
<223> Description of Artificial Sequence: DNA encoding
      an shRNA directed agains the DUB1 gene

<400> 194
gaatgtgcaa tatcctgagt tcaagagact caggatattg cacattc                47


<210> 195
<211> 47
<212> DNA
<213> Artificial Sequence

<220>
<223> Description of Artificial Sequence: DNA encoding
      an shRNA directed against the DUB1 gene

<400> 195
tggatgatgc caaggtcact tcaagagagt gaccttggca tcatcca                47

<210> 196
<211> 47
<212> DNA
<213> Artificial Sequence

<220>
<223> Description of Artificial Sequence: DNA encoding
      an shRNA directed against the DUB1 gene

<400> 196
gctccgtgct aaacctctct tcaagagaga gaggtttagc acggagc                47


<210> 197
<211> 47
<212> DNA
<213> Artificial Sequence

<220>
<223> Description of Artificial Sequence: DNA encoding
      an shRNA directed against the gene for the mouse
      USP27 homolog

<400> 197
gcctccacct caacagaggt tcaagagacc tctgttgagg tggaggc                47


<210> 198
<211> 47
<212> DNA
<213> Artificial Sequence

<220>
<223> Description of Artificial Sequence: DNA encoding
      an shRNA directed against the gene for the mouse
      USP27 homolog

<400> 198
ctgcatcata gaccaaatct tcaagagaga tttggtctat gatgcag                47


<210> 199
<211> 47
<212> DNA
<213> Artificial Sequence

<220>
<223> Description of Artificial Sequence: DNA encoding
      an shRNA directed against the gene for the mouse
      USP27 homolog

<400> 199
gatcactaca tacatttcct tcaagagagg aaatgtatgt agtgatc                47


<210> 200
<211> 47
<212> DNA
<213> Artificial Sequence

<220>
<223> Description of Artificial Sequence: DNA encoding
      an shRNA directed against the gene for the mouse
      USP27 homolog

<400> 200
gtaaagagag cagaatgaat tcaagagatt cattctgctc tctttac                47


<210> 201
<211> 47
<212> DNA
<213> Artificial Sequence

<220>
<223> Description of Artificial Sequence: DNA encoding
      an shRNA directed against the gene for the
      ubiquitin carboxyl-terminal hydrolase 4

<400> 201
cgcggggcgc agtggtatct tcaagagaga taccactgcg ccccgcg                47


<210> 202
<211> 47
<212> DNA
<213> Artificial Sequence

<220>
<223> Description of Artificial Sequence: DNA encoding
      an shRNA directed against the gene for the
      ubiquitin carboxyl-terminal hydrolase 4

<400> 202
cagaaggcag tggggaagat tcaagagatc ttccccactg ccttctg                47


<210> 203
<211> 47
<212> DNA
<213> Artificial Sequence

<220>
<223> Description of Artificial Sequence: DNA encoding
      an shRNA directed against the gene for the
      ubiquitin carboxyl-terminal hydrolase 4

<400> 203
gcctgggaga atcacaggtt tcaagagaac ctgtgattct cccaggc                47


<210> 204
<211> 47
<212> DNA
<213> Artificial Sequence

<220>
<223> Description of Artificial Sequence: DNA encoding
      an shRNA directed against the gene for the
      ubiquitin carboxyl-terminal hydrolase 4

<400> 204
accagacaag gaaataccct tcaagagagg gtatttcctt gtctggt                47

<210> 205
<211> 47
<212> DNA
<213> Artificial Sequence

<220>
<223> Description of Artificial Sequence: DNA encoding
      an shRNA directed against the TRE-2 gene

<400> 205
cacatccacc acatcgacct tcaagagagg tcgatgtggt ggatgtg                47

<210> 206
<211> 47
<212> DNA
<213> Artificial Sequence

<220>
<223> Description of Artificial Sequence: DNA encoding
      an shRNA directed against the TRE-2 gene

<400> 206
gtcacaaccc aagaccatgt tcaagagaca tggtcttggg ttgtgac                47

<210> 207
<211> 47
<212> DNA
<213> Artificial Sequence

<220>
<223> Description of Artificial Sequence: DNA encoding
      an shRNA directed against the TRE-2 gene

<400> 207
ctcaacagga caaatcccat tcaagagatg ggatttgtcc tgttgag                47

<210> 208
<211> 47
<212> DNA
<213> Artificial Sequence

<220>
<223> Description of Artificial Sequence: DNA encoding
      an shRNA directed against the TRE-2 gene

<400> 208
tagatcaatt attgtggatt tcaagagaat ccacaataat tgatcta                47

<210> 209
<211> 47
<212> DNA
<213> Artificial Sequence

<220>
<223> Description of Artificial Sequence: DNA encoding
      an shRNA directed against the gene for the
      ubiquitin carboxyl-terminal hydrolase 15 (UNPH-2)

<400> 209
ggaacacctt attgatgaat tcaagagatt catcaataag gtgttcc                47


<210> 210
<211> 47
<212> DNA
<213> Artificial Sequence

<220>
<223> Description of Artificial Sequence: DNA encoding
      an shRNA directed against the gene for the
      ubiquitin carboxyl-terminal hydrolase 15 (UNPH-2)

<400> 210
ctttaacaga aattgtctct tcaagagaga gacaatttct gttaaag                47


<210> 211
<211> 47
<212> DNA
<213> Artificial Sequence

<220>
<223> Description of Artificial Sequence: DNA encoding
      an shRNA directed agaainst the gene for the
      ubiquitin carboxyl-terminal hydrolase 15 (UNPH-2)

<400> 211
cctatgcagt acaaagtggt tcaagagacc actttgtact gcatagg                47


<210> 212
<211> 47
<212> DNA
<213> Artificial Sequence

<220>
<223> Description of Artificial Sequence: DNA encoding
      an shRNA directed against the gene for the
      ubiquitin carboxyl-terminal hydrolase 15 (UNPH-2)

<400> 212
gatcttttct tgctttggat tcaagagatc caaagcaaga aaagatc                47


<210> 213
<211> 47
<212> DNA
<213> Artificial Sequence

<220>
<223> Description of Artificial Sequence: DNA encoding
      an shRNA directed against the KIAA1372 gene

<400> 213

cagcatcctt caggccttat tcaagagata aggcctgaag gatgctg                47

<210> 214
<211> 47
<212> DNA
<213> Artificial Sequence

<220>
<223> Description of Artificial Sequence: DNA encoding
      an shRNA directed against the KIAA1372 gene

<400> 214
gatagtgact cggatctgct tcaagagagc agatccgagt cactatc                47

<210> 215
<211> 47
<212> DNA
<213> Artificial Sequence

<220>
<223> Description of Artificial Sequence: DNA encoding
      an shRNA directed against the KIAA1372 gene

<400> 215
gacatcacag cccgggagtt tcaagagaac tcccgggctg tgatgtc                47

<210> 216
<211> 47
<212> DNA
<213> Artificial Sequence

<220>
<223> Description of Artificial Sequence: DNA encoding
      an shRNA directed against the KIAA1372 gene

<400> 216
ggacacagcc tatgtgctgt tcaagagaca gcacataggc tgtgtcc                47

<210> 217
<211> 47
<212> DNA
<213> Artificial Sequence

<220>
<223> Description of Artificial Sequence: DNA encoding
      an shRNA directed against the gene for the BRCA1
      associated protein-1

<400> 217
gtggaggaga tctacgacct tcaagagagg tcgtagatct cctccac                47

<210> 218
<211> 47
<212> DNA
<213> Artificial Sequence

<220>
<223> Description of Artificial Sequence: DNA encoding
      an shRNA directed against the gene for the BRCA1
      associated protein-1

<400> 218
ctcttgtgca actcatgcct tcaagagagg catgagttgc acaagag                47


<210> 219
<211> 47
<212> DNA
<213> Artificial Sequence

<220>
<223> Description of Artificial Sequence: DNA encoding
      an shRNA directed against the gene for the BRCA1
      associated protein-1

<400> 219
acagggcccc tgcagcctct tcaagagaga ggctgcaggg gccctgt                47


<210> 220
<211> 47
<212> DNA
<213> Artificial Sequence

<220>
<223> Description of Artificial Sequence: DNA encoding
      an shRNA directed against the gene for the BRCA1
      associated protein-1

<400> 220
gaagacctgg cggcaggtgt tcaagagaca cctgccgcca ggtcttc                47

## Description

The present invention relates to a biological entity carrying a regulator construct comprising a specific repressor gene and a responder construct comprising at least one segment corresponding to a short hairpin RNA (shRNA) or corresponding to complementary short interfering RNA (siRNA) strands, said at least one segment being under control of a promoter which contains an operator sequence corresponding to the repressor. The invention further relates to a method for preparing said biological entity and its use.

### Background of the Invention

RNA interference (RNAi) has been discovered some years ago as a tool for inhibition of gene expression (Fire, A. et al., Nature 391, 806-811 (1998)). It based on the introduction of double stranded RNA (dsRNA) molecules into cells, whereby one strand is complementary to the coding region of a target gene. Through pairing of the specific mRNA with the introduced RNA molecule, the mRNA is degraded by a cellular mechanism. Since long dsRNA provokes an interferon response in mammalian cells, the technology was initially restricted to organisms or cells showing no interferon response (Bass, B.L., Nature 411, 428-429 (2001)). The finding that *short* (< 30 bp) *interfering RNAs* (siRNA) circumvent the interferon response extended the application to mammalian cells (Elbashir, S.M. et al., Nature 411, 494-498 (2001)).

Although RNAi in mice has been in principle demonstrated, the current technology does not allow performing systematic gene function analysis *in vivo*. So far the inhibition of gene expression has been achieved by injection of *purified* siRNA into the tail vain of mice (McCaffrey, A.P. et al., Nature 418, 38-39 (2002); Lewis, D.H. et al., Nature Genet. 32, 107-108 (2002)). Using this approach, gene inhibition is restricted to specific organs and persists only a few days. A further improvement of the siRNA technology is based on the intracellular transcription of the two complementary siRNA strands using separate expression units both under the control of the U6 promoter (Lee, N.S. et al., J. Nat. Biotechnol. 20(5):500-5 (2002); Du, Q. et al., Biochem. Biophys. Res. Commun. 325(1):243-9 (2004); Miyagishi, M. and Taira, K., Methods Mol. Biol.;252:483-91 (2004)). The transgene based approach was further refined by the expression of short hairpin RNA (shRNA) molecules by a single transcription unit under the control of the U6 or H1 promoter (Brummelkamp, T.R. et al., Science 296, 550-553 (2002); Paddison, P.J. et al, Genes Dev. 16, 948-958 (2002); Yu, J.Y. et al., Proc. Natl. Acad. Sci. USA 99, 6047-6052 (2002); Sui, G. et al., Proc. Natl. Acad. Sci. USA 99, 5515-5520 (2002); Paul, C.P. et al., Nature Biotechnol. 20, 505-508 (2002); Xia, H. et al., Nat. Biotechnol. 10, 1006-10 (2002); Jacque, J.M. et al., Nature 418(6896):435-8 (2002)). The activity of shRNA in mice has been demonstrated by McCaffrey A.P. et al., Nature 418, 38-39 (2002) through injection of shRNA expression vectors into the tail vain. Again, gene inhibition was temporally and spatially restricted. Although these results demonstrate that the mechanism of shRNA mediated gene silencing is functional in mice, they do not clarify whether constitutive RNAi can be achieved in transgenic animals. Brummelkamp, T.R. et al., Science 296, 550-553 (2002), Paddison, P.J. et al., Genes Dev. 16, 948-958 (2002), Hemann, M.T. et al., Nat. Genet. 33(3):396-400 (2003); and Devroe, E. et al., BMC Biotechnol. 2(1):15 (2002) have shown the long-term inhibition of gene expression through stable integration of shRNA vectors in cultivated cell lines. These experiments included random integration of shRNA transgenes and screening for clones with appropriate siRNA expression, which is not applicable for testing of a large number of different shRNA transgenes in mice. Finally, several reports have demonstrated shRNA-mediated gene silencing in transgenic mice and rats (Hasuwa, H. et al., FEBS Lett. 532(1-2):227-30 (2002); Carmell, M.A. et al., Nat. Struct. Biol. 10(2):91 -2 (2003); Rubinson, D.A. et al., Nat. Genet. 33(3):401-6 (2003); Kunath, T. et al., Nat. Biotechnol. (2003)). However, these experiments again included random integration of shRNA transgenes resulting in variable levels and patterns of shRNA expression. Thus, testing of ES cell clones or mouse lines with appropriate shRNA expression had been required, which is a laborious and time-consuming undertaking.

The *in vivo* validation of genes by RNAi mediated gene repression in a large scale setting requires the expression of siRNA at sufficiently high levels and with a predictable pattern in multiple organs. Targeted transgenesis provides the only approach to achieve reproducible expression of transgenes in the living organism (e.g. mammalians such as mice).

Most siRNA expression vectors are based on polymerase III dependent (Pol III) promoters (U6 or H1) that allow the production of transcripts carrying only a few non-homologous bases at their 3' ends. It has been shown that the presence of non-homologous RNA at the ends of the shRNA stretches lower the efficiency of RNAi mediated gene silencing (Xia, H. et al., Nat. Biotechnol. 10, 1006-10 (2002)). WO04/035782 discloses that an ubiquitous promoter driven shRNA construct provides for RNAi-mediated gene inhibition in multiple organs of the living organism. Further, an inducible gene expression system, e.g. a system based on the tetracycline dependent repressor, is suggested which allows temporary control of RNAi mediated gene silencing in transgenic cells lines and living organism. The configuration of said inducible systems as well as the choice of the repressor appeared critical with regard to the expression of inducible RNAi in multiple organs without background activity.

Temporary control of shRNA expression can be achieved by using engineered promoters containing a tetracycline operator (tetO) sequence (Ohkawa, J. and Taira, K., Hum. Gene Ther. 11(4):577-85 (2000)). The Tetracycline operator itself has no effect on shRNA expression. In the presence of the tetracycline repressor (tetR), however, transcription is blocked through binding of the repressor to the tetO sequence. De-repression is achieved by adding the inductor doxycycline, that causes the release of the TetR protein from the tetO site and allows transcription from the H1 promoter. Several attempts have been made to apply this strategy for the temporary control of antigens or shRNA expression in cultured cell lines (Ohkawa, J. and Taira, K., Hum. Gene Ther. 11(4):577-85 (2000); van de Wetering, M. et al., EMBO reports VOL 4, NO 6:609-615 (2003); Matsukura, 2003; Czauderna, F. et al., Nucleic Acids Res., 31(21):e127 (2003)). In these reports, the degree of doxycycline-inducible mRNA degradation was variable. In addition, background RNAi activity in the uninduced state was observed (van de Wetering, M. et al., EMBO reports VOL 4, No. 6:609-615 (2003)), indicating a limiting level of tetR expression in these cell lines.

The properties of such Doxycycline-responsive promoters br siRNA expression have so far not been tested in transgenic animals. In addition, the level of shRNA expression required for efficient RNAi has never been determined and, vice versa, it is unknown whether or to which extent a basal level of shRNA expression is tolerated without significant RNAi in the uninduced state of the system. It is therefore not obvious whether a tight control of RNAi can be achieved through Doxycycline inducible expression of shRNA transgenes in living animals.

Difficulties in expression of the lac repressor and tetR in transgenic animals have been attributed to their prokaryotic origin (Scrable & Stambrook, Genetics 147:297-304 (1997); Wells, D.J., Nucleic Acids Res., 27(11):2408-15 (1999); Urlinger, S. et al., Proc. Natl. Acad. Sci. U S A 97(14): 7963-8 (2000)). Alteration of the coding region by changing unfrequently used codons and eliminating putative mammalian processing signals improved the expression of these sequences (Zhang et al., Gene 105:61-72 (1991)). Scrable & Stambrook, Genetics 147:297-304 (1997) were able to show expression of a codon optimized lac repressor by Northern analysis in transgenic animals, but were unable to detect protein expression and failed to prove the activity of the repressor. Cronin, C.A. et al., Genes and Development 15:1506-1517 (2001) demonstrated that the expression of the lac repressor could only be achieved by a empirically combination of synthetic and wt parts of the repressor. No general prediction for transgene expression of bacterial genes in mice could be made, indicating that the codon optimization alone is not sufficient for improved transgene activity.

The provision of an inducible system allowing tight temporal control of RNAi in multicellular organisms without background activity was highly desirable.

### Summary of the Invention

It was surprisingly found that a codon-optimized repressor gene, such as the tetracycline repressor gene, completely suppresses the activity of shRNA/siRNA genes under the control of a promoter containing the corresponding operator, such as a tetO containing promoter, in transgenic animals. In contrast thereto the same configuration with the non codon-optimized tetracycline repressor gene showed a high degree of shRNA/siRNA background activity in transgenic anim als in the absence of doxycyclin induction. Thus, the present invention provides
(1) a biological entity selected from a vertebrate, a tissue culture derived from a vertebrate or one or more cells of a cell culture derived from a vertebrate, said biological entity carrying
   (i) a responder construct comprising at least one segment corresponding to a short hairpin RNA (shRNA) or to complementary short interfering RNA (siRNA) strands, said at least one segment being under control of a ubiquitous promoter and said promoter containing an operator sequence being perfectly regulatable by a repressor; and
   (ii) a regulator construct comprising a codon-optimized repressor gene, which provides for perfect regulation of the promoter containing the operator sequence of the responder construct;
(2) a method for preparing the biological entity as defined in (1) above or a method for constitutive and/or inducible gene knock down in a biological entity, which method comprises stably integrating
   (i) a responder construct as defined in (1) above, and
   (ii) a regulator construct as defined in (1) above
      into the genome of the biological entity; and
(3) the use of a biological entity as defined in (1) above for inducible gene knock down.

### Brief Description of the Figures

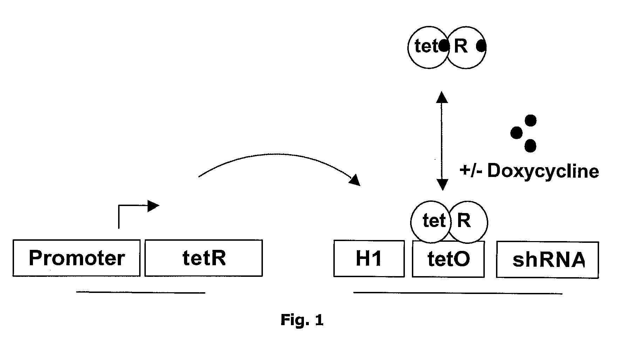

Fig. 1: Principle of the Doxycycline inducible gene expression system. The tetR acts as a doxycycline-controlled transcriptional repressor. This protein binds to a modified H1-tetO sequence via the tet operator sequences in the absence of doxycycline and represses transcription.
Fig. 2: Vectors for Pol III promoter based tet-repression system (inducible):
   A: Insertion of a wt tetracycline repressor gene (SEQ ID NO:1) or codon-optimized tetracycline repressor gene (SEQ ID NO:2) under control of a CAGGS promoter into the rosa26 locus.
   B: Insertion of a shRNA containing responder construct into a ubiquitous expressed genomic locus. The transcription of the Pol II dependent Rosa26 promoter will be stopped by the synthetic polyadenylation signal (pA) and a hGH pA. An inducible Pol I I I promoter controls the expression of sh RNA. The transcript is stopped by five thymidine bases (SEQ ID NO:3).

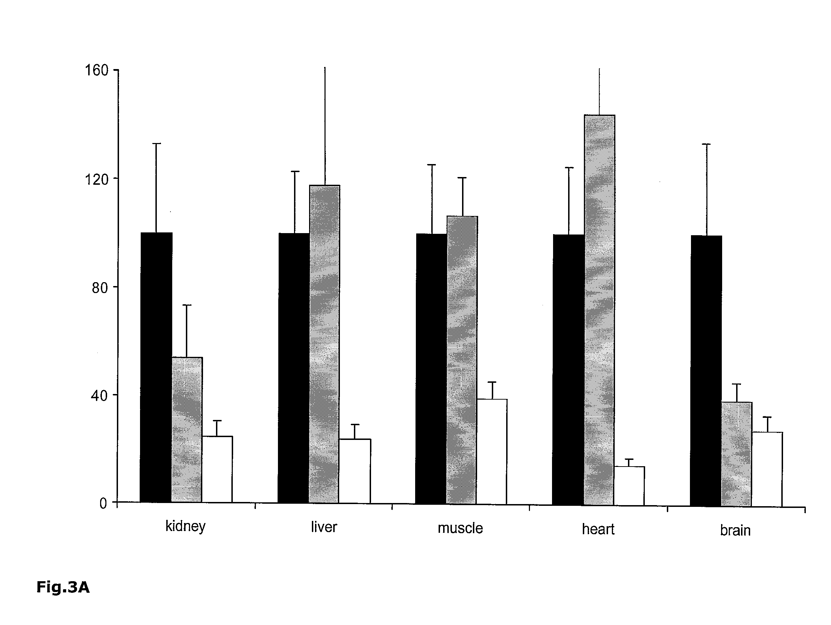

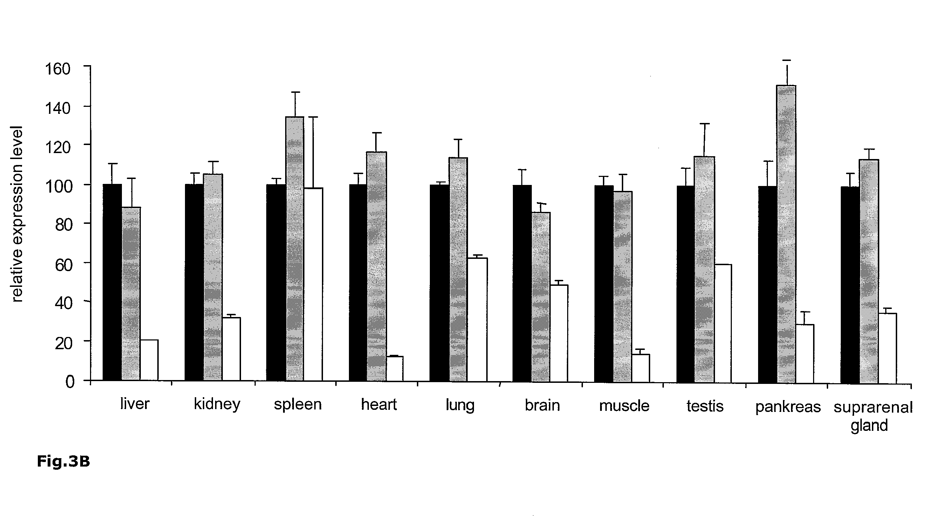

Fig. 3: ShRNA-mediated inhibition of luciferase expression in mice feeding doxycycline. Firefly luciferase activity in mice in the absence (black bars) or presence of H1 -tetO-shRNA transgenes (uninduced: grey bars; induced through 10 days feeding with doxycycline: white bars), respectively. All mice carried the firefly and the Renilla luciferase transgenes. Relative values of Firefly luciferase activitiy in different organs are given as indicated. All values of Fluc activity were normalized by using the Rluc activity for reference (+/-SEM). In A all mice carried the wt tet repressor, whereas in B all mice carried the codon optimized tet repressor.

### Detailed Description of the Invention

The "biological entity" according to the present invention includes, but is not limited to, a vertebrate, a tissue culture derived from a vertebrate, or one or more cells of a cell culture derived from a vertebrate.

The term "vertebrate" according to the present invention relates to multi-cellular organisms such as mammals, e.g. non-human animals such as rodents (including mice, rats, etc.) and humans, or non-mammals, e.g. fish. Most preferred vertebrates are mice and fish.

"Tissue culture" according to the present invention refers to parts of the above defined "vertebrates" (including organs and the like) which are cultured *in vitro.* "Cell culture" according to the present invention includes cells isolated from the above defined "vertebrates" which are cultured *in vitro.* These cells can be transformed (immortalized) or untransformed (directly derived from vertebrates; primary cell culture).

The "responder construct" and the "regulator construct" according to the invention of the present application are suitable for stable integration into the "vertebrates" or into cells of the cell culture, e.g. by homologous recombination, recombinase mediated cassette exchange (hereinafter "RMCE") reaction, or random integration. The vector(s) for integration of the constructs into the vertebrates by homologous recombination preferably contain(s) homologous sequences suitable for targeted integration at a defined locus, preferably at a polymerase II or III dependent locus of the living organisms or cells of the cell culture. Such polymerase II or III dependent loci include, but are not limited to, the Rosa26 locus (the murine Rosa26 locus being depicted in SEQ ID NO:11), collagen, RNA polymerase, actin, and HPRT. Homologous sequences suitable for integration into the murine Rosa26 locus are shown in SEQ ID Nos: 6 and 7.

The responder constuct contains at least one ubiquitous promoter which controls the expression of the at least one segment corresponding to a short hairpin RNA (shRNA) or to complementary short interfering RNA (siRNA) strands (in the following shortly referred to as "shRNA segment" and "siRNA segment", respectively). The segment corresponding to th shRNA and siRNA are preferably comprised of DNA. The regulator construct may also contain ubiquitous promoter(s) (constitutive, inducible or the like). Preferably the ubiquitous promoter of the regulator and/or responder construct is selected from polymerase I, II and I I I dependent prom oters, most preferably is a polymerase II or III dependent promoter including, but not limited to, a CMV promoter, a CAGGS promoter (see nucleotides 3231-4860 of SEQ ID NO:1), a snRNA promoter such as U6, a RNAse P RNA promoter such as H1, a tRNA promoter, a 7SL RNA promoter, a 5 S rRNA promoter, etc.

The ubiquitous promoter of the "responder construct" contains an operator sequence allowing for "perfect regulation" by a corresponding repressor. "Perfect regulation" and "perfectly regulatable" within the meaning of the invention means that it permits control of the expression to an extend that no significant background activity is determined in the biological entity. This means that the suppression of the expression of the shRNA/siRNA is controlled by a rate of at least 90%, preferably by at least 95%, more preferably by at least 98%, and most preferably by 100%. Suitable operator sequences are such operator sequences which render the promoter susceptible to regulation by the corresponding codon-optimized repressor gene present within the regulator construct, including, but not limited to, tetO, GalO, lacO, etc.

The responder construct may further contain functional sequences selected from splice acceptor sequences (such as a splice acceptor of adenovirus (see nucleotides 1129-1249 of SEQ ID NO:1), etc.), polyadenylation sites (such as synthetic polyadenylation sites (see nucleotides 2995-3173 of SEQ ID NO:1), the polyadenylation site of human growth hormones (see nucleotides 4977-5042 of SEQ ID NO:1), or the like), selectable marker sequences (such as the neomycin phosphotransferase gene of *E. coli* transposon, etc.), recombinase recognition sequences (such as loxP, FRT, etc), and so on.

Particularly preferred responder constructs carry a Pol III dependent promoter (inducible U6, H1 or the like) containing tetO (for H1 -tetO see nucleotides 4742-4975 of SEQ ID NO:3), and the at least one shRNA segment or siRNA segment. Particularly preferred regulator constructs carry a polymerase II (Pol II) dependent promoter (CMV, CAGGS or the like) and the codon optimized repressor gene tet.

In case shRNA segments are utilized within the responder construct, the responder construct preferably comprises at least one shRNA segment having a nucleotide (e.g. DNA) sequence of the structure A-B-C or C-B-A. In case siRNA segments are utilized within the responder construct, the responder construct preferably comprises at least least two DNA segments A and C or C and A, wherein each of said at least two segments is under the control of a separate promoter as defined above (such as the Pol III promoter including inducible U6, H1 or the like). In the above segments
A is a 15 to 35, preferably a 19 to 29 bp DNA sequence being at least 90%, preferably 100% complementary to the gene to be knocked down (e.g. firefly luciferase, p53, etc.);
B is a spacer DNA sequence having 5 to 9 bp forming the loop of the expressed RNA hairpin molecule, and
C is a 15 to 35, preferably a 19 to 29 bp DNA sequence being at least 85% complementary to the sequence A.

The above shRNA and siRNA segments may further comprise stop and/or polyadenylation sequences.

Suitable siRNA sequences for the knockdown of a given target gene are well known in the art (e.g. the particular siRNA sequences mentioned in Lee N.S. et al., J. Nat. Biotechnol. 20(5):500-5 (2002) gcctgtgcctcttcagctacc (SEQ ID NO: 12) and gcggagacagcgacgaagagc (SEQ ID NO: 13) and in Du, Q. et al., Nucl. Acids Res. 21; 33(5):1671-7 (2005) cttattggagagagcacga (SEQ ID NO:14)) or can readily be determined by the skilled artisan.

Suitable shRNA sequences for the knock down of a given target gene are well known in the art (see e.g. the particular shRNA sequences mentioned in Tables 1 and 2 below) or can readily be determined by the skilled artisan.

**Table 1 :**

| target gene | shRNA sequence /SEQ ID NO | Reference |
|---|---|---|
| CDH-1 | TgagaagtctcccagtcagTTCAAGAGActgactgggagacttctca (19) | Brummelkamp |
| p53 | GactccagtggtaatctacTTCAAGAGAgtagattaccactggagtc (20) | et al., |
| CDC20 | CggcaggactccgggccgaTTCAAGAGAtcggcccggagtcctgccg (21) | Science, 296: 550-3 (2002). |
| CYLD | CctcatgcagttctctttgTTCAAGAGAcaaagagaactgcatgagg (22) | Kovalenko et al, Nature, 424:801-5 (2003). |
| Ras-Gap | AagatgaagccactccctatttCAAGAGAaaatagggagtggcttcatctt (23) | Kunath et al., Nature Biotechnology, 21:559-561 (2003). |
| tubulin | GacagagccaagtggactcACAgagtccacttggctctgtc (24) | Yu et al., PNAS, 99: 6047-52 (2002) |
| lam in | Ctggacttccagaagaacattcgtgttcttctggaagtccag (25) | Paul et al., Nature Bio-technology, 20:505-8 (2002). |

**Table 2 shRNA sequences known from Brummelkamp et al., Nature, 424:797-801 (2003):**

| Target Gene | shRNA Sequence / SEQ ID NO |
|---|---|
| UBIQUITIN | GAGATTGGTCCAGAACAGTTTCAAGAGAACTGTTCTGGACCAATCTC (26) |
| CARBOXYL- | GCCCTTCCGATCATGGTAGTTCAAGAGACTACCATGATCGGAAGGGC (27) |
| TERMINAL | TCTTTAGAATTCTTAAGTATTCAAGAGATACTTAAGAATTCTAAAGA (28) |
| HYDROLASE 12 | CATTAGCTATATCAACATGTTCAAGAGACATGTTC-ATATAGCTAATG (29) |
| | |
| UBIQUITIN | ACCACAAACGGCGGAACGATTCAAGAGATCGTTCCGCCGTTTGTGGT (30) |
| CARBOXYL- | GAGGGTCTTGGAGGTCTTCTTCAAGAGAGAAGACCTCCAAGACCCTC (31) |
| TERMINAL | GTCCATGCCCAGCCGTACATTCAAGAGATGTACGGCTGGGCATGGAC (32) |
| HYDROLASE 11 | GCTGGACACCCTCGTGGAGTTCAAGAC-ACTCCACGAGGGTGTCCAGC (33) |
| | |
| UBIQUITIN | GAATATCAGAGAATTGAGTTTCAAGAGAACTCAATTCTCTGATATTC (34) |
| CARBOXYL- | TGGACTTCATGAGGAAATGTTCAAGAGACATTTCCTCATGAAGTCCA (35) |
| TERMINAL | TATTGAATATCCTGTGGACTTCAAGAGAGTCCACAGGATATTCAATA (36) |
| HYDROLASE 10 | TTGTACTGAGAGAAACTGCTTCAAGAGAGCAGTTTCTCTCAGTACAA (37) |
| | |
| HAUSP | GATCAATGATAGGTTTGAATTCAAGAGATTCAAACCTATCATTGATC (38) |
| | GGAGTTTGAGAAGTTTAAATTCAAGAGATTTAAACTTCTCAAACTCC (39) |
| | GAACTCCTCGCTTGCTGAGTTCAAGAGACTCAGCAAGCGAGGAGTTC (40) |
| | CCGAATTTAACAGAGAGAATTCAAGAGATTCTCTCTGTTAAATTCGG (41) |
| | |
| UBIQUITIN | GACAGCAGAAGAATGCAGATTCAAGAGATCTGCATTCTTCTGCTGTC (42) |
| CARBOXYL- | ATAAAGCTCAACGAGAACCTTCAAGAGAGGTTCTCGTTGAGCTTTAT (43) |
| TERMINAL | GGTGAAGTGGCAGAAGAATTTCAAGAGAATTCTTCTGCCACTTCACC (44) |
| HYDROLASE 8 | GTATTGCAGTAATCATCACTTCAAGAGAGTGATGATTACTGCAATAC (45) |
| | |
| FLJ10785 | GATATGGGGTTCCATGTCATTCAAGAGATGACATGGAACCCCATATC (46) |
| | GGAGACATGGTTCTTAGTGTTCAAGAGACACTAAGAACCATGTCTCC (47) |
| | AGCACCAAGTTCGTCTCAGTTCAAGAGACTGAGACGAACTTGGTGCT (48) |
| | GATGCAACACTGAAAGAACTTCAAGAGAGTTCTTTCAGTGTTGCATC (49) |
| | |
| KIAA0710 | GTCAATGGCAGTGATGATATTCAAGAGATATCATCACTGCCATTGAC (50) |
| | CCTGCTAGCTGCCTGTGGCTTCAAGAGAGCCACAGGCAGCTAGCAGG (51) |
| | CCACCTTTGCCAGAAGGAGTTCAAGAGACTCCTTCTGGCAAAGGTGG (52) |
| | CCCTATTGAGGCAAGTGTCTTCAAGAGAGACACTTGCCTCAATAGGG (53) |
| | |
| FLJ12552/ | GAAGGAAAACTTGCTGACGTTCAAGAGACGTCAGCAAGTTTTCCTTC (54) |
| FLJ14256 | CTCACCTGGGTCCATGAGATTCAAGAGATCTCATGGACCCAGGTGAG (55) |
| | GCTGTCTTACCGTGTGGTCTTCAAGAGAGACCACACGGTAAGACAGC (56) |
| | CCTGGACCGCATGTATGACTTCAAGAGAGTCATACATGCGGTCCAGG (57) |
| | |
| KIAA1203 | GTCAATGGCAGTGATGATATTCAAGAGATATCATCACTGCCATTGAC (58) |
| | CCTGCTAGCTGCCTGTGGCTTCAAGAGAGCCACAGGCAGCTAGCAGG (59) |
| | CCACCTTTGCCAGAAGGAGTTCAAGAGACTCCTTCTGGCAAAGGTGG (60) |
| | CCCTATTGAGGCAAGTGTCTTCAAGAGAGACACTTGCCTCAATAGGG (61) |
| | |
| FLJ23277 | GGAAATCCGAATTGCTTGGTTCAAGAGACCAAGCAATTCGGATTTCC (62) |
| | CACATTTCTTCAAGTGTGGTTCAAGAGACCACACTTGAAGAAATGTG (63) |
| | CAGCAGGATGCTCAAGAATTTCAAGAGAATTCTTGAGCATCCTGCTG (64) |
| | GCTGAATACCTACATTGGCTTCAAGAGAGCCAATGTAGGTATTCAGC (65) |
| | |
| FLJ14914 (similar | GGGCTTGTGCCTGGCCTTGTTCAAGAGACAAGGCCAGGCACAAGCCC (66) |
| to UBP4) | GCCTTGTCCTGCCAAGAAGTTCAAGAGACTTCTTGGCAGGACAAGGC (67) |
| | GATTGAAGCCAAGGGAACGTTCAAGAGACGTTCCCTTGGCTTCAATC (68) |
| | TGGCGCCTGCTCCCCATCTTTCAAGAGAAGATGGGGAGCAGGCGCCA (69) |
| | |
| UBIQUITIN CAR | GAACCAGCAGGCTCTGTGGTTCAAGAGACCACAGAGCCTGCTGGTTC (70) |
| BOXYL- TERM I NAL | GGAAGCATAATTATCTGCCTTCAAGAGAGGCAGATAATTATGCTTCC (71) |
| HYDROLASE | AGAAGAAGATGCTTTTCACTTCAAGAGAGTGAAAAGCATCTTCTTCT (72) |
| ISOZYME L5 | CTTGCAGAGGAGGAACCCATTCAAGAGATGGGTTCCTCCTCTGCAAG (73) |
| | |
| UBIQUITIN CAR- | GCAAACAATCAGCAATGCCTTCAAGAGAGGCATTGCTGATTGTTTGC (74) |
| BOXYL- TERMINAL | TTGGACTGATTCATGCTATTTCAAGAGAATAGCATGAATCAGTCCAA (75) |
| HYDROLASE | CTGGCAATTCGTTGATGTATTCAAGAGATACATCAACGAATTGCCAG (76) |
| ISOZYME L3 | TTAGATGGGCGGAAGCCATTTCAAGAGAATGGCTTCCGCCCATCTAA (77) |
| | |
| UBIQUITIN CAR- | GAGGAGTCTCTGGGCTCGGTTCAAGAGACCGAGCCCAGAGACTCCTC (78) |
| BOXYL- TERMINAL | GAGCTGAAGGGACAAGAAGTTCAAGAGACTTCTTGTCCCTTCAGCTC (79) |
| HYDROLASE | TGTCGGGTAGATGACAAGGTTCAAGAGACCTTGTCATCTACCCGACA (80) |
| ISOZYME L1 | CACAGCTGTTCTTCTGTTCTTCAAGAGAGAACAGAAGAACAGCTGTG (81) |
| | |
| KIAA1891 / | GTGGAAGCCTTTACAGATCTTCAAGAGAGATCTGTAAAGGCTTCCAC (82) |
| FLJ25263 | CAACAGCTGCCTTCATCTGTTCAAGAGACAGATGAAGGCAGCTGTTG (83) |
| | CCATAGGCAGTCCTCCTAATTCAAGAGATTAGGAGGACTGCCTATGG (84) |
| | TGTATCACTGCCACTGGTTTTCAAGAGAAACCAGTGGCAGTGATACA (85) |
| | |
| FLJ14528 (similar | CATGTTGGGCAGCTGCAGCTTCAAGAGAGCTGCAGCTGCCCAACATG (86) |
| to UBP8) | CACAACTGGAGACCTGAAGTTCAAGAGACTTCAGGTCTCCAGTTGTG (87) |
| | GTATGCCTCCAAGAAAGAGTTCAAGAGACTCTTTCTTGGAGGCATAC (88) |
| | CTTCACAGTACATTTCTCTTTCAAGAGAAGAGAAATGTACTGTGAAG (89) |
| | |
| U4/U6 TRI | SNRNPGTACTTTCAAGGCCGGGGTTTCAAGAGAACCCCGGCCTTGAAAGTAC (90) |
| 65 kDa protein | CTTGGACAAGCAAGCCAAATTCAAGAGATTTGGCTTGCTTGTCCAAG (91) |
| | GACTATTGTGACTGATGTTTTCAAGAGAAACATCAGTCACAATAGTC (92) |
| | GGAGAACTTTCTGAAGCGCTTCAAGAGAGCGCTTCAGAAAGTTCTCC (93) |
| | |
| XM_089437 | GACGAGAGAAACCTTCACCTTCAAGAGAGGTGAAGGTTTCTCTCGTC (94) |
| | ACATTATTCTACATTCTTTTTCAAGAGAAAAGAATGTAGAATAATGT (95) |
| | AGATTCGCAAATGGATGTATTCAAGAGATACATCCATTTGCGAATCT (96) |
| | CATTCCCACCATGAGTCTGTTCAAGAGACAGACTCATGGTGGGAATG (97) |
| | |
| KIAA1453 | GATCGCCCGACACTTCCGCTTCAAGAGAGCGGAAGTGTCGGGCGATC (98) |
| | CCAGCAGGCCTACGTGCTGTTCAAGAGACAGCACGTAGGCCTGCTGG (99) |
| | GCCAGCTCCTCCACAGCACTTCAAGAGAGTGCTGTGGAGGAGCTGGC (100) |
| | CGCCGCCAAGTGGAGCAGATTCAAGAGATCTGCTCCACTTGGCGGCG (101) |
| | |
| FLJ12697 | GAAGATGCCCATGAATTCCTTCAAGAGAGGAATTCATGGGCATCTTC (102) |
| | CAAACAGGCTGCGCCAGGCTTCAAGAGA GCCTGGCGCAGCCTGTTTG (103) |
| | ACGGCCTAGCGCCTGATGGTTCAAGAGACCATCAGGCGCTAGGCCGT (104) |
| | CTGTAACCTCTCTGATCGGTTCAAGAGACCGATCAGAGAGGTTACAG (105) |
| | |
| UBIQUITIN | TCTGTCAGTCCATCCTGGCTTCAAGAGAGCCAGGATGGACTGACAGA (106) |
| SPECIFIC | TGAAGCGAGAGTCTTGTGATTCAAGAGATCACAAGACTCTCGCTTCA (107) |
| PROTEASE 18 | GATGGAGTGCTAATGGAAATTCAAGAGATTTCCATTAGCACTCCATC (108) |
| (USP18) | CCTTCAGAGATTGACACGCTTCAAGAGAGCGTGTCAATCTCTGAAGG (109) |
| | |
| UBIQUITIN | CCTGACCACGTTCCGACTGTTCAAGAGACAGTCGGAACGTGGTCAGG (110) |
| CARBOXYL- | GAGTTCCTTCGCTGCCTGATTCAAGAGATCAGGCAGCGAAGGAACTC (111) |
| TERMINAL | GACTGCCTTGCTGCCTTCTTTCAAGAGAAGAAGGCAGCAAGGCAGTC (112) |
| HYDROLASE 20 | CGCCGAGGGCTACGTACTCTTCAAGAGAGAGTACGTAGCCCTCGGCG (113) |
| | |
| UBIQUITIN | GGCGAGAAGAAAGGACTGTTTCAAGAGAACAGTCCTTTCTTCTCGCC (114) |
| CARBOXYL- | GGACGAGAATTGATAAAGATTCAAGAGATCTTTATCAATTCTCGTCC (115) |
| TERMINAL | GCACGAGAATTTGGGAATCTTCAAGAGAGATTCCCAAATTCTCGTGC (116) |
| HYDROLASE 24 | CTACTTCATGAAATATTGGTTCAAGAGACCAATATTTCATGAAGTAG (117) |
| | |
| KIAA1594 | GATAACAGCTTCTTGTCTATTCAAGAGATAGACAAGAAGCTGTTATC (118) |
| | GAGAATAGGACATCAGGGCTTCAAGAGAGCCCTGATGTCCTATTCTC (119) |
| | CTTGGAAGACTGAACCTGTTTCAAGAGAACAGGTTCAGTCTTCCAAG (120) |
| | CAACTCCTTTGTGGATGCATTCAAGAGATGCATCCACAAAGGAGTTG (121) |
| | |
| KIAA1350 | GATGTTGTCTCCAAATGCATTCAAGAGATGCATTTGGAGACAACATC (122) |
| | CGTGGGGACTGTACCTCCCTTCAAGAGAGGGAGGTACAGTCCCCACG (123) |
| | GTACAGCTTCAGAACCAAGTTCAAGAGACTTGGTTCTGAAGCTGTAC (124) |
| | |
| UBIQUITIN | GATGATCTTCAGAGAGCAATTCAAGAGATTGCTCTCTGAAGATCATC (125) |
| CARBOXYL- | GGAACATCGGAATTTGCCTTTCAAGAGAAGGCAAATTCCGATGTTCC (126) |
| TERMINAL | GAGCTAGTGAGGGACTCTTTTCAAGAGAAAGAGTCCCTCACTAGCTC (127) |
| HYDROLASE 25 | GCAGGGTTCTTTAAGGCAATTCAAGAGATTGCCTTAAAGAACCCTGC (128) |
| | |
| UBIQUITIN | TCGATGATTCCTCTGAAACTTCAAGAGAGTTTCAGAGGAATCATCGA (129) |
| CARBOXYL- | GATAATGGAAATATTGAACTTCAAGAGAGTTCAATATTTCCATTATC (130) |
| TERMINAL | GTTCTTCATTTAAATGATATTCAAGAGATATCATTTAAATGAAGAAC (131) |
| HYDROLASE 16 | GTTAACAAACACATAAAGTTTCAAGAGAACTTTATGTGTTTGTTAAC (132) |
| | |

| | |
|---|---|
| USP9X | GTTAGAGAAGATTCTTCGTTTCAAGAGAACGAAGAATCTTCTCTAAC (133) |
| | GTTGATTGGACAATTAAACTTCAAGAGAGTTTAATTGTCCAATCAAC (134) |
| | GGTTGATACCGTAAAGCGCTTCAAGAGAGCGCTTTACGGTATCAACC (135) |
| | GCAATGAAACGTCCAATGGTTCAAGAGACCATTGGACGTTTCATTGC (136) |
| | |
| USP9Y | AGCTAGAGAAAATTCTTCGTTCAAGAGACGAAGAATTTTCTCTAGCT (137) |
| | GATCCTATATGATGGATGATTCAAGAGATCATCCATCATATAGGATC (138) |
| | GTTCTTCTTGTCAGTGAAATTCAAGAGATTTCACTGACAAGAAGAAC (139) |
| | CTTGAGCTTGAGTGACCACTTCAAGAGAGTGGTCACTCAAGCTCAAG (140) |
| | |
| UBIQUITIN | GACCGGCCAGCGAGTCTACTTCAAGAGAGTAGACTCGCTGGCCGGTC (141) |
| CARBOXYL- | GGACCTGGGCTACATCTACTTCAAGAGAGTAGATGTAGCCCAGGTCC (142) |
| TERMINAL | CTCTGTGGTCCAGGTGCTCTTCAAGAGAGAGCACCTGGACCACAGAG (143) |
| HYDROLASE 5 | GACCACACGATTTGCCTCATTCAAGAGATGAGGCAAATCGTGTGGTC (144) |
| | |
| UBIQUITIN | TGGCTTGTTTATTGAAGGATTCAAGAGATCCTTCAATAAACAAGCCA (145) |
| CARBOXYL- | GTGAATTTGGGGAAGATAATTCAAGAGATTATCTTCCCCAAATTCAC (146) |
| TERMINAL | CGCTATAGCTTGAATGAGTTTCAAGAGAACTCATTCAAGCTATAGCG (147) |
| HYDROLASE 26 | GATATCCTGGCTCCACACATTCAAGAGATGTGTGGAGCCAGGATATC (148) |
| | |
| KIAA1097 | GAGCCAGTCGGATGTAGATTTCAAGAGAATCTACATCCGACTGGCTC (149) |
| | GTAAATTCTGAAGGCGAATTTCAAGAGAATTCGCCTTCAGAATTTAC (150) |
| | GCCCTCCTAAATCAGGCAATTCAAGAGATTGCCTGATTTAGGAGGGC (151) |
| | GTTGAGAAATGGAGTGAAGTTCAAGAGACTTCACTCCATTTCTCAAC (152) |
| | |
| UBI QUI TI N | GCTTGGAAAATGCAAGGCGTTCAAGAGACGCCTTGCATTTTCCAAGC (153) |
| SPECIFIC | CTGCATCATAGACCAGATCTTCAAGAGAGATCTGGTCTATGATGCAG (154) |
| PROTEASE 22 | GATCACCACGTATGTGTCCTTCAAGAGAGGACACATACGTGGTGATC (155) |
| (USP22) | TGACAACAAGTATTCCCTGTTCAAGAGACAGGGAATACTTGTTGTCA (156) |
| | |
| UBIQUITIN- | GAAATATAAGACAGATTCCTTCAAGAGAGGAATCTGTCTTATATTTC (157) |
| SPECIFIC | CCCATCAAGTTTAGAGGATTTCAAGAGAATCCTCTAAACTTGATGGG (158) |
| PROCESSING | GGTGTCCCATGGGAATATATTCAAGAGATATATTCCCATGGGACACC (159) |
| PROTEASE 29 | GAATGCCGACCTACAAAGATTCAAGAGATCTTTGTAGGTCGGCATTC (160) |
| | |
| CYLD | CAGTTATATTCTGTGATGTTTCAAGAGAACATCACAGAATATAACTG (161) |
| | GAGGTGTTGGGGACAAAGGTTCAAGAGACCTTTGTCCCCAACACCTC (162) |
| | GTGGGCTCATTGGCTGAAGTTCAAGAGACTTCAGCCAATGAGCCCAC (163) |
| | GAGCTACTGAGGACAGAAATTCAAGAGATTTCTGTCCTCAGTAGCTC (164) |
| | |
| UBIQUITIN | TCAGCAGGATGCTCAGGAGTTCAAGAGACTCCTGAGCATCCTGCTGA (165) |
| CARBOXYL- | GAAGTTCTCCATCCAGAGGTTCAAGAGACCTCTGGATGGAGAACTTC (166) |
| TERMINAL | GCCGGTCCCCACCAGCAGCTTCAAGAGAGCTGCTGGTGGGGACCGGC (167) |
| HYDROLASE 2 | CACTCGGGAGTTGAGAGATTTCAAGAGAATCTCTCAACTCCCGAGTG (168) |
| | |
| UBI QUI TI N | GCCCTTGGGTCTGTTTGACTTCAAGAGAGTCAAACAGACCCAAGGGC (169) |
| SPECIFIC | CTCAACACTAAACAGCAAGTTCAAGAGACTTGCTGTTTAGTGTTGAG (170) |
| PROTEASE 3 | GATTTCATTGGACAGCATATTCAAGAGATATGCTGTCCAATGAAATC (171) |
| (USP3) | CATGGGGCACCAACTAATTTTCAAGAGAAATTAGTTGGTGCCCCATG (172) |
| | |

| | |
|---|---|
| UBIQUITIN | GGTGTCTCTGCGGGATTGTTTCAAGAGAACAATCCCGCAGAGACACC (173) |
| CARBOXYL- | AGTTCAGTAGGTGTAGACTTTCAAGAGAAGTCTACACCTACTGAACT (174) |
| TERMINAL | GAGTTCCTGAAGCTCCTCATTCAAGAGATGAGGAGCTTCAGGAACTC 175) |
| HYDROLASE 23 | GGATTTGCTGGGGGCAAGGTTCAAGAGACCTTGCCCCCAGCAAATCC (176) |
| | |
| UBP-32.7 | CTCAGAAAGCCAACATTCATTCAAGAGATGAATGTTGGCTTTCTGAG (177) |
| | CGCATTGTAATAAGAAGGTTTCAAGAGAACCTTCTTATTACAATGCG (178) |
| | GGGAGGAAAATGCAGAAATTTCAAGAGAATTTCTGCATTTTCCTCCC (179) |
| | TTACAAATTTAGGAAATACTTCAAGAGAGTATTTCCTAAATTTGTAA (180) |
| | |
| HOMO SAPIENS | GTTATGAATTGATATGCAGTTCAAGAGACTGCATATCAATTCATAAC (181) |
| UBIQUITIN SPE- | GTGATAACACAACTAATGGTTCAAGAGACCATTAGTTGTGTTATCAC (182) |
| CIFIC PROTEASE | GTAGAGGAGAGTTCTGAAATTCAAGAGATTTCAGAACTCTCCTCTAC (183) |
| 13 (ISOPEP- | GCCTCTAATCCTGATAAGGTTCAAGAGACCTTATCAGGATTAGAGGC (184) |
| TIDASE T- 3) | |
| | |
| UBI QUI TI N | GATGATCTTCAGGCTGCCATTCAAGAGATGGCAGCCTGAAGATCATC (185) |
| CARBOXYL- | GTATGGACAAGAGCGTTGGTTCAAGAGACCAACGCTCTTGTCCATAC (186) |
| TERMINAL | CGAACCCTTCTGGAACAGTTTCAAGAGAACTGTTCCAGAAGGGTTCG (187) |
| HYDROLASE 28 | GTGGCATGAAGATTATAGTTTCAAGAGAACTATAATCTTCATGCCAC (188) |
| | |
| UBI QUI TI N | GGTGAACAAGGACAGTATCTTCAAGAGAGATACTGTCCTTGTTCACC (189) |
| CARBOXYL- | GCAATAGAGGATGATTCTGTTCAAGAGACAGAATCATCCTCTATTGC (190) |
| TERMINAL | TCTGTGAATGCCAAAGTTCTTCAAGAGAGAACTTTGGCATTCACAGA (191) |
| HYDROLASE 14 | CACACCAGGGAAGGTCTAGTTCAAGAGACTAGACCTTCCCTGGTGTG (192) |
| | |
| DUB1 | GCAGGAAGATGCCCATGAATTCAAGAGATTCATGGGCATCTTCCTGC (193) |
| | GAATGTGCAATATCCTGAGTTCAAGAGACTCAGGATATTGCACATTC (194) |
| | TGGATGATGCCAAGGTCACTTCAAGAGAGTGACCTTGGCATCATCCA (195) |
| | GCTCCGTGCTAAACCTCTCTTCAAGAGAGAGAGGTTTAGCACGGAGC (196) |
| | |
| MOUSE USP27 | GCCTCCACCTCAACAGAGGTTCAAGAGACCTCTGTTGAGGTGGAGGC (197) |
| HOMOLOG | CTGCATCATAGACCAAATCTTCAAGAGAGATTTGGTCTATGATGCAG (198) |
| | GATCACTACATACATTTCCTTCAAGAGAGGAAATGTATGTAGTGATC (199) |
| | GTAAAGAGAGCAGAATGAATTCAAGAGATTCATTCTGCTCTCTTTAC (200) |
| | |
| UBIQUITIN | CGCGGGGCGCAGTGGTATCTTCAAGAGAGATACCACTGCGCCCCGCG (201) |
| CARBOXYL- | CAGAAGGCAGTGGGGAAGATTCAAGAGATCTTCCCCACTGCCTTCTG (202) |
| TERMINAL | GCCTGGGAGAATCACAGGTTTCAAGAGAACCTGTGATTCTCCCAGGC (203) |
| HYDROLASE 4 | ACCAGACAAGGAAATACCCTTCAAGAGAGGGTATTTCCTTGTCTGGT (204) |
| | |
| TRE-2 | CACATCCACCACATCGACCTTCAAGAGAGGTCGATGTGGTGGATGTG (205) |
| | GTCACAACCCAAGACCATGTTCAAGAGACATGGTCTTGGGTTGTGAC (206) |
| | CTCAACAGGACAAATCCCATTCAAGAGATGGGATTTGTCCTGTTGAG (207) |
| | TAGATCAATTATTGTGGATTTCAAGAGAATCCACAATAATTGATCTA (208) |
| | |
| UBIQUITIN CAR | GGAACACCTTATTGATGAATTCAAGAGATTCATCAATAAGGTGTTCC (209) |
| BOXYL-TERMINAL | CTTTAACAGAAATTGTCTCTTCAAGAGAGAGACAATTTCTGTTAAAG (210) |
| HYDROLASE 15 | CCTATGCAGTACAAAGTGGTTCAAGAGACCACTTTGTACTGCATAGG (211) |
| (UNPH-2). | GATCTTTTCTTGCTTTGGATTCAAGAGATCCAAAGCAAGAAAAGATC (212) |

| | |
|---|---|
| KIAA1372 | CAGCATCCTTCAGGCCTTATTCAAGAGATAAGGCCTGAAGGATGCTG (213) |
| | GATAGTGACTCGGATCTGCTTCAAGAGAGCAGATCCGAGTCACTATC (214) |
| | GACATCACAGCCCGGGAGTTTCAAGAGAACTCCCGGGCTGTGATGTC (215) |
| | GGACACAGCCTATGTGCTGTTCAAGAGACAGCACATAGGCTGTGTCC (216) |
| | |
| BRCA1 | GTGGAGGAGATCTACGACCTTCAAGAGAGGTCGTAGATCTCCTCCAC (217) |
| ASSOCIATED | CTCTTGTGCAACTCATGCCTTCAAGAGAGGCATGAGTTGCACAAGAG (218) |
| PROTEIN-1 | ACAGGGCCCCTGCAGCCTCTTCAAGAGAGAGGCTGCAGGGGCCCTGT (219) |
| | GAAGACCTGGCGGCAGGTGTTCAAGAGACACCTGCCGCCAGGTCTTC (220) |

The "regulator construct" comprises a repressor gene, which provides for perfect regulation of the operators of the responder construct. In particular, the repressor gene encodes a repressor, i.e. a molecule acting on the operator of the promoter to therewith inhibit (downregulate) the expression of the shRNA/siRNA. Suitable repressor genes include codon-optimized repressors (i.e., repressor genes where the codon usage is adapted to the codon usage of vertebrates), including, but not limited to, a codon-optimized tet repressor, a codon-optimized Gal repressor, a codon-optimized lac repressor and variants threreof. Particularly preferred is the codon optimized tet repressor, most preferred a codon-optimized tet repressor having the sequence of nucleotides 5149 to 5916 of SEQ ID NOs:2 or 3.

Embodiment (2) of the invention pertains to a method for preparing the biological entity as defined hereinbefore and to a method for constitutive and/or inducible gene knock down in a biological entity, which stably integrating
(i) the responder construct as defined hereinbefore, and
(ii) a regulator construct as defined hereinbefore
   into the genome of the biological entity.

In particular the method comprises subsequent or contemporary integration of the responder construct, and the regulator construct into the genome of vertebrate cells. In case of (non-human) mammals the constructs are preferably integrated into embryonic stem (ES) cells of said mammals.

Various methods are applicable for the integration of the constructs.

A first integration method is the so called "homologous recombination" which utilizes an integration vector comprising the functional nucleotide sequence to be integrated and DNA sequences homologous to the integration site, where said homologous DNA sequences flank the functional nucleotide sequence. In a particular preferred embodiment of the invention, both, the responder construct and the regulator construct are integrated by homologous recombination on the same or different allel(s).

A second integration method is the RMCE reaction, which comprises the steps of
(i) modifying a starting cell by introducing an acceptor DNA which integrates into the genome of the starting cell (e.g. by homologous recombination), and wherein the acceptor DNA comprises two mutally incompatible recombinase recognition sites (RRSs), and introducing into such modified cell;
(ii) a donor DNA comprising the same two mutually incompatible RRSs contained in the acceptor DNA by utilizing an integration vector comprising a functional DNA sequence flanked by the RRSs; and
(iii) a recombinase which catalyzes recombination between the RRSs of the acceptor and donor.

In a preferred embodiment of the invention the integration of at least one of the responder construct and the regulator construct is effected by RMCE reaction.

Details of the first and second method, in particular for integration at the murine Rosa26 locus are discussed in detail in applicant's WO 2004/063381, the disclosure of which is herewith incorporated by reference. For the integration at the murine Rosa26 locus (the sequence thereof being depicted in SEQ ID NO:11) by homologous recombination, the integration vector caries homologous flanking sequences of 0.2 to 20 kB, preferably 1 to 8 kB length. Suitable sequences include, but are not limited to, the sequences depicted in SEQ ID NOs:6 and 7.

A third integration method is the so-called "random transgenesis" where an integration vector is randomly integrated into the genome of the cell. By pronucleus injection of the linearized vector one or more copies of the DNA-fragment integrates randomly into the genome of the mouse embryo. The resulting founder lines have to be characterized for the expression of the transgene (Palmiter, R.D. and Brinster, R.L., Annu. Rev. Genet. 20:465-499 (1986)). Hasuwa H. et al. FEBS Lett. 532(1-2):227-230 (2002) used this technology for the generation of siRNA expressing mice and rats.

Particularly preferred in the invention is that the integration vector (in all three integration methods discussed above) carries both, the responder construct and the regulator construct.

The preparation of the vertebrate is hereinafter further described by reference to the mouse system. This shall, however, not be construed as limiting the invention. The preferred method for producing a shRNA h a mouse (and also mouse tissue and cells derived from such mouse) that expresses the codon optimized repressor protein comprising the steps of:
(i) insertion of a repressor construct carrying a codon-optimized repressor gene, such as the tet repressor gene, into the mouse genome; and
(ii) insertion of a responder construct containing
   - one or more promoter sequence(s), each carrying at least one operator sequence (such as tetO, etc.) positioned 1 to 10 bp, preferably 1 to 2 bp 3' and/or 5' of the TATA element and
   - a DNA sequence encoding a shRNA or siRNA as defined hereinbefore lying 3' to the said at least one operator sequence
   into the mouse genome; and
(iii) generation of mice from steps (i) and (ii); or
(iv) generation of mice from step (i) and generation of mice from step (ii) and a subsequent breeding of these two lines.

The inducible gene knock-down according to embodiments (2) and (3) of the invention moreover comprises the step of administering a suitable inducer compound to the biological entity (in particular the vertebrate) or ceasing the administering of the inducer compound to therewith induce or cease the expression of the respective siRNA.

The technology of the present application provides for the following advantages:
(i) a stable and body wide inhibition of gene expression by generating transgenic animals (such as mice);
(ii) a reversible inhibition of gene expression using the inducible constructs. The invention is furthermore described by the following examples which are, however, not to be construed so as to limit the invention.

### Examples

Plasmid construction: All plasmid constructs were generated by standard DNA cloning methods.

Basic rosa26 targeting vector: A 129 SV/EV-BAC library (Incyte Genomics) was screened using a probe against exo n2 of the Rosa26 locus (amplified from mouse genomic DNA using Rscreen1s (GACAGGACAGTGCTTGTTTAAGG; SEQ ID NO:4) and Rscreen1as (TGACTACACAATATTGCTCGCAC; SEQ ID NO:5)). Out of the identified BACclone a 11 kb EcoRV subfragment was inserted into the HindII site of pBS. Two fragments (a 1 kb SacII/XbaI- and a 4 kb Xbal-fragment; see SEQ ID NOs:6 and 7) were used as homology arms and inserted into a vector containing a FRT-flanked neomycin resistance gene or hygromycin resistance gene to generate the basic Rosa26 targeting vectors. The splice acceptor site (SA) from adenovirus (Friedrich, G. and Soriano, P., Genes Dev., 5:1513-23 (1991)) was inserted as PCR-fragment (amplified using the oligonucleotides ATACCTGCAGGGGTGACCTGCACGTCTAGG (SEQ ID NO: 15) and ATACCTGCAGGAG TACTGGAAAGACCGCGAAG (SEQ ID NO:16)) between the 5' arm and the FRT flanked neomycin resistance gene or the FRT flanked hygromycin resistant gene. The Renilla luciferase (Rluc) and firefly luciferase (Fluc) coding regions (Promega) were placed 3' of the SA site (Friedrich, G. and Soriano, P., Genes Dev. 9:1513-23 (1991); see SEQ ID NOs:1, 2 and 3)) to facilitate transcription from the endogenous *rosa26* promoter.

Insertion of transgenes into the targeting vector: All subsequently described transgenes were inserted 3' of the Renilla luciferase (Rluc) or firefly luciferase genes. The H1-promoter fragments were amplified from human genomic DNA (using the oligonucleotides AACTATGGCCGGCCGAAGAACTCGTCAAGAAGGCG (SEQ ID NO: 17) and TATGGTACCGTTTAAACGCGGCCGCAAATTTATTAGAGC (SEQ ID NO:18)) and the tet-operator sequences was placed 3' of the TATA-box. 3' of the the H1 -promoter with the tet-operator sequence a Fluc-specific shRNA was inserted by Bbsl/Ascl using annealed oligonucleotides forming the sequence aggattccaattcagcgggagccacctgatgaagcttgatcgggtggctctcgctgagttggaatccattttttt (SEQ ID NO:8; Paddison, P. J. et al., Genes Dev. 16:948-58 (2002)). The codon optimized tet-repressor was PCR amplified from pBS-hTA+nls (Anastassiadis, K. et al., Gene 298:159-72 (2002)) using the oligonucleotides atcgaattcaccatgtccagactgg (sense; SEQ ID NO:9), ataggatccttaagagccagactca catttcagc (antisense; SEQ ID NO:10)) and inserted 3' of the CAGGS promoter.
Vector 1 (SEQ ID NO:1) contains the following elements in 5' to 3' orientation: 5' homology region for murine rosa26 locus (nucleotides 24-1079), adenovirus splice acceptor site (nucleotides 1129-1249), firefly luciferase (nucleotides 1325-2977), synthetic poly A (2995-3173), CAGGS promoter (nucleotides 3231 -4860), synthetic intron (nucleotides 4862-5091), coding region of the wt tet repressor (nucleotides 5148-5750), synthetic poly A (nucleotides 5782-5960), FRT-site (nucleotides 6047-6094), PGK-hygro-polyA (nucleotides 6114-8169), FRT-site, 3' homology region for rosa26 locus (nucleotides 8312-12643), PGK-Tk-polyA (nucleotides 12664-14848).
Vector 2 (SEQ ID NO:2) contains the following elements in 5' to 3' orientation: 5' homology region for rosa26 locus (nucleotides 24-1102), adenovirus splice acceptor site (nucleotides 1129-1249), firefly luciferase (nucleotides 1325-2977), synthetic poly A (nucleotides 2995-3173), CAGGS promoter (nucleotides 3231-4860), synthetic intron (nucleotides 4862-5091), coding region of the codon optimized tet repressor (nucleotides 5149-5916), synthetic poly A (nucleotides 5946-6124), FRT-site (nucleotides 6211-6258), PGK-hygro-polyA (nucleotides 6278-8333), FRT-site, 3' homology region for rosa26 locus (nucleotides 8476-12807), PGK-Tk-polyA (nucleotides 12828-15012).
Vector 3 (SEQ ID NO:3) contains the following elements in 5' to 3' orientation: 5' homology region for rosa26 locus (nucleotides 31-2359), adenovirus splice acceptor site (nucleotides 2409-2529), Renilla luciferase (nucleotides 2605-3540), synthetic poly A (nucleotides 3558-3736), hgH-poly A (nucleotides 3769-4566) H1-tetO (nucleotides 4742-4975), shRNA (nucleotides 4977-5042), loxP-site (nucleotides 4587-4620), TTTTTT, loxP-site, synthetic intron (nucleotides 4862-5091), coding region of the codon optimized tet repressor (nucleotides 5149-5916), synthetic poly A (nucleotides 5946-6124), FRT-site (nucleotides 6211-6258), PGK-hygro-polyA (nucleotides 6278-8333), FRT-site (nucleotides 5105-5152), 3' homology region for rosa26 locus (nucleotides 7042-11373), PGK-Tk-polyA (nucleotides 11394-13578).

Cell culture: Culture and targeted mutagenesis of ES cells were carried out as described in Hogan, B. et al., A Laboratory Manual. In Manipulating the Mouse Embryo. Cold Spring Harbor Laboratory Press, Cold Spring Harbor NY, pp. 253-289 (1994) with ES cell lines derived from F1 embryos. Cre-mediated deletion has been performed for the deletion of the shRNA part of the constructs to generate the control mice without knockdown. Therefore 5 µg of a cre-expressing construct has been electroporated and the following day 1000 cells were plated at a 10 cm dish. The developing clones were isolated and screened by southern for cre-mediated deletion of the shRNA responder construct.

Generation of chimeric mice: Recombinant ES cells were injected into blastocysts from Balb/C mice and chimeric mice were obtained upon transfer of blastocysts into pseudopregnant females using standard protocols (Hogan, B. et al. Manipulating the Mouse Embryo: A Laboratory Manual. Cold Spring Harbor Laboratory Press, Cold Spring Harbor NY. 253-289 (1994)).

Preparation and application of doxycycline: 2 mg doxycycline (Sigma, D-9891) was solved in 1 liter H₂O with 10 % Sucrose. This solution was given in drinking bottles of mice and prepared freshly every 3 days.

Luciferase measurement in organs: Organs were homogenized at 4°C in lysis buffer (0.1 M KH₂PO₄, 1 mM DTT, 0.1% Triton^{®} X-100) using a tissue grinder. Spin for 5' at 2000xg (4°C) to pellet debris and assay supernatant for Luc activities using the Dual Luciferase Assay (Promega, Inc.) according to the manufacturer protocol.

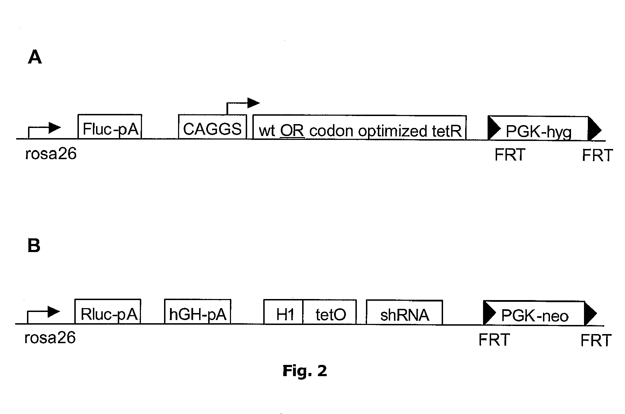

Discussion: The coding regions of the wt (Gossen and Bujard, PNAS. 89: 5547-5551; figure 2; SEQ ID NO:1) or the codon optimized tet repressor (Anastassiadis, K. et al., Gene 298:159-72 (2002)) under control of the strong CAGGS promoter along with a hygromycine resistance gene and a firefly luciferase gene were inserted into the first allele of rosa26 by homologous recombination in ES cells (figure 2A; SEQ ID NO:2). The shRNA coding region under the control of the H1 promoter containing tet-operator sequences (H1-tetO), along with a Renilla luciferase gene and a neomycin resistance gene for positive selection of recombinant clones was inserted into the second allele of the rosa26 locus (figure 2B; SEQ ID NO:3). To examine the activity of the Rosa26 and H1-tetO-shRNA transgenes in vivo, recombinant ES cells of the three independent constructs described above (SEQ ID NOs:1 to 3) were injected into diploid blastocysts and chimeric mice were obtained upon transfer of blastocysts into pseudopregnant females. Mice were bred to generate double transgenic animals containing the constructs shown in SEQ ID NOs:1 and 3 or SEQ ID NOs:2 and 3, respectively.

Mice were fed for 10 days with drinking water in the presence or absence of 2 µg/ml Doxycycline. Figure 3 shows the firefly luciferase activity measured in different organs of mice. The Renilla luciferase gene at the second Rosa26 allele served as a reference to normalize the values of firefly luciferase activity. Doxycycline inducible expression of the shRNA under the control of the H1-tetO promoter (SEQ ID NO:3) resulted in a efficient reduction of firefly luciferase activity in most organs of mice expressing the wt tet repressor or expressing the codon optimized tet repressor (Figure 3). Unexpectedly in the absence of doxycycline a efficient knockdown was measured for mice expressing the wt tet repressor (Figure 3A; SEQ ID NOs:1 and 3). This demonstrate that the wt tet repressor is not able to inhibit the activation of H1 -tetO driven shRNA through Polymerase III dependent promoter. In contrast, mice carrying the codon optimized tet repressor (Figure 3B; SEQ ID NOs:2 and 3) did not show any detectable knockdown of luciferase in the absence of doxycycline. Moreover, the degree of RNAi upon induction was similar compared to the system using the wt repressor.

### Sequence Listing Free Text (to be completed)

- SEQ I D NO:1: Targeting vector for rosa26 locus expressing the wt tet-repressor.
- SEQ I D NO:2: Targeting vector for rosa26 locus expressing the codon optimized tet-repressor.
- SEQ I D NO:3: Targeting vector for rosa26 locus containing the H1 -tet inducible shRNA.
- SEQ ID NOs:4 and 5: Primer Rscreen1s and Rscreen1as, respectively.
- SEQ ID NO:6: 5' arm for Rosa26
- SEQ ID NO:7: 3' arm for Rosa26
- SEQ ID NO:8: firefly luciferase-specific shRNA.
- SEQ ID NOs:9 and 10: Primer for isolation of codon optimized tet repressor
- SEQ ID NO:11: Murine Rosa26 locus
- SEQ ID NOs:12 to 14: siRNA sequences
- SEQ ID NOs:15 and 16: Primer for isolation of SA from adenovirus
- SEQ ID NO: 17 and 18: Primer for isolation of H1 promoter
- SEQ ID NOs: 19 to 220: shRNA sequences, the function thereof being given in Tables 1 and 2

## Claims

1. A biological entity selected from a vertebrate, a tissue culture derived from a vertebrate or one or more cells of a cell culture derived from a vertebrate, said biological entity carrying
(i) a responder construct comprising at least one segment corresponding to a short hairpin RNA (shRNA) or to complementary short interfering RNA (siRNA) strands, said segment being under control of a ubiquitous promoter, said promoter containing at least one operator sequence being perfectly regulatable by a repressor; and
(ii) a regulator construct comprising a repressor gene, which provides for perfect regulation of the promoter of the responder construct.

2. The biological entity according to claim 1, wherein
(i) said responder construct and said regulator construct allow inducible gene knock down in said biological entity, the regulation by said repressor permits control of the expression and the suppression of the expression of the shRNA or the siRNA by a rate of at least 90%, preferably by a rate of at least 95%, more preferably by a rate of at least 98%, and most preferably by a rate of 100%; and/or
(ii) the responder construct aid/or the regulator construct is (are) stably integrated into the genome of the biological entity, preferably at a defined locus, by homologous recombination, recombinase mediated cassette exchange (RMCE) or the like, or is (are) inserted through random transgenesis; and/or
(iii) the responder construct and/or the regulator construct is(are) stably integrated, through homologous recombination or RMCE, at a defined genomic locus, preferably at a ubiquitously active polymerase (Pol) II or Pol III dependent locus in the genome of the biological entity, most preferably is(are) stably integrated at a polymerase II dependent locus selected from a Rosa26, collagen, RNA polymerase, actin and HPRT locus; and/or
(iv) the promoters of the responder construct and/or the regulator construct are independently selected from polymerase (Pol) I, II and III dependent promoters, preferably are Pol II and III dependent promoters, more preferably are selected from a CMV promoter, a CAGGS promoter, a snRNA promoter such as U6, a RNAse P RNA promoter such as H1, a tRNA promoter, a 7SL RNA promoter, and a 5 S rRNA promoter; and/or
(v) the responder construct and/or the regulator construct further contain functional sequences selected from splice acceptor sequences, polyadenylation sites, selectable marker sequences, recombinase recognition sequences, etc.; and/or
(vi) the responder construct and the regulator construct are integrated at the same locus or at different loci in the genome of the biological entity, preferably are integrated at different loci or at different alleles of the same locus in the genome of the biological entity; and/or
(vii) the vertebrate is a non-human vertebrate, preferably is a rodent, such as mouse or rat, or a fish, most preferably the vertebrate is a mouse.

3. The biological entity according to claim 1 or 2, wherein in the responder construct
(i) the promoter is (are) inducible promoter(s) selected from polymerase (Pol) I, II and III dependent promoters, preferably is (are) Pol III dependent promoter(s), more preferably is (are) snRNA promoter(s) such as U6, or RNAse P RNA promoter(s) such as H1, and most preferably is a H1 -promoter; and/or
(ii) the promoter(s) contain(s) an operator sequence(s) selected from tetO, GalO, lacO, etc., preferably the operator sequence is tetO; and/or
(iii) the operator sequence(s) of the promoter is(are) positioned 1 to 10 bp, preferably1 to 2 bp 3' (i.e., downstream) and/or 5' (i.e., upstream) of the TATA element; and/or
(iv) the DNA sequence corresponding to the shRNA or siRNA is (are) positioned 3' to said operator sequence.

4. The biological entity according to any of claims 1 to 3, wherein the responder construct
(i) is integrated into a ubiquitously active Pol II dependent locus; and/or
(ii) carries a Pol III dependent promoter, preferably an inducible U6 or H1 promoter, containing the operator, such as tetO, and the segment(s) corresponding to a shRNA or siRNA; and/or
(iii) comprises at least one shRNA segment having a DNA sequence A-B-C or C-B-A, or comprises at least two siRNA segments A and C or C and A, each of said at least two siRNA segments being under the control of a separate promoter, wherein
A is a 15 to 35, preferably a 19 to 29 bp DNA sequence with at least 95%, preferably 100% complementarily to the gene to be knocked down;
B is a spacer DNA sequence having 5 to 9 bp forming the loop of the expressed RNA hair pin molecule; and
C is a 15 to 35, preferably a 19 to 29 bp DNA sequence with at least 85% complementarily to the sequence A; and/or
(iv) comprises a stop and/or a polyadenylation sequence.

5. The biological entity according to any of claims 1 to 4 wherein in the regulator construct
(i) the repressor gene is under control of an ubiquitous promoter preferably selected from polymerase (Pol) I, II and III dependent promoters, preferably is a Pol II dependent promoter, more preferably is a CMV promoter or a CAGGS promoter; and/or
(ii) the repressor gene is selected from codon-optimized repressors preferably is a codon optimized tet repressor, a codon optimized Gal4 repressor, a codon optimized lac repressor or a variant theref, preferably is codon-optimized tet repressor having the sequence of nucleotides 5149 to 5916 of SEQ ID NOs:2.

6. The biological entity according to anyone of claims 1 to 5, wherein the biological entity is a mouse, mouse cell or mouse tissue, the responder construct comprises a H1 -promoter sequence with one tet operator sequence positioned 1-2 bp 3' of the TATA element and a DNA sequence encoding a shRNA lying 3' to the said tet operator sequence, and the regulator construct comprises a codon-optimized tet repressor gene.

7. A method for preparing the biological entity as defined in any of claims 1 to 6 or a method for inducible gene knock down in a biological entity, which method comprises stably integrating
(i) a responder construct as defined in any of claims 1 to 4 or 6, and
(ii) a regulator construct as defined in claim 1, 2, 5 or 6
into the genome of the biological entity.

8. The method of claim 6
(i) which comprises subsequent or contemporary integration of the responder construct, and the regulator construct into the genome of vertebrate cells, preferably into embryonic stem (ES) cells of mammals; and/or
(ii) wherein the integration of both, the responder construct and the regulator construct is effected by homologous recombination; and/or
(iii) wherein the integration of at least one of the responder construct and the regulator construct is effected by RMCE; and/or
(iv) wherein the integration of at least one of the responder construct and the regulator construct is effected by random transgenesis; and/or
(v) wherein the integration is effected by using an integration vector carrying both, the responder construct and the regulator construct.

9. The method of claim 7, which is suitable for preparing a transgenic vertebrate, preferably a transgenic mammal and which comprises
(i) generating a first vertebrate or a first vertebrate line being transformed with the responder construct,
(ii) generating a second vertebrate or second vertebrate line being transformed with the regulator construct, and
(iii) crossing at least one of said first vertebrates with at least one of said second vertebrates.

10. Use of a biological entity as defined in any of claims 1 to 6 for inducible gene knock down.
